# EUROPEAN PATENT APPLICATION

(11) **EP 4 538 370 A1**
(43) Date of publication of application: **16.04.2025**
(21) Application number: 23819043.3
(22) Date of filing: 05.06.2023
(51) Int. Cl.: C12N 9/52, C12N 15/62, C07K 16/12, C12N 15/57, A61K 38/48, A61P 13/12, A61P 37/00

(54) **FUSION PROTEIN CONTAINING TRUNCATED IGA PROTEASE AND USE THEREOF**

(30) Priority: 07.06.2022 CN 202210637468; 26.05.2023 CN 202310610754
(71) Applicant: Peking University First Hospital, Beijing 100034 (CN); Shanghai Alezyme Pharmaceuticals Ltd., Pudong New Area, Shanghai 201210 (CN)
(72) Inventor: LV, Jicheng, Beijing 100034 (CN); ZHANG, Hong, Beijing 100034 (CN); SHU, Chutian, Shanghai 201210 (CN)
(74) Representative: Simmons & Simmons LLP (Munich)
(86) International application number: PCT/CN2023/098207
(87) International publication number: WO 2023/236878

(57) **Abstract**

The present application relates to a truncated IgA protease, a fusion protein comprising the truncated IgA protease (e.g., a fusion protein comprising the truncated IgA protease and an Fc), and use thereof in treating an IgA deposition disease (e.g., IgA nephropathy).

## Description

### FIELD OF THE INVENTION

The present disclosure relates to the biopharmaceutical field. In particular, the present disclosure relates to a truncated form of IgA protease, a fusion protein comprising the truncated form of IgA protease, a pharmaceutical composition comprising the truncated form of IgA protease or the fusion protein, a nucleic acid encoding the truncated form of IgA protease or the fusion protein, a method for preparing the truncated form of IgA protease or the fusion protein, and use of the truncated form of IgA protease or the fusion protein in the manufacture of a medicament for treating diseases associated with IgA deposition.

### BACKGROUND OF THE INVENTION

IgA nephropathy is currently one of the most common primary glomerular diseases in the world and places a heavy burden on patients and society. There is a lack of specific treatment for IgA nephropathy. Most clinical treatment is based on supportive therapy with RAS blockers to slow down the deterioration of renal function. Patients who fail to respond to supportive therapy are treated with a combination of hormonal immunosuppressive agents. However, long-term use of hormonal immunosuppressants may cause serious side effects to patients.

There is an urgent need to develop effective therapeutic agents with low side effects.

### SUMMARY OF THE INVENTION

In one aspect, the present disclosure provides a fusion protein comprising a first polypeptide and a second polypeptide, wherein the first polypeptide is a truncated form of IgA protease, and the second polypeptide cannot be cleaved by an IgA protease or a truncated form of IgA protease.

In some embodiments, the truncated form of IgA protease comprises a non-natural truncated fragment of a wild-type IgA protease obtained from or derived from a *Streptococcus pneumoniae* TIGR4 strain, or having at least 70% sequence identity to the non-natural truncated fragment. In some embodiments, the non-natural truncated fragment has an amino acid mutation, deletion, insertion or modification compared to the wild-type IgA protease of *Streptococcus pneumoniae* TIGR4 strain, such that the truncated form of IgA protease loses or reduces its self-cleaving function. In some embodiments, the amino acid mutation, deletion, insertion or modification occurs at a natural self-cleaving site of the wild-type IgA protease of *Streptococcus pneumoniae* TIGR4 strain, within 5 amino acid residues upstream and/or within 5 amino acid residues downstream of the natural self-cleaving site. In some embodiments, the natural self-cleaving site is between position 43 and position 745 of the amino acid sequence as set forth in SEQ ID NO: 1. In some embodiments, the natural self-cleaving site is between position 71 and position 72, between position 210 and position 211, between position 228 and position 229, between position 239 and position 240, between position 245 and position 246, between position 382 and position 383, between position 418 and position 419, between position 439 and position 440, between position 490 and position 491, between position 506 and position 507, between position 509 and position 510, between position 514 and position 515, between position 533 and position 534, between position 536 and position 537, between position 563 and position 564, between position 594 and position 595, between position 613 and position 614, between position 616 and position 617, between position 639 and position 640, between position 645 and position 646, or between position 678 and position 679 of the amino acid sequence as set forth in SEQ ID NO: 1.

In some embodiments, the non-natural truncated fragment is a N-terminal or C-terminal truncated fragment of the wild-type IgA protease obtained from or derived from the *Streptococcus pneumoniae* TIGR4 strain. In some embodiments, the C-terminal truncated fragment comprises a polypeptide fragment of at least 703 continuous amino acids starting from position 43 of the wild-type IgA protease obtained from or derived from the *Streptococcus pneumoniae* TIGR4 strain, or having at least 70% sequence identity to the polypeptide fragment.

In some embodiments, deposit number of *Streptococcus pneumoniae* TIGR4 is ATCC BAA-334. In some embodiments, the amino acid sequence of the wild-type IgA protease of *Streptococcus pneumoniae* TIGR4 strain is as set forth in SEQ ID NO: 1.

In some embodiments, the first polypeptide comprises a polypeptide fragment of at least 1300 *(e.g.,* at least 1310, at least 1320, at least 1330, or at least 1340) continuous amino acids starting from position 665 of the amino acid sequence as set forth in SEQ ID NO: 1. In some embodiments, the first polypeptide comprises a polypeptide fragment of amino acids starting from position 665 to position 2004 of the amino acid sequence as set forth in SEQ ID NO: 1, or having at least 70% sequence identity to the polypeptide fragment. In some embodiments, the fusion protein has an amino acid conservative substitution at one or more sites compared to the amino acid sequence of the polypeptide fragment. In some embodiments, the first polypeptide comprises an amino acid as set forth in SEQ ID NO: 2. In some embodiments, the second polypeptide is located at N-terminus or C-terminus of the first polypeptide.

In some embodiments, the fusion protein has an enzymatic activity of specifically cleaving human IgA. In some embodiments, the fusion protein has an enzymatic activity of specifically cleaving human IgA heavy chain. In some embodiments, the fusion protein has an enzymatic activity of specifically cleaving human IgA heavy chain hinge region. In some embodiments, the fusion protein has an enzymatic activity of specifically cleaving human IgA1. In some embodiments, the fusion protein has an enzymatic activity of specifically cleaving human IgA1 heavy chain. In some embodiments, the fusion protein has an enzymatic activity of specifically cleaving human IgA1 heavy chain hinge region.

In some embodiments, the first polypeptide and the second polypeptide are linked via a linker. In some embodiments, the linker is selected from the group consisting of a cleavable linker, a non-cleavable linker, a peptide linker, a flexible linker, a rigid linker, a helical linker and a non-helical linker. In some embodiments, the linker comprises a peptide linker. In some embodiments, the peptide linker comprises a linker comprising a glycine and a serine. In some embodiments, the linker comprising a glycine and a serine comprises one, two, three, four or more repeats of the amino acid sequence as set forth in SEQ ID NO: 15 (GGGS), SEQ ID NO: 16 (GGGGS), SEQ ID NO: 17 (GGGGGGS) or SEQ ID NO: 19 (GSS). In some embodiments, the linker comprises an amino sequence as set forth in SEQ ID NO: 18 (GGGGSGGGGSGGGGS), SEQ ID NO: 20 (GSSGSSG) or SEQ ID NO: 21 (RSGSSGSSG).

In some embodiments, the first polypeptide and the second polypeptide are directly linked to each other.

In some embodiments, the second polypeptide comprises an amino acid sequence for extending half-life of the first polypeptide in a subject. In some embodiments, the second polypeptide is selected from an Fc domain and albumin. In some embodiments, the Fc domain comprises a hinge region. In some embodiments, the Fc domain is derived from human IgG Fc domain. In some embodiments, the Fc domain is derived from human IgG4 Fc domain. In some embodiments, the Fc domain comprises an amino acid sequence having at least 70%, at least 75%, at least 80%, at least 85%, at least 90% or at least 95% sequence identity to SEQ ID NO: 14. In some embodiment, the Fc domain comprises an amino acid sequence as set forth in SEQ ID NO: 14. In some embodiments, the Fc domain comprises one or more mutations that extend half-life of the fusion protein. In some embodiments, the Fc domain is linked to C-terminus or N-terminus of the first polypeptide. In some embodiments, the fusion protein comprises a first polypeptide and a second polypeptide, wherein the amino acid sequence of the first polypeptide is as set forth in SEQ ID NO: 2, and the amino acid sequence of the second polypeptide is as set forth in SEQ ID NO: 14. In some embodiments, the fusion protein comprises a first polypeptide and a second polypeptide, wherein the first polypeptide and the second polypeptide are directly linked via a linker as set forth in SEQ ID NO: 20 or SEQ ID NO: 21. In some embodiments, the amino acid sequence of the fusion protein is as set forth in SEQ ID NO: 8. In some embodiments, the amino acid sequence of the fusion protein is as set forth in SEQ ID NO: 24. In some embodiments, the fusion protein comprises a first polypeptide and a second polypeptide, wherein the first polypeptide and the second polypeptide are linked via a linker as set forth in SEQ ID NO: 20, wherein the amino acid sequence of the first polypeptide is as set forth in SEQ ID NO: 2, and the amino acid sequence of the second polypeptide is as set forth in SEQ ID NO: 14. In some embodiments, the fusion protein comprises a first polypeptide and a second polypeptide, wherein the first polypeptide and the second polypeptide are linked via a linker as set forth in SEQ ID NO: 21, wherein the amino acid sequence of the first polypeptide is as set forth in SEQ ID NO: 2, and the amino acid sequence of the second polypeptide is as set forth in SEQ ID NO: 14. In some embodiments, the albumin comprises one or more domains of human serum albumin. In some embodiments, the albumin comprises a D3 domain of human serum albumin.

In some embodiments, the fusion protein further comprises a label. In some embodiments, the label is selected from the group consisting of a fluorescent label, a luminescent label, a purification label and a chromogenic label. In some embodiments, the label is selected from the group consisting of a c-Myc tag, an HA tag, a VSV-G tag, a FLAG tag, a V5 tag and a HIS tag. In some embodiments, the label is a HIS tag comprising 6, 7, 8, 9, 10 or more histidine. In some embodiments, the second polypeptide is located at C-terminus of the first polypeptide and the label is located at C-terminus of the second polypeptide.

In some embodiments, the fusion protein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days in blood circulation of a subject.

In another aspect, the present disclosure provides an isolated nucleic acid comprising a nucleotide sequence encoding the fusion protein described herein. In some embodiments, the nucleic acid of the present disclosure comprises a nucleotide sequence as set forth in SEQ ID NO: 12 or a nucleotide sequence having at least 70% sequence identity to SEQ ID NO: 12. In some embodiments, the nucleic acid of the present disclosure comprises a nucleotide sequence as set forth in SEQ ID NO: 25 or a nucleotide sequence having at least 70% sequence identity to SEQ ID NO: 25.

In another aspect, the present disclosure provides an isolated truncated form of IgA protease, comprising a non-natural truncated fragment of a wild-type IgA protease obtained from or derived from a *Streptococcus pneumoniae* TIGR4 strain, or having at least 70% sequence identity to the non-natural truncated fragment. In some embodiments, the non-natural truncated fragment has an amino acid mutation, deletion, insertion or modification compared to the wild-type IgA protease of *Streptococcus pneumoniae* TIGR4 strain, such that the truncated form of IgA protease loses or reduces its self-cleaving function. In some embodiments, the amino acid mutation, deletion, insertion or modification occurs at a natural self-cleaving site of the wild-type IgA protease of *Streptococcus pneumoniae* TIGR4 strain, within 5 amino acid residues upstream and/or within 5 amino acid residues downstream of the natural self-cleaving site.

In some embodiments, the non-natural truncated fragment is a N-terminal or C-terminal truncated fragment of the wild-type IgA protease obtained from or derived from *Streptococcus pneumoniae* TIGR4 strain. In some embodiments, the deposit number of *Streptococcus pneumoniae* TIGR4 strain is ATCC BAA-334. In some embodiments, the C-terminal truncated fragment comprises a polypeptide fragment of at least 703 continuous amino acids starting from position 43 of the wild-type IgA protease obtained from or derived from *Streptococcus pneumoniae* TIGR4 strain, or having at least 70% sequence identity to the polypeptide fragment. In some embodiments, the amino acid sequence of the wild-type IgA protease of *Streptococcus pneumoniae* TIGR4 strain is as set forth in SEQ ID NO: 1.

In some embodiments, the natural self-cleaving site is between position 43 and position 745 of the amino acid sequence as set forth in SEQ ID NO: 1. In some embodiments, the natural self-cleaving site is between position 71 and position 72, between position 210 and position 211, between position 228 and position 229, between position 239 and position 240, between position 245 and position 246, between position 382 and position 383, between position 418 and position 419, between position 439 and position 440, between position 490 and position 491, between position 506 and position 507, between position 509 and position 510, between position 514 and position 515, between position 533 and position 534, between position 536 and position 537, between position 563 and position 564, between position 594 and position 595, between position 613 and position 614, between position 616 and position 617, between position 639 and position 640, between position 645 and position 646, or between position 678 and position 679 of the amino acid sequence as set forth in SEQ ID NO: 1.

In some embodiments, the truncated form of IgA protease comprises a polypeptide fragment of at least 1300 *(e.g.,* at least 1310, at least 1320, at least 1330, or at least 1340) continuous amino acids starting from position 665 of the amino acid sequence as set forth in SEQ ID NO: 1. In some embodiments, the truncated form of IgA protease comprises a polypeptide fragment of amino acids starting from position 665 to position 2004 of the amino acid sequence as set forth in SEQ ID NO: 1, or having at least 70% sequence identity to the polypeptide fragment. In some embodiments, the truncated form of IgA protease has an amino acid conservative substitution at one or more sites compared to the amino acid sequence of the polypeptide fragment. In some embodiments, the truncated form of IgA protease comprises an amino acid as set forth in SEQ ID NO: 2. In some embodiments, the truncated form of IgA protease consists of an amino acid as set forth in SEQ ID NO: 2.

In some embodiments, the truncated form of IgA protease provided herein has an enzymatic activity of specifically cleaving human IgA. In some embodiments, the truncated form of IgA protease provided herein has an enzymatic activity of specifically cleaving human IgA heavy chain. In some embodiments, the truncated form of IgA protease provided herein has an enzymatic activity of specifically cleaving interchange of human IgA heavy chain CH1 and hinge region. In some embodiments, the truncated form of IgA protease provided herein has an enzymatic activity of specifically cleaving human IgA1. In some embodiments, the truncated form of IgA protease provided herein has an enzymatic activity of specifically cleaving human IgA1 heavy chain. In some embodiments, the truncated form of IgA protease provided herein has an enzymatic activity of specifically cleaving the interchange of human IgA1 heavy chain CH1 and hinge region.

In another aspect, the present disclosure provides an isolated nucleic acid comprising a nucleotide sequence encoding the truncated form of IgA protease provided herein.

In another aspect, the present disclosure provides a vector comprising the nucleic acid described herein.

In another aspect, the present disclosure provides a cell comprising the nucleic acid or vector described herein. In some embodiments, the cell is a prokaryotic cell or a eukaryotic cell. In some embodiments, the prokaryotic cell is an *E. coli* cell. In some embodiments, the eukaryotic cell is a mammalian cell. In some embodiments, the mammalian cell is a human cell or a Chinese hamster ovary (CHO) cell. In some embodiments, the mammalian cell is a human embryonic kidney cell 293 (HEK293 cell).

In another aspect, the present disclosure provides a pharmaceutical composition comprising the fusion protein described herein, comprising the nucleic acid described herein, comprising the vector described herein or comprising the cell described herein, and a pharmaceutically acceptable carrier.

In another aspect, the present disclosure provides a method of producing a fusion protein comprising a step of culturing the cell described herein.

In another aspect, the present disclosure provides a method of treating or preventing a disease associated with IgA deposition, comprising administering to a subject in need thereof the truncated form of IgA protease provided herein, the fusion protein described herein, or the pharmaceutical composition described herein.

In another aspect, the present disclosure provides use of the truncated form of IgA protease described herein, the fusion protein described herein, or the pharmaceutical composition described herein in the manufacture of a medicament for treating or preventing a disease associated with IgA deposition.

In another aspect, the present disclosure provides the truncated form of IgA protease described herein, the fusion protein described herein, or the pharmaceutical composition described herein for use in treating or preventing a disease associated with IgA deposition.

In another aspect, the present disclosure provides a method of treating or preventing a disease associated with IgA deposition, comprising administering to a subject in need thereof an IgA protease or a truncated form thereof, a fusion protein comprising the IgA protease or the truncated form thereof, or a pharmaceutical composition comprising the IgA protease or the truncated form thereof or the fusion protein, wherein, the amino acid sequence of the IgA protease is selected from the group consisting of: SEQ ID NO: 26, SEQ ID NO: 27, SEQ ID NO: 28, SEQ ID NO: 29, SEQ ID NO: 30 or combination thereof.

In another aspect, the present disclosure provides use of an IgA protease or the truncated form thereof, a fusion protein comprising the IgA protease or the truncated form thereof, or a pharmaceutical composition comprising the IgA protease or the truncated form thereof or the fusion protein in the manufacture of a medicament for treating or preventing a disease associated with IgA deposition, wherein, the amino acid sequence of the IgA protease is selected from the group consisting of: SEQ ID NO: 26, SEQ ID NO: 27, SEQ ID NO: 28, SEQ ID NO: 29, SEQ ID NO: 30 or combination thereof.

In another aspect, the present disclosure provides an IgA protease or the truncated form thereof, a fusion protein comprising the IgA protease or the truncated form thereof, or a pharmaceutical composition comprising the IgA protease or the truncated form thereof or the fusion protein for use in treating or preventing a disease associated with IgA deposition, wherein, the amino acid sequence of the IgA protease is selected from the group consisting of: SEQ ID NO: 26, SEQ ID NO: 27, SEQ ID NO: 28, SEQ ID NO: 29, SEQ ID NO: 30 or combination thereof.

In some embodiments, the disease associated with IgA deposition comprises IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis. In some embodiments, the disease associated with IgA deposition is IgA nephropathy, IgA vasculitis or Kawasaki disease.

### BRIEF DESCRIPTION OF THE DRAWINGS

FIG. 1 shows the experimental result of *in vitro* enzymatic cleaving activities against IgA1 of different truncated forms of TIGR4 IgA, *i.e.,* TIGR4 (745-2004), TIGR4 (845-2004) and TIGR4 (945-2004).
FIG. 2a shows the experimental result of *in vitro* enzymatic cleaving activity against IgA1 of Fusion Protein 1, and FIG. 2b shows an expression of Fusion Protein 1 in prokaryotic cells BL21 (DE3).
FIG. 3a shows the experimental result of *in vitro* enzymatic cleaving activity against IgA1 of Fusion Protein 2, and FIG. 3b shows an expression of Fusion Protein 2 in prokaryotic cells BL21 (DE3).
FIG. 4a shows the experimental result of *in vitro* enzymatic cleaving activity against IgA1 of Fusion Protein 3, and FIG. 4b shows an expression of Fusion Protein 3 in prokaryotic cells BL21 (DE3).
FIG. 5 shows the experimental result of *in vitro* enzymatic cleaving activity against IgA1 of Fusion Protein 3 obtained by purifying with nickel and S200, respectively.
FIG. 6 shows aggregation states of Fusion Protein 3 obtained by purifying with nickel at different time points when being placed at 4°C; an aggregation state of different flow-through peaks of Fusion Protein 3 obtained by purifying with S200; and a liquid chromatogram of a purified product.
FIG. 7a shows stability of a first flow-through peak F1 of Fusion Protein 3 obtained by purifying with S200 after being placed at 37°C for different time periods; and FIG. 7b shows stability of Fusion Protein 3 obtained by purifying with S200 after being placed at 37°C for different time periods.
FIG. 8a shows aggregation states of Fusion Protein 4 obtained by purifying with nickel and S200, respectively; and FIG. 8b shows stability of Fusion Protein 4 obtained by purifying with nickel when being placed at 37°C for different time.
FIG. 9 shows *in vivo* activity of Fusion Protein 4 in humanized IgA1 (α1KI-Tg) C57BL/6 mice.
FIG. 10a shows the experimental result of *in vitro* enzymatic activity against IgA1 of Fusion Protein 5; and FIG. 10b shows stability of Fusion Protein 5 obtained by purifying with nickel after being placed at 37°C for different time periods.
FIG. 11 shows *in vivo* activity of Fusion Protein 5 in humanized IgA1 (α1KI-Tg) C57BL/6 mice.
FIG. 12 shows the experimental result of enzymatic cleaving activities against IgA1 OF five TIGR4 homologoases.

### DETAILED DESCRIPTION OF THE INVENTION

Although the present disclosure will disclose various aspects and embodiments below, it will be apparent to one skilled in the art that various equivalents, changes, and modifications may be made without departing from the scope of the disclosure, and it is understood that such equivalent embodiments are to be included herein. The various aspects and embodiments disclosed herein are for illustrative purposes only and are not intended to limit the scope of the present application, and the actual protection scope of this application is subject to the claims. Unless otherwise indicated, all technical and scientific terms used herein have the same meanings as those generally understood by those of ordinary skill in the art to which the present application pertains. All references cited herein, including literatures, patents and patent applications, are incorporated herein by reference in their entirety.

### Definitions

As used herein, the term *"Streptococcus pneumoniae"* refers to an opportunistic pathogen belonging to Gram-positive cocci which can produce IgA protease.

As used herein, the term "protease" refers to an enzyme capable of cleaving protein and peptides. Proteases can cleave protein by hydrolyzing peptide bonds that connect amino acids together in the peptide or polypeptide chains that form protein. Various methods are known in the prior art to test the proteolytic activity of a protease. For example, the proteolytic activity of proteases can be determined by colorimetric assays that analyze the ability of various proteases to hydrolyze suitable substrates. Exemplary substrates for proteolytic activity analysis include, for example, dimethyl casein, bovine collagen, bovine elastin, and the like. Colorimetric assays using these substrates are also known in the art (see, for example, WO99/34011 and U.S. Pat. No. 6,376,450).

As used herein, the term "IgA protease" refers to an enzyme that is capable of specifically cleaving or decomposing an IgA immunoglobulin molecule (e.g., IgA1 or IgA2) in a subject (e.g., human). For example, IgA protease obtained from or derived from *Streptococcus pneumoniae* TIGR4 strain can specifically cleave the peptide bond between proline (Pro) at position 227 and threonine (Thr) at position 228 of IgA1, thereby decomposing IgA1.

When referring to a polypeptide or protein, the term "wild-type" used herein refers to a naturally occurring polypeptide or protein that does not include artificial mutation, insertion, deletion or modification at one or more amino acid positions; when referring to a nucleic acid, nucleotide or polynucleotide, the term "wild-type" used herein refers to a naturally occurring nucleic acid, nucleotide or polynucleotide that does not include artificial mutation, insertion, deletion or modification at one or more nucleotide positions. However, polynucleotides encoding wild-type polypeptides are not limited to naturally occurring polynucleotides, but also include any polynucleotides (for example, synthetic polynucleotides) encoding wild-type polypeptides.

As used herein, the term "TIGR4" refers to TIGR4 strain of *Streptococcus pneumoniae.* In some embodiments, an amino acid sequence of a wild-type IgA protease produced from *Streptococcus pneumoniae* TIGR4 strain is as set forth in SEQ ID NO: 1, wherein amino acids at positions 1 to 42 (underlined in SEQ ID NO: 1 below) are the signal peptide.

MEKYFGEKQERFSFRKLSVGLVSATISSLFFMSVLASSSVDAQETAGVHYKYVADSE
LSSEEKKQLVYDIPTYVENDDETYYLVYKLNSQNQLAELPNTGSKNERQALVAGAS
LAAMGILIFAVSKKKVKNKTVLHLVLVAGIGNGVLVSVHALENHLLLNYNTDYELT
SGEKLPLPKEISGYTYIGYIKEGKTTSESEVSNQKSSVATPTKQQKVDYNVTPNFVDH
PSTVQAIQEQTPVSSTKPTEVQVVEKPFSTELINPRKEEKQSSDSQEQLAEHKNLETKK
EEKISPKEKTGVNTLNPQDEVLSGQLNKPELLYREETMETKIDFQEEIQENPDLAEGT
VRVKQEGKLGKKVEIVRIFSVNKEEVSREIVSTSTTAPSPRIVEKGTKKTQVIKEQPET
GVEHKDVQSGAIVEPAIQPELPEAVVSDKGEPEVQPTLPEAVVTDKGETEVQPESPDT
VVSDKGEPEQVAPLPEYKGNIEQVKPETPVEKTKEQGPEKTEEVPVKPTEETPVNPNE
GTTEGTSIQEAENPVQPAEESTTNSEKVSPDTSSKNTGEVSSNPSDSTTSVGESNKPEH
NDSKNENSEKTVEEVPVNPNEGTVEGTSNQETEKPVQPAEETQTNSGKIANENTGEV
SNKPSDSKPPVEESNQPEKNGTATKPENSGNTTSENGQTEPEPSNGNSTEDVSTESNT
SNSNGNEEIKQENELDPDKKVEEPEKTLELRNVSDLELYSLSNGTYKQHISLEQVPSN
PNSYFVKVKSSSFKDVYLPVASISEERKNDKILYKITAKVEKLQQEIESRYKDNFTFYL
AKKGTEETTNFTSFSNLVKAINQNPSGTYHLAASLNANEVELGPDERSYIKDTFTGRL
IGEKDGKNYAIYNLKKPLFENLSGATVEKLSLKNVAISGKDDIGSLANEAQNNTKIKQ
VHVDGVLAGERGIGGLLAKAEQSSITESSFKGRIINTYETTAAYNIGGMVGHLTGDK
ALLTKSKATVAISSNTNTSDQTVGGLAGLVDRDAQIQDSYAEGDINNVKHFGRVAG
VAGNLWDRTSGDVRHAGSLTNVLSDVNVTNGNAITGYHYNEMKVKDTFSSKANRV
YNVTLVKDEVVSKESFEERGTMLDASQIASKKAEINPLILPTVEPLSTSGKKDSDFSK
VAYYQAKRNLTYKNIEKLLPFYNKATIVKYGNLVNENSLLYQKELLSAVMMKDNQ
VITDIVSNKQTANKLLLHYKDDLSEKLDLKYQNDFAKLAEYSLGNTGLLYTPNQFLY

DQTSIIKQVLPDLQKVDYHSEAIRKTLGISPNVKQTELYLEDQFAKTKQQLEDSLKKL
LSADAGLASANPVTEGYLVDKIKRNKEALLLGLTYLERWYNFSYGQVNVKDLVLY
HLDFFGKGNASPLDTLIELGKSGFNNLLAKNNVDTYGISLASQHGTTDLFSTLEHYRK
VFLPNTSNNDWFKSETKAYIVEEKSTIEEVKTKQGLAGTKYSIGVYDRITSATWKYR
NMVLPLLTLPERSVFVISTMSSLGFGAYDRYRSSDHKAGKALNDFVEENARETAKRQ
RDHYDYWYRILDDNAREKLYRNILLYDAYKFGDDNTVGKATEVADFDNPNPAMQH
FFGPVGNKVGHNQHGAYATGDAVYYMGYRMLDKDGAITYTHEMTHDSDQDIYLG
GYGRRSGLGPEFFAKGLLQAPDHPDDATITINSILKHSKSDSTESRRLQVLDPTTRFNN
ADDLKQYVHNMFDVVYMLEYLEGNSILKLDTNQKQQLLRKVTNEYHPDPDGNKVY
ATNVVRNLTVEEVERLRSFNDLIDNNILSSREYASGKYERNGYFTIKLFAPIYAALSN
DIGTPGDLMGRRIAYELLAAKGFKDGMVPYISNQYEEEAKQKGKTINLYGKTRGLV
TDDLVLEKVFNNQYHTWSEFKKAMYQERQDQFDRLNKVTFNDTTQPWQTFAKKTT
SSVDELQKLMDVAVRKDAEHNYYHWNNYNPDIDSEVHKLKRAIFKAYLDQTNDFR
SSIFENKK (SEQ ID NO: 1)

As used herein, the term "signal peptide" refers to the sequence of amino acid residues that can participate in the secretion or targeted transport of a protein in a matured form or a precursor form. Signal peptides are usually located at N-terminus of the sequence of a precursor or a matured protein. The signal peptide can be endogenous or exogenous. Generally, there is no signal peptide in matured protein. Usually, after a protein is transported, a signal peptide is cleaved from the protein by a signal peptidase. For example, the amino acid sequence as shown in SEQ ID NO: 22 is formed upon removing the N-terminal signal peptide from the amino acid sequence as set forth in SEQ ID NO: 1.

QETAGVHYKYVADSELSSEEKKQLVYDIPTYVENDDETYYLVYKLNSQNQLAELPN
TGSKNERQALVAGASLAAMGILIFAVSKKKVKNKTVLHLVLVAGIGNGVLVSVHAL
ENHLLLNYNTDYELTSGEKLPLPKEISGYTYIGYIKEGKTTSESEVSNQKSSVATPTKQ
QKVDYNVTPNFVDHPSTVQAIQEQTPVSSTKPTEVQVVEKPFSTELINPRKEEKQSSD
SQEQLAEHKNLETKKEEKISPKEKTGVNTLNPQDEVLSGQLNKPELLYREETMETKI
DFQEEIQENPDLAEGTVRVKQEGKLGKKVEIVRIFSVNKEEVSREIVSTSTTAPSPRIV
EKGTKKTQVIKEQPETGVEHKDVQSGAIVEPAIQPELPEAVVSDKGEPEVQPTLPEAV
VTDKGETEVQPESPDTVVSDKGEPEQVAPLPEYKGNIEQVKPETPVEKTKEQGPEKT
EEVPVKPTEETPVNPNEGTTEGTSIQEAENPVQPAEESTTNSEKVSPDTSSKNTGEVSS
NPSDSTTSVGESNKPEHNDSKNENSEKTVEEVPVNPNEGTVEGTSNQETEKPVQPAE
ETQTNSGKIANENTGEVSNKPSDSKPPVEESNQPEKNGTATKPENSGNTTSENGQTEP

EPSNGNSTEDVSTESNTSNSNGNEEIKQENELDPDKKVEEPEKTLELRNVSDLELYSL
SNGTYKQHISLEQVPSNPNSYFVKVKSSSFKDVYLPVASISEERKNDKILYKITAKVE
KLQQEIESRYKDNFTFYLAKKGTEETTNFTSFSNLVKAINQNPSGTYHLAASLNANEV
ELGPDERSYIKDTFTGRLIGEKDGKNYAIYNLKKPLFENLSGATVEKLSLKNVAISGK
DDIGSLANEAQNNTKIKQVHVDGVLAGERGIGGLLAKAEQSSITESSFKGRIINTYETT
AAYNIGGMVGHLTGDKALLTKSKATVAISSNTNTSDQTVGGLAGLVDRDAQIQDSY
AEGDINNVKHFGRVAGVAGNLWDRTSGDVRHAGSLTNVLSDVNVTNGNAITGYHY
NEMKVKDTFSSKANRVYNVTLVKDEVVSKESFEERGTMLDASQIASKKAEINPLILP
TVEPLSTSGKKDSDFSKVAYYQAKRNLTYKNIEKLLPFYNKATIVKYGNLVNENSLL
YQKELLSAVMMKDNQVITDIVSNKQTANKLLLHYKDDLSEKLDLKYQNDFAKLAE
YSLGNTGLLYTPNQFLYDQTSIIKQVLPDLQKVDYHSEAIRKTLGISPNVKQTELYLE
DQFAKTKQQLEDSLKKLLSADAGLASANPVTEGYLVDKIKRNKEALLLGLTYLERW
YNFSYGQVNVKDLVLYHLDFFGKGNASPLDTLIELGKSGFNNLLAKNNVDTYGISLA
SQHGTTDLFSTLEHYRKVFLPNTSNNDWFKSETKAYIVEEKSTIEEVKTKQGLAGTK
YSIGVYDRITSATWKYRNMVLPLLTLPERSVFVISTMSSLGFGAYDRYRSSDHKAGK
ALNDFVEENARETAKRQRDHYDYWYRILDDNAREKLYRNILLYDAYKFGDDNTVG
KATEVADFDNPNPAMQHFFGPVGNKVGHNQHGAYATGDAVYYMGYRMLDKDGAI
TYTHEMTHDSDQDIYLGGYGRRSGLGPEFFAKGLLQAPDHPDDATITINSILKHSKSD
STESRRLQVLDPTTRFNNADDLKQYVHNMFDVVYMLEYLEGNSILKLDTNQKQQLL
RKVTNEYHPDPDGNKVYATNVVRNLTVEEVERLRSFNDLIDNNILSSREYASGKYER
NGYFTIKLFAPIYAALSNDIGTPGDLMGRRIAYELLAAKGFKDGMVPYISNQYEEEAK
QKGKTINLYGKTRGLVTDDLVLEKVFNNQYHTWSEFKKAMYQERQDQFDRLNKVT
FNDTTQPWQTFAKKTTSSVDELQKLMDVAVRKDAEHNYYHWNNYNPDIDSEVHKL
KRAIFKAYLDQTNDFRSSIFENKK (SEQ ID NO: 22)

As used herein, the term "subject" includes both human and non-human animals. Non-human animals include all vertebrate animals, such as mammals and non-mammals. A "subject" may also be a domestic animal, such as cattle, pigs, sheep, poultry and horses; or a rodent, such as rats, mice; or a primate, such as apes, monkeys, chimpanzees, gorillas, orangutans, baboons; or domesticated animals, such as dogs and cats. A "subject" may be male or female and may be elderly, adult, adolescent, child or infant. Human "subject" may be Caucasian, African, Asian, Semitic, or other races or a combination of these ethnic backgrounds.

As used herein, the terms "protein", "polypeptide" and "peptide" are used interchangeably and refer to a polymer of amino acids. The protein, polypeptide or peptide described herein may contain naturally occurring amino acids, or may contain non-naturally occurring amino acids, or analogues or mimics of amino acids. The protein, polypeptide or peptide described herein may be obtained by any method known in the art, for example, but not limited to, by natural isolation, recombinant expression, chemical synthesis, and the like.

The term "amino acid" used herein refers to an organic compound containing amino (-NH₂) and carboxyl (-COOH) functional groups and a side chain specific to each amino acid. The names of amino acid are also represented in this application by standard single-letter or three-letter codes, which are summarized as follows:

| **Name** | **Three-letter code** | **Single-letter code** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

A "conservative substitution" with reference to amino acid sequence refers to replacing an amino acid residue with a different amino acid residue having a side chain with similar physiochemical properties. For example, conservative substitutions can be made among amino acid residues with hydrophobic side chains (e.g., Met, Ala, Val, Leu, and Ile), among residues with neutral hydrophilic side chains (e.g., Cys, Ser, Thr, Asn and Gln), among residues with acidic side chains (e.g., Asp, Glu), among amino acids with basic side chains *(e.g.,* His, Lys, and Arg), or among residues with aromatic side chains *(e.g.,* Trp, Tyr, and Phe). As known in the art, conservative substitution usually does not cause significant change in the protein conformational structure, and therefore could retain the biological activity of a protein.

As used herein, the term "homologous" refers to a nucleic acid sequence (or its complementary strand) or amino acid sequence having at least 60% (e.g., at least 65%, 70%, 75%, 80%, 85%, 88%, 90%, 91%, 92%, 93%, 94%, 95%, 96%, 97%, 98%, 99%) sequence identity to another sequence when optimally aligned.

As used herein, the term "percent (%) sequence identity" is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum number of identical amino acids (or nucleic acids). In other words, percent (%) sequence identity of an amino acid sequence (or nucleic acid sequence) can be calculated by dividing the number of amino acid residues (or bases) that are identical relative to the reference sequence to which it is being compared by the total number of the amino acid residues (or bases) in the candidate sequence or in the reference sequence, whichever is shorter. Conservative substitution of the amino acid residues may or may not be considered as identical residues. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al., J. Mol. Biol., 215:403-410 (1990); Stephen F. et al., Nucleic Acids Res., 25:3389-3402

(1997)), ClustalW2 (available on the website of European Bioinformatics Institute, see also, Higgins D.G. et al., Methods in Enzymology, 266:383-402 (1996); Larkin M.A. et al., Bioinformatics (Oxford, England), 23(21): 2947-8 (2007)), and ALIGN or Megalign (DNASTAR) software. Those skilled in the art may use the default parameters provided by the tool or may customize the parameters as appropriate for the alignment, such as for example, by selecting a suitable algorithm.

An "isolated" substance has been artificially altered from its natural state. If an "isolated" composition or substance occurs in nature, it has been altered or removed from its original state, or both. For example, naturally occurring polynucleotides or polypeptides in a living animal are not "isolated", but may be considered "isolated" if they are sufficiently separated from the substance with which they coexist in their natural state and exist in an essentially pure state. An "isolated nucleic acid sequence" refers to the sequence of the isolated nucleic acid molecule. In some embodiments, an "isolated truncated form of IgA protease" refers to a truncated form of IgA protease with a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99%, wherein the purity is determined by electrophoretic methods (e.g., SDS-PAGE, isoelectric focusing, capillary electrophoresis), or chromatographic methods *(e.g.,* ion exchange chromatography or reversed-phase HPLC).

The term "vector" as used herein refers to a vehicle into which a genetic element may be operably inserted so as to bring about the expression of that genetic element, such as to produce the protein encoded by the genetic element, RNA or DNA, or to replicate the genetic element. A vector may be used to transform, transduce, or transfect a host cell so as to bring about expression of the genetic element it carries within the host cell. Examples of vectors include plasmids, phagemids, cosmids, and artificial chromosomes such as yeast artificial chromosome (YAC), bacterial artificial chromosome (BAC), or P1-derived artificial chromosome (PAC), bacteriophages such as lambda phage or M13 phage, and animal viruses. A vector may contain a variety of elements for controlling expression, including a promoter sequence, a transcription initiation sequence, an enhancer sequence, a selectable element, and a reporter gene. In addition, the vector may further contain an origin of replication. A vector may also include materials to aid in its entry into the cell, including but not limited to a viral particle, a liposome, or a protein coating. A vector can be an expression vector or a cloning vector. The present disclosure provides vectors (e.g., expression vectors) comprising the nucleic acid sequence provided herein encoding the truncated form of IgA protease or the fusion protein, at least one promoter (e.g., SV40, CMV, EF-1α) operably linked to the nucleic acid sequence, and at least one selection marker.

As used herein, a "treatment" or "therapy" for a disease, disorder or condition comprises preventing or alleviating a disease, disorder or condition, reducing the rate of occurrence or progression of a disease, disorder or condition, reducing the risk of developing a disease, disorder or condition, preventing or delaying the development of symptoms associated with a disease, disorder or condition, reducing or terminating symptoms associated with a disease, disorder or condition, generating a complete or partial reversal of a disease, disorder or condition, and curing a disease, disorder or condition, or a combination of the above.

The term "pharmaceutically acceptable" indicates that the designated carrier, medium, diluent, excipient and/or salt is generally chemically and/or physically compatible with the other ingredients that constitute the formulation and physiologically compatible with the recipient thereof.

The term "disease associated with IgA deposition" refers to a disease associated with an aggregation of IgA immunoglobulin (in an aggregated or non-aggregated form) in a tissue or organ of a subject. For example, a disease associated with IgA deposition includes but is not limited to, IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis.

The term "IgA nephropathy" refers to a kidney disease characterized by IgA deposition in the kidney.

### Truncated Forms of IgA Protease

In one aspect, the present disclosure provides an isolated truncated form of IgA protease comprising a non-natural truncated fragment of a wild-type IgA protease obtained from or derived from *Streptococcus pneumoniae* TIGR4 strain, or having at least 70% sequence identity (e.g., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the non-natural truncated fragment. In some embodiments, the truncated form of IgA protease having at least 70% sequence identity (e.g., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the non-natural truncated fragment still retains the function or activity (such as, proteolytic activity or enzymatic activity of specifically cleaving IgA) of IgA protease.

As used herein, the term "truncated form" or "truncated fragment" refers to a peptide formed by removing one or more amino acids from one or both ends of a wild-type polypeptide. Thus, a "truncated form" or "truncated fragment" described herein does not include the full length of the corresponding wild-type polypeptide, but may have one or more amino acid mutation, deletion, insertion or modification *etc.* compared to the truncated form of the wild-type polypeptide. For example, a "truncated form of IgA protease" or "truncated fragment of IgA protease" may comprise a peptide formed by removing one or more amino acids from one or both ends of a wild-type IgA protease, and may also comprise a peptide with one or more amino acids mutations, deletions, insertions or modifications compared to the truncated form of the wild-type IgA protease.

In some embodiments, the truncated form of IgA protease described herein has one or more amino acid mutations, deletions, insertions or modifications compared to its corresponding wild-type IgA protease. For example, in some embodiments, the truncated form of IgA protease described herein comprises a non-natural truncated fragment of a wild-type IgA protease obtained from or derived from *Streptococcus pneumoniae* TIGR4 strain, and the non-natural truncated fragment has an amino acid mutation, deletion, insertion or modification compared to the wild-type IgA protease of *Streptococcus pneumoniae* TIGR4 strain, such that the truncated form of IgA protease loses or reduces its self-cleaving function.

The terms "obtained from" and "derived from" used herein include not only protein produced or producible by the mentioned organisms, but also include protein encoded by DNA sequences isolated from such organisms and produced in host organisms containing such DNA sequences, and also include protein encoded by synthetic and/or cDNA-derived DNA sequences and having the identification characteristics of the said protein. For example, wild-type IgA protease obtained from or derived from *Streptococcus pneumoniae* TIGR4 strain includes both IgA protease naturally produced by *Streptococcus pneumoniae* TIGR4 strain and IgA protease produced by other host cells (e.g., *Escherichia coli*) transformed with nucleic acid encoding IgA protease using genetically engineering technology.

As used herein, the term "non-natural truncated fragment" refers to a fragment with an amino acid sequence that is different (*e.g*., different amino acid length, different amino acid type, *etc.)* from the amino acid sequence of the truncated fragment formed by self-cleavage of the wild-type IgA protease of *Streptococcus pneumoniae* TIGR4 strain in natural environment.

In some embodiments, the amino acid mutation, deletion, insertion or modification occurs at a natural self-cleaving site of the wild-type IgA protease of *Streptococcus pneumoniae* TIGR4 strain.

The term "natural self-cleaving site" means that an IgA protease can recognize certain specific peptide bonds of the IgA protease *per se* to carry out autocatalytic cleavage, thus releasing an IgA protease with matured C-terminus.

In some embodiments, the amino acid mutation, deletion, insertion or modification occurs within 5 amino acid residues (e.g., 1 amino acid residue, 2 amino acid residues, 3 amino acid residues, 4 amino acid residues or 5 amino acid residues) upstream of the natural self-cleaving site of the wild-type IgA protease of *Streptococcus pneumoniae* TIGR4 strain. In some embodiments, the amino acid mutation, deletion, insertion or modification occurs within 5 amino acid residues (*e.g*., 1 amino acid residue, 2 amino acid residues, 3 amino acid residues, 4 amino acid residues or 5 amino acid residues) downstream of the natural self-cleaving site of the wild-type IgA protease of *Streptococcus pneumoniae* TIGR4 strain. In some embodiments, the amino acid mutation or deletion occurs within 5 amino acid residues *(e.g.,* 1 amino acid residue, 2 amino acid residues, 3 amino acid residues, 4 amino acid residues or 5 amino acid residues) upstream of the natural self-cleaving site and within 5 amino acid residues (*e.g*., 1 amino acid residue, 2 amino acid residues, 3 amino acid residues, 4 amino acid residues or 5 amino acid residues) downstream of the natural self-cleaving site of the wild-type IgA protease of *Streptococcus pneumoniae* TIGR4 strain.

In some embodiments, the amino acid mutation, deletion, insertion or modification occurs at one or more amino acid positions of the wild-type IgA protease of *Streptococcus pneumoniae* TIGR4 strain (as set forth in SEQ ID NO: 1), the one or more amino acid positions is selected from the group consisting of: position 71, position 97, position 175, position 177, position 210, position 222, position 228, position 239, position 245, position 254, position 262, position 292, position 303, position 315, position 338, position 382, position 384, position 401, position 418, position 425, position 435, position 439, position 442, position 456, position 459, position 469, position 474, position 476, position 487, position 490, position 499, position 506, position 509, position 514, position 517, position 533, position 536, position 549, position 563, position 576, position 594, position 597, position 613, position 616, position 639, position 644, position 645, position 652 and position 661.

In some embodiments, the non-natural truncated fragment is a N-terminal or C-terminal truncated fragment of the wild-type IgA protease obtained from or derived from the *Streptococcus pneumoniae* TIGR4 strain.

As used herein, the term "N-terminal truncated fragment" refers to a truncated fragment comprising an amino acid sequence of the amino terminus of a wild-type IgA protease of *Streptococcus pneumoniae* TIGR4 strain. The starting position of the "amino terminus" can be any position near the amino terminus of the amino acid sequence of the wild-type IgA protease of *Streptococcus pneumoniae* TIGR4 strain, for example, it can be position 1 from the amino terminus or other positions from the amino terminus. For another example, if the full-length amino acid sequence of a wild-type IgA protease consists of 1000 amino acids, the starting position of the amino terminus of its N-terminal truncated fragment can be any position from the position 1 to position 500 of its amino acid sequence starting from the amino terminus.

In some embodiments, the N-terminal starting site of the non-natural truncated fragment of the IgA protease described herein is within 5 amino acid residues (e.g., 1 amino acid residue, 2 amino acid residues, 3 amino acid residues, 4 amino acid residues or 5 amino acid residues) upstream of the natural self-cleaving site of the wild-type IgA protease of *Streptococcus pneumoniae* TIGR4 strain. In some embodiments, the N-terminal starting site of the non-natural truncated fragment of IgA protease described herein is within 5 amino acid residues (*e.g.*, 1 amino acid residue, 2 amino acid residues, 3 amino acid residues, 4 amino acid residues or 5 amino acid residues) downstream of the natural self-cleaving site of the wild-type IgA protease of *Streptococcus pneumoniae* TIGR4 strain.

In some embodiments, the N-terminal starting site of the non-natural truncated fragment of IgA protease is the natural self-cleaving site (*e.g*., corresponding to position 71, position 97, position 175, position 177, position 210, position 222, position 228, position 239, position 245, position 254, position 262, position 292, position 303, position 315, position 338, position 382, position 384, position 401, position 418, position 425, position 435, position 439, position 442, position 456, position 459, position 469, position 474, position 476, position 487, position 490, position 499, position 506, position 509, position 514, position 517, position 533, position 536, position 549, position 563, position 576, position 594, position 597, position 613, position 616, position 639, position 644, position 645, position 652 or position 661 of the amino acid sequence as set forth in SEQ ID NO: 1) of wild-type IgA protease of *Streptococcus pneumoniae* TIGR4 strain. In some embodiments, the N-terminal starting site of the non-natural truncated fragment of IgA protease is at 1 amino acid residue downstream of the natural self-cleaving site (*e.g.*, corresponding to position 72, position 98, position 176, position 178, position 211, position 223, position 229, position 240, position 246, position 255, position 263, position 293, position 304, position 316, position 339, position 383, position 385, position 402, position 419, position 426, position 436, position 440, position 443, position 457, position 460, position 470, position 475, position 477, position 488, position 491, position 500, position 507, position 510, position 515, position 518, position 534, position 537, position 550, position 564, position 577, position 595, position 598, position 614, position 617, position 640, position 645, position 646, position 653 or position 662 of the amino acid sequence as set forth in SEQ ID NO: 1) of wild-type IgA protease of *Streptococcus pneumoniae* TIGR4 strain.

As used herein, the term "C-terminal truncated fragment" refers to a truncated fragment comprising an amino acid sequence of the carboxyl terminus of a wild-type IgA protease of *Streptococcus pneumoniae* TIGR4 strain. The ending position of the "carboxyl terminus" can be any position near the carboxyl terminus of the amino acid sequence of the wild-type IgA protease of Streptococcus pneumoniae TIGR4 strain, for example, it can be position 1 from the carboxyl terminus or other positions from the carboxyl terminus. For another example, if the full-length amino acid sequence of wild-type IgA protease consists of 1000 amino acids, the ending position of the carboxyl terminus of its C-terminal truncated fragment can be any position from the position 501 to position 1000 of its amino acid sequence starting from the amino terminus.

In some embodiments, the deposit number of *Streptococcus pneumoniae* TIGR4 is ATCC BAA-334.

In some embodiments, the C-terminal truncated fragment comprises a polypeptide fragment of at least 703 *(e.g.,* at least 710, at least 750, at least 800, at least 850, at least 900, at least 950, at least 1000, at least 1050, at least 1100, at least 1150, at least 1200, at least 1250, at least 1300, at least 1350, at least 1400, at least 1450, at least 1500, at least 1550, at least 1600, at least 1650, at least 1700, at least 1750, at least 1800, at least 1850, at least 1900, at least 1910, at least 1920, at least 1930, at least 1940, at least 1950, at least 1960, at least 1961, or at least 1962) continuous amino acids starting from position 43 of the wild-type IgA protease obtained from or derived from the *Streptococcus pneumoniae* TIGR4 strain, or having at least 70% *(e.g.,* at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%) sequence identity to the polypeptide fragment. In some embodiments, the N-terminal truncated fragment having at least 70% sequence identity (*e.g*., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the polypeptide fragment still retain the function or activity (such as, proteolytic activity or enzymatic activity of specifically cleaving IgA) of IgA protease. In some embodiments, the amino acid sequence of the wild-type IgA protease of *Streptococcus pneumoniae* TIGR4 strain is as set forth in SEQ ID NO: 1.

Unless otherwise specified, the amino acid position of TIGR4 IgA protease described herein is the amino acid position corresponding to wild-type TIGR4 IgA protease (the amino acid sequence of the wild-type TIGR4 IgA protease is as set forth in SEQ ID NO: 1). For example, position 665 of the TIGR4 IgA protease described herein corresponds to position 665 of the amino acid sequence as set forth in SEQ ID NO: 1. Unless otherwise specified, the naming rule of the truncated form of the TIGR4 IgA protease is TIGR4 (start position corresponding to SEQ ID NO: 1 - end position corresponding to SEQ ID NO: 1). For example, TIGR4 (665-2004) refers to the truncated form of TIGR4 IgA protease formed by amino acids from position 665 to position 2004 of the amino acid sequence as set forth in

### SEQ ID NO: 1.

In some embodiments, the natural self-cleaving site of the IgA protease described herein is between position 43 and position 745 of the amino acid sequence as set forth in SEQ ID NO: 1. In some embodiments, the natural self-cleaving site of the IgA protease described herein is between position 43 and position 665 of the amino acid sequence as set forth in SEQ ID NO: 1. In some embodiments, the natural self-cleaving site is between position 71 and position 72, between position 210 and position 211, between position 228 and position 229, between position 239 and position 240, between position 245 and position 246, between position 382 and position 383, between position 418 and position 419, between position 439 and position 440, between position 490 and position 491, between position 506 and position 507, between position 509 and position 510, between position 514 and position 515, between position 533 and position 534, between position 536 and position 537, between position 563 and position 564, between position 594 and position 595, between position 613 and position 614, between position 616 and position 617, between position 639 and position 640, between position 645 and position 646, or between position 678 and position 679 of the amino acid sequence as set forth in SEQ ID NO: 1.

In some embodiments, the truncated form of IgA protease comprises a polypeptide fragment of at least 1300 continuous amino acids starting from position 665 of the amino acid sequence as set forth in SEQ ID NO: 1. For example, in some embodiments, the truncated form of IgA protease described herein comprises a polypeptide fragment of at least 1310, at least 1320, at least 1321, at least 1322, at least 1323, at least 1324, at least 1325, at least 1326, at least 1327, at least 1328, at least 1329, at least 1330, at least 1331, at least 1332, at least 1333, at least 1334, at least 1335, at least 1336, at least 1337, at least 1338, at least 1339, or at least 1340 continuous amino acids starting from position 665 of the amino acid sequence as set forth in SEQ ID NO: 1. In some embodiments, the truncated form of IgA protease provided herein comprises a polypeptide fragment of 1340 continuous amino acids starting from position 665 of the amino acid sequence as set forth in SEQ ID NO: 1.

In some embodiments, the truncated form of IgA protease comprises a polypeptide fragment of amino acids starting from position 665 to position 2004 of the amino acid sequence as set forth in SEQ ID NO: 1, or having at least 70% *(e.g.,* at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%) sequence identity to the polypeptide fragment. In some embodiments, the truncated form of IgA protease having at least 70% sequence identity (e.g., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the polypeptide fragment still retain the function or activity (such as, proteolytic activity or enzymatic activity of specifically cleaving IgA) of IgA protease.

In some embodiments, the truncated form of IgA protease described herein refers to a truncated form of TIGR4 (665-2004) whose amino acid sequence is as set forth in SEQ ID NO: 2.

GNTTSENGQTEPEPSNGNSTEDVSTESNTSNSNGNEEIKQENELDPDKKVEEPEKTLE
LRNVSDLELYSLSNGTYKQHISLEQVPSNPNSYFVKVKSSSFKDVYLPVASISEERKN
DKILYKITAKVEKLQQEIESRYKDNFTFYLAKKGTEETTNFTSFSNLVKAINQNPSGT
YHLAASLNANEVELGPDERSYIKDTFTGRLIGEKDGKNYAIYNLKKPLFENLSGATV
EKLSLKNVAISGKDDIGSLANEAQNNTKIKQVHVDGVLAGERGIGGLLAKAEQSSITE
SSFKGRIINTYETTAAYNIGGMVGHLTGDKALLTKSKATVAISSNTNTSDQTVGGLA
GLVDRDAQIQDSYAEGDINNVKHFGRVAGVAGNLWDRTSGDVRHAGSLTNVLSDV
NVTNGNAITGYHYNEMKVKDTFSSKANRVYNVTLVKDEVVSKESFEERGTMLDAS
QIASKKAEINPLILPTVEPLSTSGKKDSDFSKVAYYQAKRNLTYKNIEKLLPFYNKATI
VKYGNLVNENSLLYQKELLSAVMMKDNQVITDIVSNKQTANKLLLHYKDDLSEKL
DLKYQNDFAKLAEYSLGNTGLLYTPNQFLYDQTSIIKQVLPDLQKVDYHSEAIRKTL
GISPNVKQTELYLEDQFAKTKQQLEDSLKKLLSADAGLASANPVTEGYLVDKIKRNK
EALLLGLTYLERWYNFSYGQVNVKDLVLYHLDFFGKGNASPLDTLIELGKSGFNNLL
AKNNVDTYGISLASQHGTTDLFSTLEHYRKVFLPNTSNNDWFKSETKAYIVEEKSTIE
EVKTKQGLAGTKYSIGVYDRITSATWKYRNMVLPLLTLPERSVFVISTMSSLGFGAY
DRYRSSDHKAGKALNDFVEENARETAKRQRDHYDYWYRILDDNAREKLYRNILLY
DAYKFGDDNTVGKATEVADFDNPNPAMQHFFGPVGNKVGHNQHGAYATGDAVYY
MGYRMLDKDGAITYTHEMTHDSDQDIYLGGYGRRSGLGPEFFAKGLLQAPDHPDD
ATITINSILKHSKSDSTESRRLQVLDPTTRFNNADDLKQYVHNMFDVVYMLEYLEGN
SILKLDTNQKQQLLRKVTNEYHPDPDGNKVYATNVVRNLTVEEVERLRSFNDLIDN
NILSSREYASGKYERNGYFTIKLFAPIYAALSNDIGTPGDLMGRRIAYELLAAKGFKD
GMVPYISNQYEEEAKQKGKTINLYGKTRGLVTDDLVLEKVFNNQYHTWSEFKKAM
YQERQDQFDRLNKVTFNDTTQPWQTFAKKTTSSVDELQKLMDVAVRKDAEHNYYH
WNNYNPDIDSEVHKLKRAIFKAYLDQTNDFRSSIFENKK (SEQ ID NO: 2)

In some embodiments, the truncated form of IgA protease described herein refers to a truncated form of TIGR4 (745-2004) whose amino acid sequence is as set forth in SEQ ID NO: 3.

LEQVPSNPNSYFVKVKSSSFKDVYLPVASISEERKNDKILYKITAKVEKLQQEIESRYK
DNFTFYLAKKGTEETTNFTSFSNLVKAINQNPSGTYHLAASLNANEVELGPDERSYIK
DTFTGRLIGEKDGKNYAIYNLKKPLFENLSGATVEKLSLKNVAISGKDDIGSLANEAQ
NNTKIKQVHVDGVLAGERGIGGLLAKAEQSSITESSFKGRIINTYETTAAYNIGGMVG
HLTGDKALLTKSKATVAISSNTNTSDQTVGGLAGLVDRDAQIQDSYAEGDINNVKHF
GRVAGVAGNLWDRTSGDVRHAGSLTNVLSDVNVTNGNAITGYHYNEMKVKDTFSS

KANRVYNVTLVKDEVVSKESFEERGTMLDASQIASKKAEINPLILPTVEPLSTSGKKD
SDFSKVAYYQAKRNLTYKNIEKLLPFYNKATIVKYGNLVNENSLLYQKELLSAVMM
KDNQVITDIVSNKQTANKLLLHYKDDLSEKLDLKYQNDFAKLAEYSLGNTGLLYTP
NQFLYDQTSIIKQVLPDLQKVDYHSEAIRKTLGISPNVKQTELYLEDQFAKTKQQLED
SLKKLLSADAGLASANPVTEGYLVDKIKRNKEALLLGLTYLERWYNFSYGQVNVKD
LVLYHLDFFGKGNASPLDTLIELGKSGFNNLLAKNNVDTYGISLASQHGTTDLFSTLE
HYRKVFLPNTSNNDWFKSETKAYIVEEKSTIEEVKTKQGLAGTKYSIGVYDRITSAT
WKYRNMVLPLLTLPERSVFVISTMSSLGFGAYDRYRSSDHKAGKALNDFVEENARE
TAKRQRDHYDYWYRILDDNAREKLYRNILLYDAYKFGDDNTVGKATEVADFDNPN
PAMQHFFGPVGNKVGHNQHGAYATGDAVYYMGYRMLDKDGAITYTHEMTHDSD
QDIYLGGYGRRSGLGPEFFAKGLLQAPDHPDDATITINSILKHSKSDSTESRRLQVLDP
TTRFNNADDLKQYVHNMFDVVYMLEYLEGNSILKLDTNQKQQLLRKVTNEYHPDP
DGNKVYATNVVRNLTVEEVERLRSFNDLIDNNILSSREYASGKYERNGYFTIKLFAPI
YAALSNDIGTPGDLMGRRIAYELLAAKGFKDGMVPYISNQYEEEAKQKGKTINLYG
KTRGLVTDDLVLEKVFNNQYHTWSEFKKAMYQERQDQFDRLNKVTFNDTTQPWQT
FAKKTTSSVDELQKLMDVAVRKDAEHNYYHWNNYNPDIDSEVHKLKRAIFKAYLD
QTNDFRSSIFENKK (SEQ ID NO: 3)

In some embodiments, the truncated form of IgA protease described herein refers to a truncated form of TIGR4 (845-2004) whose amino acid sequence is as set forth in SEQ ID NO: 4.

LNANEVELGPDERSYIKDTFTGRLIGEKDGKNYAIYNLKKPLFENLSGATVEKLSLKN
VAISGKDDIGSLANEAQNNTKIKQVHVDGVLAGERGIGGLLAKAEQSSITESSFKGRII
NTYETTAAYNIGGMVGHLTGDKALLTKSKATVAISSNTNTSDQTVGGLAGLVDRDA
QIQDSYAEGDINNVKHFGRVAGVAGNLWDRTSGDVRHAGSLTNVLSDVNVTNGNA
ITGYHYNEMKVKDTFSSKANRVYNVTLVKDEVVSKESFEERGTMLDASQIASKKAEI
NPLILPTVEPLSTSGKKDSDFSKVAYYQAKRNLTYKNIEKLLPFYNKATIVKYGNLVN
ENSLLYQKELLSAVMMKDNQVITDIVSNKQTANKLLLHYKDDLSEKLDLKYQNDFA
KLAEYSLGNTGLLYTPNQFLYDQTSIIKQVLPDLQKVDYHSEAIRKTLGISPNVKQTE
LYLEDQFAKTKQQLEDSLKKLLSADAGLASANPVTEGYLVDKIKRNKEALLLGLTY
LERWYNFSYGQVNVKDLVLYHLDFFGKGNASPLDTLIELGKSGFNNLLAKNNVDTY
GISLASQHGTTDLFSTLEHYRKVFLPNTSNNDWFKSETKAYIVEEKSTIEEVKTKQGL
AGTKYSIGVYDRITSATWKYRNMVLPLLTLPERSVFVISTMSSLGFGAYDRYRSSDH
KAGKALNDFVEENARETAKRQRDHYDYWYRILDDNAREKLYRNILLYDAYKFGDD

NTVGKATEVADFDNPNPAMQHFFGPVGNKVGHNQHGAYATGDAVYYMGYRMLD
KDGAITYTHEMTHDSDQDIYLGGYGRRSGLGPEFFAKGLLQAPDHPDDATITINSILK
HSKSDSTESRRLQVLDPTTRFNNADDLKQYVHNMFDVVYMLEYLEGNSILKLDTNQ
KQQLLRKVTNEYHPDPDGNKVYATNVVRNLTVEEVERLRSFNDLIDNNILSSREYAS
GKYERNGYFTIKLFAPIYAALSNDIGTPGDLMGRRIAYELLAAKGFKDGMVPYISNQ
YEEEAKQKGKTINLYGKTRGLVTDDLVLEKVFNNQYHTWSEFKKAMYQERQDQFD
RLNKVTFNDTTQPWQTFAKKTTSSVDELQKLMDVAVRKDAEHNYYHWNNYNPDID
SEVHKLKRAIFKAYLDQTNDFRSSIFENKK (SEQ ID NO: 4)

In some embodiments, the truncated form of IgA protease has an amino acid conservative substitution at one or more amino acid residues (e.g., at 1, 2, 3, 4, 5 or more amino acid residues) compared to the amino acid sequence of the polypeptide fragment. An amino acid conservative substitution refers to a substitution between amino acids with similar properties, for example, between polar amino acids (e.g., between glutamine and asparagine), between hydrophobic amino acids (e.g., between leucine, isoleucine, methionine and valine) and between amino acids with the same charge (e.g., between arginine, lysine and histidine, or substitutions between glutamic acid and aspartic acid), *etc.* In some embodiments, the truncated form of IgA protease described herein has an amino acid conservative substitution at 1, 2, 3, 4, 5, 6, 7, 8, 9, 10, 11, 15, 20 or more amino acid residues compared to the amino acid sequence as set forth in SEQ ID NO: 2, SEQ ID NO: 3 or SEQ ID NO: 4.

Provided that activity is not compromised, the truncated form of IgA protease described herein may also comprise non-natural amino acids. Non-natural amino acids comprise, for example, β-fluorosubstituted alanine, 1-methylhistidine, γ-methylene glutamic acid, α-methylleucine, 4,5-dehydrolysine, hydroxyproline, 3-fluorosubstituted phenylalanine, 3-amino-tyrosine, 4-methyltryptophan and the like.

The truncated form of IgA protease described herein can also be modified using methods well known in the art. Examples include, but are not limited to, PEGylation, glycosylation, amino-terminal modification, fatty acylation, carboxy-terminal modification, phosphorylation, methylation and the like. A person skilled in the art shall understand that after modification using methods well known in the art, the truncated form of IgA protease provided herein still retains substantially similar functions to IgA protease or the truncated form of IgA protease.

In some embodiments, the truncated form of IgA protease described herein has an enzymatic activity of specifically cleaving human IgA. In some embodiments, the truncated form of IgA protease described herein has an enzymatic activity of specifically cleaving human IgA heavy chain. In some embodiments, the truncated form of IgA protease described herein has an enzymatic activity of specifically cleaving human IgA heavy chain hinge region. In some embodiments, the truncated form of IgA protease described herein has an enzymatic activity of specifically cleaving human IgA1. In some embodiments, the truncated form of IgA protease has an enzymatic activity of specifically cleaving human IgA1 heavy chain. In some embodiments, the truncated form of IgA protease has an enzymatic activity of specifically cleaving human IgA1 heavy chain hinge region.

In some embodiments, the truncated form of IgA protease described herein has an amino acid conservative substitution at one or more amino acid residues compared to the amino acid sequence of the polypeptide fragment, but retains an enzymatic activity of cleaving human IgA *(e.g.,* IgA1). In some embodiments, the truncated form of IgA protease described herein has at least 70% *(e.g.,* at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%) sequence identity to the polypeptide fragment, and retains an enzymatic activity of cleaving human IgA *(e.g.,* IgA1).

### Fusion Proteins

In another aspect, the present disclosure provides a fusion protein comprising a first polypeptide and a second polypeptide, wherein the first polypeptide comprises the full length of a wild-type IgA protease obtained from or derived from *Streptococcus pneumoniae* TIGR4 strain, a polypeptide formed by removing a signal peptide from the wild-type IgA protease obtained from or derived from *Streptococcus pneumoniae* TIGR4 strain or the truncated form of IgA protease described herein; and the second polypeptide cannot be cleaved by the IgA protease or the truncated form of IgA protease. Without being limited by any theory, it is considered that the second polypeptide not being able to be cleaved by the IgA protease or the truncated form of IgA protease is beneficial, because in such case both the integrity and stability of the fusion protein and the activity of the first polypeptide for cleaving IgA can be maintained.

In some embodiments, the first polypeptide and the second polypeptide of the fusion protein described herein are linked via a linker. In some embodiments, the first polypeptide and the second polypeptide are directly linked to each other (*i.e.,* linked without a linker). As used herein, the term "linker" refers to an artificial amino acid sequence having 1, 2, 3, 4 or 5 amino acid residues, or between 5 and 15, 20, 30, 50 or more amino acid residues in length, linked by a peptide bond and used to link one or more polypeptides. The linker may or may not have a secondary structure. Linker sequences are known in the art, for example, see Holliger et al., Proc. Natl. Acad. Sci. USA 90:6444-6448 (1993); Poljak et al., Structure 2:1121-1123 (1994).

In some embodiments, the linker is selected from the group consisting of a cleavable linker, a non-cleavable linker, a peptide linker, a flexible linker, a rigid linker, a helical linker and a non-helical linker. Any suitable linker known in the art can be used. In some embodiments, the linker comprises a peptide linker. For example, useful linkers in the present disclosure may be rich in glycine and serine residues. Examples include linkers having single or repeated sequences comprising threonine/serine and glycine, such as GGGS (SEQ ID NO: 15), GGGGS (SEQ ID NO: 16), GGGGGGS (SEQ ID NO: 17) or GSS (SEQ ID NO: 19), or tandem repeats (e.g., 2, 3, 4, 5, 6, 7, 8, 9, 10 or more repeats) thereof. In some embodiment, the linker as used herein comprises (GGGGSGGGGSGGGGS (SEQ ID NO: 18). In some embodiment, the linker as used herein comprises GSSGSSG (SEQ ID NO: 20). In some embodiment, the linker as used herein comprises RSGSSGSSG (SEQ ID NO: 21). In some embodiment, the linker as used herein comprises RSGGGGS (SEQ ID NO: 31). In some embodiments, the linker as used herein comprises or consists of an amino acid sequence selected from the group consisting of: amino acid sequences having at least 80%, at least 85%, at least 90%, at least 91%, at least 92%, at least 93%, at least 94%, at least 95%, at least 96%, at least 97%, at least 98%, at least 99% sequence identity to the amino acid sequence as set forth in any one of SEQ ID NOs: 18, 20, 21 and 31.

In some embodiments, the second polypeptide comprises an amino acid sequence for extending half-life of the first polypeptide in a subject. In some embodiments, the second polypeptide is selected from an Fc domain and albumin. In some embodiments, the Fc domain comprises a hinge region. In some embodiments, the Fc domain comprises a lower hinge region. In some embodiments, the Fc domain comprises a core hinge region and a lower hinge region. In some embodiments, the Fc domain comprises an upper hinge region, a core hinge region and a lower hinge region. In some embodiments, the Fc domain does not comprise a hinge region. In some embodiments, the Fc domain is derived from human IgG Fc domain. In some embodiments, the Fc domain is derived from human IgG1 Fc domain, human IgG2 Fc domain, human IgG3 Fc domain or human IgG4 Fc domain.

In some embodiments, the Fc domain is derived from human IgG1 Fc domain. In some embodiments, the Fc domain comprises an amino acid sequence as set forth in SEQ ID NO: 13. In some embodiments, the Fc domain consists of an amino acid sequence as set forth in SEQ ID NO: 13. In some embodiments, the amino acid sequence of the Fc domain has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 13.

TCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK (SEQ
ID NO: 13)

In some embodiments, the Fc domain is derived from human IgG4 Fc domain. In some embodiments, the Fc domain comprises an amino acid sequence as set forth in SEQ ID NO: 14. In some embodiments, the Fc domain consists of an amino acid sequence as set forth in SEQ ID NO: 14. In some embodiments, the amino acid sequence of the Fc domain has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 14.

ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLG
K (SEQ ID NO: 14)

Inventors of the present disclosure unexpectedly discovered that, although both the fusion protein formed by the truncated form of TIGR4 IgA protease and the IgG4 Fc domain and the fusion protein formed by the truncated form of TIGR4 IgA protease and the IgG1 Fc domain had enzymatic activities of cleaving IgA, the stability of the fusion protein formed by the truncated form of TIGR4 IgA protease and the IgG4 Fc domain was superior to that of the fusion protein formed by the truncated form of TIGR4 IgA protease and the IgG1 Fc domain. In some embodiments, the half-life of the fusion protein formed by the truncated form of TIGR4 IgA protease and the IgG4 Fc domain is at least 10 hours (h), at least 12 h, at least 24 h, at least 36 h, at least 48 h, at least 60 h, at least 72 h, at least 84 h or at least 96 h longer than that of the fusion protein formed by the truncated form of TIGR4 IgA protease and the IgG1 Fc domain.

In some embodiments, the Fc domain comprises one or more mutations that extend half-life of the fusion protein. In some embodiments, the Fc domain is linked to C-terminus of the first polypeptide. In some embodiments, the Fc domain is linked to N-terminus of the first polypeptide.

In some embodiments, the second polypeptide is albumin. In some embodiments, the albumin comprises an amino acid sequence as set forth in SEQ ID NO: 23. In some embodiments, the albumin comprises one or more domains of human serum albumin. In some embodiments, the albumin comprises a D3 domain of human serum albumin.











 (SEQ ID NO: 23)

In some embodiments, the fusion protein provided herein further comprises a label. In some embodiments, the label is selected from the group consisting of a fluorescent label, a luminescent label, a purification label and a chromogenic label. In some embodiments, the label is selected from the group consisting of a c-Myc tag, an HA tag, a VSV-G tag, a FLAG tag, a V5 tag and a HIS tag. In some embodiments, the label is a HIS tag. In some embodiments, the label is a HIS tag comprising 6, 7, 8, 9, 10 or more histidine. In some embodiments, the second polypeptide is located at C-terminus of the first polypeptide and the label is located at C-terminus of the second polypeptide.

In some embodiments, the present disclosure provides a fusion protein, which comprises a first polypeptide and a second polypeptide, wherein the first polypeptide comprises an amino acid sequence as set forth in SEQ ID NO: 2, SEQ ID NO: 3 or SEQ ID NO: 4, and the second polypeptide comprises an amino acid sequence as set forth in SEQ ID NO: 13 or SEQ ID NO: 14.

In some embodiments, the present disclosure provides a fusion protein, which comprises a first polypeptide and a second polypeptide, wherein the first polypeptide comprises an amino acid sequence as set forth in SEQ ID NO: 2, and the second polypeptide comprises an amino acid sequence as set forth in SEQ ID NO: 13. In some embodiments, the present disclosure provides a fusion protein, which comprises a first polypeptide and a second polypeptide, wherein the first polypeptide consists of an amino acid sequence as set forth in SEQ ID NO: 2, and the second polypeptide consists of an amino acid sequence as set forth in SEQ ID NO: 13. In some embodiments, the present disclosure provides a fusion protein, which comprises a first polypeptide and a second polypeptide, wherein the first polypeptide consists of an amino acid sequence as set forth in SEQ ID NO: 2, the second polypeptide consists of an amino acid sequence as set forth in SEQ ID NO: 13, and the first polypeptide is at the N-terminus of the second polypeptide. In some embodiments, the present disclosure provides a fusion protein, which comprises a first polypeptide and a second polypeptide, wherein the first polypeptide consists of an amino acid sequence as set forth in SEQ ID NO: 2, the second polypeptide consists of an amino acid sequence as set forth in SEQ ID NO: 13, and the first polypeptide is at the C-terminus of the second polypeptide.

In some embodiments, the present disclosure provides a fusion protein, which comprises a first polypeptide and a second polypeptide, wherein the first polypeptide comprises an amino acid sequence as set forth in SEQ ID NO: 2, and the second polypeptide comprises an amino acid sequence as set forth in SEQ ID NO: 14. In some embodiments, the present disclosure provides a fusion protein, which comprises a first polypeptide and a second polypeptide, wherein the first polypeptide consists of an amino acid sequence as set forth in SEQ ID NO: 2, and the second polypeptide consists of an amino acid sequence as set forth in SEQ ID NO: 14. In some embodiments, the present disclosure provides a fusion protein, which comprises a first polypeptide and a second polypeptide, wherein the first polypeptide consists of an amino acid sequence as set forth in SEQ ID NO: 2, the second polypeptide consists of an amino acid sequence as set forth in SEQ ID NO: 14, and the first polypeptide is at the N-terminus of the second polypeptide. In some embodiments, the present disclosure provides a fusion protein, which comprises a first polypeptide and a second polypeptide, wherein the first polypeptide consists of an amino acid sequence as set forth in SEQ ID NO: 2, the second polypeptide consists of an amino acid sequence as set forth in SEQ ID NO: 14, and the first polypeptide is at the C-terminus of the second polypeptide.

In some embodiments, the fusion protein described herein comprises an amino acid sequence as set forth in SED ID NO: 5. In some embodiments, the fusion protein as described herein consists of an amino acid sequence as set forth in SED ID NO: 5, or an amino acid sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 5. In some embodiments, the fusion protein having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 5 still retain the function or activity (such as, proteolytic activity or enzymatic activity of specifically cleaving IgA) of IgA protease.

TCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGKGGG
GGGSRQETAGVHYKYVADSELSSEEKKQLVYDIPTYVENDDETYYLVYKLNSQNQL
AELPNTGSKNERQALVAGASLAAMGILIFAVSKKKVKNKTVLHLVLVAGIGNGVLV
SVHALENHLLLNYNTDYELTSGEKLPLPKEISGYTYIGYIKEGKTTSESEVSNQKSSV
ATPTKQQKVDYNVTPNFVDHPSTVQAIQEQTPVSSTKPTEVQVVEKPFSTELINPRKE
EKQSSDSQEQLAEHKNLETKKEEKISPKEKTGVNTLNPQDEVLSGQLNKPELLYREE
TMETKIDFQEEIQENPDLAEGTVRVKQEGKLGKKVEIVRIFSVNKEEVSREIVSTSTTA
PSPRIVEKGTKKTQVIKEQPETGVEHKDVQSGAIVEPAIQPELPEAVVSDKGEPEVQPT
LPEAVVTDKGETEVQPESPDTVVSDKGEPEQVAPLPEYKGNIEQVKPETPVEKTKEQ

GPEKTEEVPVKPTEETPVNPNEGTTEGTSIQEAENPVQPAEESTTNSEKVSPDTSSKNT
GEVSSNPSDSTTSVGESNKPEHNDSKNENSEKTVEEVPVNPNEGTVEGTSNQETEKP
VQPAEETQTNSGKIANENTGEVSNKPSDSKPPVEESNQPEKNGTATKPENSGNTTSEN
GQTEPEPSNGNSTEDVSTESNTSNSNGNEEIKQENELDPDKKVEEPEKTLELRNVSDL
ELYSLSNGTYKQHISLEQVPSNPNSYFVKVKSSSFKDVYLPVASISEERKNDKILYKIT
AKVEKLQQEIESRYKDNFTFYLAKKGTEETTNFTSFSNLVKAINQNPSGTYHLAASLN
ANEVELGPDERSYIKDTFTGRLIGEKDGKNYAIYNLKKPLFENLSGATVEKLSLKNV
AISGKDDIGSLANEAQNNTKIKQVHVDGVLAGERGIGGLLAKAEQSSITESSFKGRIIN
TYETTAAYNIGGMVGHLTGDKALLTKSKATVAISSNTNTSDQTVGGLAGLVDRDAQ
IQDSYAEGDINNVKHFGRVAGVAGNLWDRTSGDVRHAGSLTNVLSDVNVTNGNAIT
GYHYNEMKVKDTFSSKANRVYNVTLVKDEVVSKESFEERGTMLDASQIASKKAEIN
PLILPTVEPLSTSGKKDSDFSKVAYYQAKRNLTYKNIEKLLPFYNKATIVKYGNLVNE
NSLLYQKELLSAVMMKDNQVITDIVSNKQTANKLLLHYKDDLSEKLDLKYQNDFAK
LAEYSLGNTGLLYTPNQFLYDQTSIIKQVLPDLQKVDYHSEAIRKTLGISPNVKQTEL
YLEDQFAKTKQQLEDSLKKLLSADAGLASANPVTEGYLVDKIKRNKEALLLGLTYL
ERWYNFSYGQVNVKDLVLYHLDFFGKGNASPLDTLIELGKSGFNNLLAKNNVDTYG
ISLASQHGTTDLFSTLEHYRKVFLPNTSNNDWFKSETKAYIVEEKSTIEEVKTKQGLA
GTKYSIGVYDRITSATWKYRNMVLPLLTLPERSVFVISTMSSLGFGAYDRYRSSDHK
AGKALNDFVEENARETAKRQRDHYDYWYRILDDNAREKLYRNILLYDAYKFGDDN
TVGKATEVADFDNPNPAMQHFFGPVGNKVGHNQHGAYATGDAVYYMGYRMLDK
DGAITYTHEMTHDSDQDIYLGGYGRRSGLGPEFFAKGLLQAPDHPDDATITINSILKH
SKSDSTESRRLQVLDPTTRFNNADDLKQYVHNMFDVVYMLEYLEGNSILKLDTNQK
QQLLRKVTNEYHPDPDGNKVYATNVVRNLTVEEVERLRSFNDLIDNNILSSREYASG
KYERNGYFTIKLFAPIYAALSNDIGTPGDLMGRRIAYELLAAKGFKDGMVPYISNQY
EEEAKQKGKTINLYGKTRGLVTDDLVLEKVFNNQYHTWSEFKKAMYQERQDQFDR
LNKVTFNDTTQPWQTFAKKTTSSVDELQKLMDVAVRKDAEHNYYHWNNYNPDIDS
EVHKLKRAIFKAYLDQTNDFRSSIFENKK (SEQ ID NO: 5)

In some embodiments, the fusion protein provided herein comprises an amino acid sequence as set forth in SEQ ID NO: 6. In some embodiments, the fusion protein as described herein consists of an amino acid sequence as set forth in SED ID NO: 6, or an amino acid sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 6. In some embodiments, the fusion protein having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 6 still retain the function or activity (such as, proteolytic activity or enzymatic activity of specifically cleaving IgA of IgA protease).

TCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGKGGG
GGGSRGNTTSENGQTEPEPSNGNSTEDVSTESNTSNSNGNEEIKQENELDPDKKVEEP
EKTLELRNVSDLELYSLSNGTYKQHISLEQVPSNPNSYFVKVKSSSFKDVYLPVASISE
ERKNDKILYKITAKVEKLQQEIESRYKDNFTFYLAKKGTEETTNFTSFSNLVKAINQN
PSGTYHLAASLNANEVELGPDERSYIKDTFTGRLIGEKDGKNYAIYNLKKPLFENLSG
ATVEKLSLKNVAISGKDDIGSLANEAQNNTKIKQVHVDGVLAGERGIGGLLAKAEQS
SITESSFKGRIINTYETTAAYNIGGMVGHLTGDKALLTKSKATVAISSNTNTSDQTVG
GLAGLVDRDAQIQDSYAEGDINNVKHFGRVAGVAGNLWDRTSGDVRHAGSLTNVL
SDVNVTNGNAITGYHYNEMKVKDTFSSKANRVYNVTLVKDEVVSKESFEERGTML
DASQIASKKAEINPLILPTVEPLSTSGKKDSDFSKVAYYQAKRNLTYKNIEKLLPFYN
KATIVKYGNLVNENSLLYQKELLSAVMMKDNQVITDIVSNKQTANKLLLHYKDDLS
EKLDLKYQNDFAKLAEYSLGNTGLLYTPNQFLYDQTSIIKQVLPDLQKVDYHSEAIR
KTLGISPNVKQTELYLEDQFAKTKQQLEDSLKKLLSADAGLASANPVTEGYLVDKIK
RNKEALLLGLTYLERWYNFSYGQVNVKDLVLYHLDFFGKGNASPLDTLIELGKSGF
NNLLAKNNVDTYGISLASQHGTTDLFSTLEHYRKVFLPNTSNNDWFKSETKAYIVEE
KSTIEEVKTKQGLAGTKYSIGVYDRITSATWKYRNMVLPLLTLPERSVFVISTMSSLG
FGAYDRYRSSDHKAGKALNDFVEENARETAKRQRDHYDYWYRILDDNAREKLYRN
ILLYDAYKFGDDNTVGKATEVADFDNPNPAMQHFFGPVGNKVGHNQHGAYATGD
AVYYMGYRMLDKDGAITYTHEMTHDSDQDIYLGGYGRRSGLGPEFFAKGLLQAPD
HPDDATITINSILKHSKSDSTESRRLQVLDPTTRFNNADDLKQYVHNMFDVVYMLEY
LEGNSILKLDTNQKQQLLRKVTNEYHPDPDGNKVYATNVVRNLTVEEVERLRSFND
LIDNNILSSREYASGKYERNGYFTIKLFAPIYAALSNDIGTPGDLMGRRIAYELLAAKG

FKDGMVPYISNQYEEEAKQKGKTINLYGKTRGLVTDDLVLEKVFNNQYHTWSEFKK
AMYQERQDQFDRLNKVTFNDTTQPWQTFAKKTTSSVDELQKLMDVAVRKDAEHN
YYHWNNYNPDIDSEVHKLKRAIFKAYLDQTNDFRSSIFENKK (SEQ ID NO: 6)

In some embodiments, the fusion protein described herein comprises an amino acid sequence as set forth in SED ID NO: 7. In some embodiments, the fusion protein as described herein consists of an amino acid sequence as set forth in SED ID NO: 7, or an amino acid sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 7. In some embodiments, the fusion protein having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 7 still retain the function or activity (such as, proteolytic activity or enzymatic activity of specifically cleaving IgA) of IgA protease.

GNTTSENGQTEPEPSNGNSTEDVSTESNTSNSNGNEEIKQENELDPDKKVEEPEKTLE
LRNVSDLELYSLSNGTYKQHISLEQVPSNPNSYFVKVKSSSFKDVYLPVASISEERKN
DKILYKITAKVEKLQQEIESRYKDNFTFYLAKKGTEETTNFTSFSNLVKAINQNPSGT
YHLAASLNANEVELGPDERSYIKDTFTGRLIGEKDGKNYAIYNLKKPLFENLSGATV
EKLSLKNVAISGKDDIGSLANEAQNNTKIKQVHVDGVLAGERGIGGLLAKAEQSSITE
SSFKGRIINTYETTAAYNIGGMVGHLTGDKALLTKSKATVAISSNTNTSDQTVGGLA
GLVDRDAQIQDSYAEGDINNVKHFGRVAGVAGNLWDRTSGDVRHAGSLTNVLSDV
NVTNGNAITGYHYNEMKVKDTFSSKANRVYNVTLVKDEVVSKESFEERGTMLDAS
QIASKKAEINPLILPTVEPLSTSGKKDSDFSKVAYYQAKRNLTYKNIEKLLPFYNKATI
VKYGNLVNENSLLYQKELLSAVMMKDNQVITDIVSNKQTANKLLLHYKDDLSEKL
DLKYQNDFAKLAEYSLGNTGLLYTPNQFLYDQTSIIKQVLPDLQKVDYHSEAIRKTL
GISPNVKQTELYLEDQFAKTKQQLEDSLKKLLSADAGLASANPVTEGYLVDKIKRNK
EALLLGLTYLERWYNFSYGQVNVKDLVLYHLDFFGKGNASPLDTLIELGKSGFNNLL
AKNNVDTYGISLASQHGTTDLFSTLEHYRKVFLPNTSNNDWFKSETKAYIVEEKSTIE
EVKTKQGLAGTKYSIGVYDRITSATWKYRNMVLPLLTLPERSVFVISTMSSLGFGAY
DRYRSSDHKAGKALNDFVEENARETAKRQRDHYDYWYRILDDNAREKLYRNILLY

DAYKFGDDNTVGKATEVADFDNPNPAMQHFFGPVGNKVGHNQHGAYATGDAVYY
MGYRMLDKDGAITYTHEMTHDSDQDIYLGGYGRRSGLGPEFFAKGLLQAPDHPDD
ATITINSILKHSKSDSTESRRLQVLDPTTRFNNADDLKQYVHNMFDVVYMLEYLEGN
SILKLDTNQKQQLLRKVTNEYHPDPDGNKVYATNVVRNLTVEEVERLRSFNDLIDN
NILSSREYASGKYERNGYFTIKLFAPIYAALSNDIGTPGDLMGRRIAYELLAAKGFKD
GMVPYISNQYEEEAKQKGKTINLYGKTRGLVTDDLVLEKVFNNQYHTWSEFKKAM
YQERQDQFDRLNKVTFNDTTQPWQTFAKKTTSSVDELQKLMDVAVRKDAEHNYYH
WNNYNPDIDSEVHKLKRAIFKAYLDQTNDFRSSIFENKKRSGGGGSHHHHHHHHH
TCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK (SEQ
ID NO: 7)

In some embodiments, the fusion protein described herein comprises an amino acid sequence as set forth in SED ID NO: 8. In some embodiments, the fusion protein as described herein consists of an amino acid sequence as set forth in SED ID NO: 8, or an amino acid sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 8. In some embodiments, the fusion protein having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 8 still retain the function or activity (such as, proteolytic activity or enzymatic activity of specifically cleaving IgA) of IgA protease.

GNTTSENGQTEPEPSNGNSTEDVSTESNTSNSNGNEEIKQENELDPDKKVEEPEKTLE
LRNVSDLELYSLSNGTYKQHISLEQVPSNPNSYFVKVKSSSFKDVYLPVASISEERKN
DKILYKITAKVEKLQQEIESRYKDNFTFYLAKKGTEETTNFTSFSNLVKAINQNPSGT
YHLAASLNANEVELGPDERSYIKDTFTGRLIGEKDGKNYAIYNLKKPLFENLSGATV
EKLSLKNVAISGKDDIGSLANEAQNNTKIKQVHVDGVLAGERGIGGLLAKAEQSSITE
SSFKGRIINTYETTAAYNIGGMVGHLTGDKALLTKSKATVAISSNTNTSDQTVGGLA

GLVDRDAQIQDSYAEGDINNVKHFGRVAGVAGNLWDRTSGDVRHAGSLTNVLSDV
NVTNGNAITGYHYNEMKVKDTFSSKANRVYNVTLVKDEVVSKESFEERGTMLDAS
QIASKKAEINPLILPTVEPLSTSGKKDSDFSKVAYYQAKRNLTYKNIEKLLPFYNKATI
VKYGNLVNENSLLYQKELLSAVMMKDNQVITDIVSNKQTANKLLLHYKDDLSEKL
DLKYQNDFAKLAEYSLGNTGLLYTPNQFLYDQTSIIKQVLPDLQKVDYHSEAIRKTL
GISPNVKQTELYLEDQFAKTKQQLEDSLKKLLSADAGLASANPVTEGYLVDKIKRNK
EALLLGLTYLERWYNFSYGQVNVKDLVLYHLDFFGKGNASPLDTLIELGKSGFNNLL
AKNNVDTYGISLASQHGTTDLFSTLEHYRKVFLPNTSNNDWFKSETKAYIVEEKSTIE
EVKTKQGLAGTKYSIGVYDRITSATWKYRNMVLPLLTLPERSVFVISTMSSLGFGAY
DRYRSSDHKAGKALNDFVEENARETAKRQRDHYDYWYRILDDNAREKLYRNILLY
DAYKFGDDNTVGKATEVADFDNPNPAMQHFFGPVGNKVGHNQHGAYATGDAVYY
MGYRMLDKDGAITYTHEMTHDSDQDIYLGGYGRRSGLGPEFFAKGLLQAPDHPDD
ATITINSILKHSKSDSTESRRLQVLDPTTRFNNADDLKQYVHNMFDVVYMLEYLEGN
SILKLDTNQKQQLLRKVTNEYHPDPDGNKVYATNVVRNLTVEEVERLRSFNDLIDN
NILSSREYASGKYERNGYFTIKLFAPIYAALSNDIGTPGDLMGRRIAYELLAAKGFKD
GMVPYISNQYEEEAKQKGKTINLYGKTRGLVTDDLVLEKVFNNQYHTWSEFKKAM
YQERQDQFDRLNKVTFNDTTQPWQTFAKKTTSSVDELQKLMDVAVRKDAEHNYYH
WNNYNPDIDSEVHKLKRAIFKAYLDQTNDFRSSIFENKKRSGGGGSESKYGPPCPSCP
APEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK (SEQ ID NO: 8)

In some embodiments, the fusion protein described herein comprises an amino acid sequence as set forth in SED ID NO: 24. In some embodiments, the fusion protein as described herein consists of the amino acid sequence as set forth in SED ID NO: 24, or having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 24. In some embodiments, the fusion protein having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as set forth in SEQ ID NO: 24 still retain the function or activity (such as, proteolytic activity or enzymatic activity of specifically cleaving IgA) of IgA protease.

GNTTSENGQTEPEPSNGNSTEDVSTESNTSNSNGNEEIKQENELDPDKKVEEPEKTLE
LRNVSDLELYSLSNGTYKQHISLEQVPSNPNSYFVKVKSSSFKDVYLPVASISEERKN
DKILYKITAKVEKLQQEIESRYKDNFTFYLAKKGTEETTNFTSFSNLVKAINQNPSGT
YHLAASLNANEVELGPDERSYIKDTFTGRLIGEKDGKNYAIYNLKKPLFENLSGATV
EKLSLKNVAISGKDDIGSLANEAQNNTKIKQVHVDGVLAGERGIGGLLAKAEQSSITE
SSFKGRIINTYETTAAYNIGGMVGHLTGDKALLTKSKATVAISSNTNTSDQTVGGLA
GLVDRDAQIQDSYAEGDINNVKHFGRVAGVAGNLWDRTSGDVRHAGSLTNVLSDV
NVTNGNAITGYHYNEMKVKDTFSSKANRVYNVTLVKDEVVSKESFEERGTMLDAS
QIASKKAEINPLILPTVEPLSTSGKKDSDFSKVAYYQAKRNLTYKNIEKLLPFYNKATI
VKYGNLVNENSLLYQKELLSAVMMKDNQVITDIVSNKQTANKLLLHYKDDLSEKL
DLKYQNDFAKLAEYSLGNTGLLYTPNQFLYDQTSIIKQVLPDLQKVDYHSEAIRKTL
GISPNVKQTELYLEDQFAKTKQQLEDSLKKLLSADAGLASANPVTEGYLVDKIKRNK
EALLLGLTYLERWYNFSYGQVNVKDLVLYHLDFFGKGNASPLDTLIELGKSGFNNLL
AKNNVDTYGISLASQHGTTDLFSTLEHYRKVFLPNTSNNDWFKSETKAYIVEEKSTIE
EVKTKQGLAGTKYSIGVYDRITSATWKYRNMVLPLLTLPERSVFVISTMSSLGFGAY
DRYRSSDHKAGKALNDFVEENARETAKRQRDHYDYWYRILDDNAREKLYRNILLY
DAYKFGDDNTVGKATEVADFDNPNPAMQHFFGPVGNKVGHNQHGAYATGDAVYY
MGYRMLDKDGAITYTHEMTHDSDQDIYLGGYGRRSGLGPEFFAKGLLQAPDHPDD
ATITINSILKHSKSDSTESRRLQVLDPTTRFNNADDLKQYVHNMFDVVYMLEYLEGN
SILKLDTNQKQQLLRKVTNEYHPDPDGNKVYATNVVRNLTVEEVERLRSFNDLIDN
NILSSREYASGKYERNGYFTIKLFAPIYAALSNDIGTPGDLMGRRIAYELLAAKGFKD
GMVPYISNQYEEEAKQKGKTINLYGKTRGLVTDDLVLEKVFNNQYHTWSEFKKAM
YQERQDQFDRLNKVTFNDTTQPWQTFAKKTTSSVDELQKLMDVAVRKDAEHNYYH
WNNYNPDIDSEVHKLKRAIFKAYLDQTNDFRSSIFENKKRSGSSGSSGESKYGPPCPS
CPAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK (SEQ ID NO:
24)

In some embodiments, the fusion protein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days in blood circulation of a subject.

### Nucleic Acids

In another aspect, the present disclosure provides an isolated nucleic acid comprising a nucleotide sequence encoding the truncated form of IgA protease or comprising a nucleotide sequence encoding the fusion protein described herein.

As used herein, the term "nucleic acid" or "nucleotide" refers to deoxyribonucleic acid (DNA) or ribonucleic acid (RNA) in single-stranded or double-stranded form and polymers thereof. Unless otherwise indicated, a particular polynucleotide sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more (or all) selected codons is substituted with mixed-base and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

Using the convention procedures, the DNA encoding the truncated form of IgA protease or the fusion protein described herein can be easily isolated and sequenced (e.g., by using oligonucleotide probes capable of binding specifically to the gene encoding the fusion protein). The encoding DNA may also be obtained by synthetic methods.

In some embodiments, the nucleic acid as described herein comprises a nucleic acid sequence as set forth in SED ID NO: 9. In some embodiments, the nucleic acid as described herein consists of a nucleotide sequence as set forth in SED ID NO: 9, or a nucleotide sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the nucleotide sequence as set forth in

### SEQ ID NO: 9.

ACATGCCCACCGTGCCCAGCACCTGAACTCCTGGGGGGACCGTCAGTCTTCCTCT
TCCCCCCAAAACCCAAGGACACCCTCATGATCTCCCGGACCCCTGAGGTCACATG

CGTGGTGGTGGACGTGAGCCACGAAGACCCTGAGGTCAAGTTCAACTGGTACGT
GGACGGCGTGGAGGTGCATAATGCCAAGACAAAGCCGCGGGAGGAGCAGTACA
ACAGCACGTACCGGGTGGTCAGCGTCCTCACCGTCCTGCACCAGGACTGGCTGA
ATGGCAAGGAGTACAAGTGCAAGGTCTCCAACAAAGCCCTCCCAGCCCCCATCG
AGAAAACCATCTCCAAAGCCAAAGGGCAGCCCCGAGAACCACAGGTGTACACCC
TGCCCCCATCCCGGGATGAGCTGACCAAGAACCAGGTCAGCCTGACCTGCCTGGT
CAAAGGCTTCTATCCCAGCGACATCGCCGTGGAGTGGGAGAGCAATGGGCAGCC
GGAGAACAACTACAAGACCACGCCTCCCGTGCTGGACTCCGACGGCTCCTTCTTC
CTCTACAGCAAGCTCACCGTGGACAAGAGCAGGTGGCAGCAGGGGAACGTCTTC
TCATGCTCCGTGATGCATGAGGCTCTGCACAACCACTACACGCAGAAGAGCCTCT
CCCTGTCTCCGGGTAAAGGTGGCGGTGGCGGCGGTTCTAGACAAGAAACGGCAG
GTGTGCATTACAAATACGTGGCAGATAGCGAACTGAGCAGCGAAGAAAAAAAAC
AACTGGTCTACGACATCCCGACCTACGTCGAAAATGATGACGAAACGTATTACCT
GGTGTATAAACTGAATTCACAGAACCAACTGGCAGAACTGCCGAATACCGGCTC
GAAAAACGAACGTCAGGCTCTGGTGGCAGGTGCTAGCCTGGCAGCAATGGGTAT
TCTGATCTTTGCCGTGAGCAAGAAAAAAGTTAAAAACAAAACCGTCCTGCATCTG
GTCCTGGTGGCAGGCATTGGTAATGGCGTTCTGGTCAGCGTGCATGCTCTGGAAA
ACCACCTGCTGCTGAACTACAATACCGATTATGAACTGACGAGCGGTGAAAAAC
TGCCGCTGCCGAAAGAAATCTCTGGCTATACCTACATTGGTTACATCAAAGAAGG
CAAAACCACGTCGGAAAGCGAAGTCTCCAACCAGAAAAGCTCTGTGGCAACCCC
GACGAAACAGCAAAAAGTTGACTATAATGTCACCCCGAACTTCGTTGATCATCCG
TCTACCGTCCAAGCGATTCAGGAACAAACGCCGGTCAGCTCCACCAAACCGACG
GAAGTGCAGGTGGTTGAAAAACCGTTTTCAACCGAACTGATCAATCCGCGTAAA
GAAGAAAAACAGTCATCGGATTCGCAGGAACAACTGGCGGAACACAAAAACCT
GGAAACCAAAAAAGAAGAAAAAATCAGCCCGAAAGAAAAAACCGGCGTGAATA
CGCTGAACCCGCAGGATGAAGTTCTGTCTGGTCAACTGAATAAACCGGAACTGCT
GTATCGTGAAGAAACCATGGAAACGAAAATTGACTTCCAGGAAGAAATCCAAGA
AAACCCGGATCTGGCGGAAGGCACCGTTCGCGTCAAACAGGAAGGTAAACTGGG
CAAAAAAGTTGAAATTGTCCGTATCTTTTCCGTGAATAAAGAAGAAGTTTCACGC
GAAATTGTCTCTACCAGTACCACGGCACCGAGCCCGCGTATTGTCGAAAAAGGT
ACCAAGAAAACCCAGGTGATCAAAGAACAACCTGAAACCGGCGTGGAACATAA
AGATGTTCAGAGTGGTGCTATTGTGGAACCGGCGATCCAACCGGAACTGCCGGA
AGCGGTCGTGTCCGATAAAGGCGAACCGGAAGTTCAGCCGACCCTGCCGGAAGC
CGTTGTCACCGACAAAGGTGAAACGGAAGTGCAGCCGGAATCCCCGGACACCGT

GGTTTCAGATAAAGGTGAACCGGAACAGGTTGCACCGCTGCCGGAATACAAGGG
TAACATCGAACAAGTGAAACCGGAAACCCCGGTGGAAAAAACGAAAGAACAGG
GCCCGGAAAAAACCGAAGAAGTGCCGGTTAAACCGACCGAAGAAACGCCGGTG
AACCCGAATGAAGGTACCACGGAAGGCACCAGCATCCAGGAAGCCGAAAATCC
GGTTCAACCGGCAGAAGAATCAACCACGAACTCGGAAAAAGTCAGCCCGGACAC
CAGCTCTAAAAATACGGGCGAAGTTAGTTCCAACCCGTCCGATTCAACCACGTCT
GTCGGTGAAAGTAATAAACCGGAACACAACGACTCCAAAAACGAAAATTCAGAA
AAAACCGTCGAAGAAGTCCCGGTTAATCCGAATGAAGGTACCGTGGAAGGCACG
AGCAATCAGGAAACCGAAAAACCGGTTCAGCCGGCAGAAGAAACCCAAACGAA
TAGTGGCAAAATTGCTAACGAAAATACCGGTGAAGTGTCCAACAAACCGTCGGA
TAGCAAACCGCCGGTTGAAGAAAGCAATCAGCCGGAGAAAAACGGCACCGCGA
CGAAACCGGAAAATTCGGGCAACACCACGAGCGAAAACGGTCAGACCGAACCG
GAACCGTCCAACGGCAATTCAACGGAAGATGTTAGTACCGAATCCAATACGTCT
AACAGTAACGGTAACGAAGAAATCAAACAGGAAAACGAACTGGATCCGGACAA
AAAAGTCGAAGAACCGGAAAAAACCCTGGAACTGCGCAATGTGAGCGATCTGGA
ACTGTATTCCCTGTCAAACGGCACGTACAAACAGCATATCTCTCTGGAACAAGTT
CCGTCGAATCCGAACAGCTACTTTGTTAAAGTCAAATCATCGAGCTTCAAAGATG
TGTATCTGCCGGTTGCATCGATCAGCGAAGAACGTAAAAATGACAAAATCCTGT
ACAAAATCACCGCTAAAGTGGAAAAACTGCAGCAAGAAATCGAAAGCCGCTATA
AAGATAACTTTACGTTCTACCTGGCGAAAAAAGGTACCGAAGAAACCACGAATT
TTACGTCTTTCAGTAACCTGGTTAAAGCGATTAATCAGAACCCGTCAGGCACCTA
CCACCTGGCAGCTTCGCTGAATGCCAACGAAGTGGAACTGGGTCCGGACGAACG
TAGCTATATTAAAGATACCTTCACGGGTCGCCTGATCGGCGAAAAAGATGGTAA
AAACTACGCCATCTACAACCTGAAAAAACCGCTGTTTGAAAATCTGTCTGGCGCG
ACCGTTGAAAAACTGTCGCTGAAAAACGTCGCCATTAGCGGTAAAGATGACATC
GGCTCTCTGGCGAATGAAGCCCAGAACAATACCAAAATCAAACAAGTGCATGTT
GATGGCGTGCTGGCAGGTGAACGTGGCATTGGCGGTCTGCTGGCGAAAGCCGAA
CAGTCTAGTATCACCGAATCCTCATTTAAAGGTCGCATTATCAATACGTATGAAA
CCACGGCGGCCTACAACATTGGCGGTATGGTTGGCCACCTGACCGGTGATAAAG
CCCTGCTGACCAAAAGCAAAGCAACGGTGGCTATCTCGAGCAATACCAACACGT
CTGATCAGACCGTTGGCGGTCTGGCTGGTCTGGTCGATCGTGACGCGCAGATTCA
AGACTCTTATGCCGAAGGCGATATCAACAATGTTAAACATTTTGGTCGCGTGGCG
GGTGTTGCCGGCAATCTGTGGGACCGTACCAGTGGCGATGTTCGCCACGCAGGTT
CGCTGACGAATGTCCTGAGCGATGTCAACGTGACCAATGGTAACGCTATTACGG

GCTACCATTACAACGAAATGAAAGTGAAAGATACCTTCTCTAGTAAAGCGAATC
GTGTCTATAACGTGACGCTGGTTAAAGATGAAGTCGTGTCAAAAGAATCGTTTGA
AGAACGCGGCACCATGCTGGACGCATCTCAGATCGCTAGTAAAAAAGCGGAAAT
TAACCCGCTGATCCTGCCGACCGTTGAACCGCTGTCCACGTCAGGTAAAAAAGAT
AGCGACTTCTCTAAAGTGGCCTATTACCAGGCAAAACGTAACCTGACCTACAAA
AACATCGAAAAACTGCTGCCGTTCTACAACAAAGCCACGATCGTCAAATACGGC
AACCTGGTGAATGAAAACAGCCTGCTGTATCAGAAAGAACTGCTGTCTGCGGTG
ATGATGAAAGACAATCAGGTCATTACCGATATCGTGAGTAATAAACAAACGGCC
AACAAACTGCTGCTGCATTATAAAGATGACCTGTCCGAAAAACTGGACCTGAAA
TATCAGAACGATTTCGCAAAACTGGCTGAATACTCACTGGGTAATACCGGCCTGC
TGTATACGCCGAACCAGTTTCTGTACGATCAAACCTCGATTATCAAACAGGTGCT
GCCGGACCTGCAAAAAGTTGATTACCACTCAGAAGCAATTCGCAAAACCCTGGG
TATCTCGCCGAATGTGAAACAGACGGAACTGTATCTGGAAGATCAATTTGCTAAA
ACCAAACAGCAACTGGAAGACTCTCTGAAAAAACTGCTGAGTGCAGATGCTGGC
CTGGCGTCTGCCAATCCGGTTACCGAAGGTTACCTGGTCGATAAAATTAAACGTA
ACAAAGAAGCCCTGCTGCTGGGCCTGACCTATCTGGAACGCTGGTACAATTTCAG
CTATGGTCAGGTTAACGTCAAAGACCTGGTGCTGTATCATCTGGATTTCTTTGGT
AAAGGCAACGCAAGTCCGCTGGATACCCTGATTGAACTGGGTAAATCCGGCTTT
AACAATCTGCTGGCGAAAAACAATGTTGACACCTACGGCATCAGTCTGGCCTCCC
AGCATGGTACCACGGACCTGTTTAGCACGCTGGAACACTATCGTAAAGTGTTCCT
GCCGAATACCAGTAACAATGATTGGTTTAAATCCGAAACGAAAGCGTATATTGT
GGAAGAAAAAAGCACCATCGAAGAAGTTAAAACGAAACAGGGTCTGGCGGGCA
CCAAATACAGTATTGGCGTTTATGATCGCATCACCAGCGCCACGTGGAAATATCG
CAACATGGTGCTGCCGCTGCTGACCCTGCCGGAACGCTCTGTGTTCGTTATTAGT
ACGATGTCCTCACTGGGTTTTGGCGCCTACGACCGTTATCGCTCGAGCGATCATA
AAGCGGGTAAAGCCCTGAATGATTTTGTGGAAGAAAACGCTCGTGAAACCGCGA
AACGTCAGCGCGACCACTATGATTACTGGTATCGCATTCTGGATGACAATGCGCG
TGAAAAACTGTATCGCAACATCCTGCTGTACGATGCCTATAAATTCGGCGATGAC
AATACCGTGGGTAAAGCCACGGAAGTTGCAGATTTTGACAATCCGAACCCGGCG
ATGCAACATTTCTTTGGTCCGGTCGGCAATAAAGTGGGTCATAACCAGCACGGCG
CGTATGCGACCGGTGATGCAGTGTATTACATGGGCTACCGTATGCTGGATAAAGA
CGGTGCTATTACCTATACGCATGAAATGACCCACGATAGTGACCAGGATATCTAC
CTGGGCGGTTATGGTCGTCGCTCCGGTCTGGGCCCGGAATTTTTCGCTAAAGGTC
TGCTGCAGGCGCCGGATCATCCGGATGACGCAACCATTACGATCAATTCAATTCT

GAAACACTCGAAAAGCGACTCTACCGAATCGCGTCGCCTGCAAGTTCTGGATCC
GACCACGCGCTTCAACAATGCGGATGACCTGAAACAGTATGTGCATAACATGTTT
GATGTTGTCTACATGCTGGAATATCTGGAAGGCAATAGCATTCTGAAACTGGACA
CCAACCAGAAACAGCAACTGCTGCGTAAAGTTACGAATGAATATCACCCGGACC
CGGATGGTAACAAAGTGTACGCGACCAATGTGGTTCGCAACCTGACGGTCGAAG
AAGTGGAACGTCTGCGCTCATTCAACGACCTGATTGATAACAATATCCTGTCTAG
TCGTGAATACGCATCGGGCAAATATGAACGCAATGGTTACTTTACCATTAAACTG
TTCGCTCCGATCTACGCAGCTCTGAGTAACGACATTGGTACGCCGGGTGATCTGA
TGGGTCGTCGCATCGCCTATGAACTGCTGGCGGCCAAAGGCTTTAAAGATGGTAT
GGTTCCGTATATTAGCAATCAGTACGAAGAAGCGAAACAAAAAGGTAAAAC
CATCAACCTGTATGGCAAAACCCGTGGTCTGGTGACGGATGACCTGGTTCTGGAA
AAAGTCTTCAACAATCAATACCATACCTGGAGCGAATTTAAAAAAGCGATGTAT
CAGGAACGTCAGGACCAATTTGATCGCCTGAATAAAGTTACCTTCAACGATACCA
CGCAGCCGTGGCAAACGTTTGCGAAGAAAACCACGTCCTCAGTGGATGAACTGC
AGAAACTGATGGACGTCGCCGTGCGTAAAGATGCAGAACATAACTACTACCACT
GGAACAACTACAACCCGGACATTGATAGCGAAGTGCACAAACTGAAACGCGCGA
TCTTCAAAGCGTACCTGGACCAAACCAATGACTTCCGTTCTAGTATCTTCGAAAA
CAAAAAA (SEQ ID NO: 9)

In some embodiments, the nucleic acid provided herein comprises a nucleic acid sequence as set forth in SEQ ID NO: 10. In some embodiments, the nucleic acid as described herein consists of a nucleotide sequence as set forth in SED ID NO: 10, or a nucleotide sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the nucleotide sequence as set forth in SEQ ID NO: 10.

ACATGCCCACCGTGCCCAGCACCTGAACTCCTGGGGGGACCGTCAGTCTTCCTCT
TCCCCCCAAAACCCAAGGACACCCTCATGATCTCCCGGACCCCTGAGGTCACATG
CGTGGTGGTGGACGTGAGCCACGAAGACCCTGAGGTCAAGTTCAACTGGTACGT
GGACGGCGTGGAGGTGCATAATGCCAAGACAAAGCCGCGGGAGGAGCAGTACA
ACAGCACGTACCGGGTGGTCAGCGTCCTCACCGTCCTGCACCAGGACTGGCTGA
ATGGCAAGGAGTACAAGTGCAAGGTCTCCAACAAAGCCCTCCCAGCCCCCATCG
AGAAAACCATCTCCAAAGCCAAAGGGCAGCCCCGAGAACCACAGGTGTACACCC

TGCCCCCATCCCGGGATGAGCTGACCAAGAACCAGGTCAGCCTGACCTGCCTGGT
CAAAGGCTTCTATCCCAGCGACATCGCCGTGGAGTGGGAGAGCAATGGGCAGCC
GGAGAACAACTACAAGACCACGCCTCCCGTGCTGGACTCCGACGGCTCCTTCTTC
CTCTACAGCAAGCTCACCGTGGACAAGAGCAGGTGGCAGCAGGGGAACGTCTTC
TCATGCTCCGTGATGCATGAGGCTCTGCACAACCACTACACGCAGAAGAGCCTCT
CCCTGTCTCCGGGTAAAGGTGGCGGTGGCGGTTCTAGAGGCAACACCACGA
GCGAAAACGGTCAGACCGAACCGGAACCGTCCAACGGCAATTCAACGGAAGATG
TTAGTACCGAATCCAATACGTCTAACAGTAACGGTAACGAAGAAATCAAACAGG
AAAACGAACTGGATCCGGACAAAAAAGTCGAAGAACCGGAAAAAACCCTGGAA
CTGCGCAATGTGAGCGATCTGGAACTGTATTCCCTGTCAAACGGCACGTACAAAC
AGCATATCTCTCTGGAACAAGTTCCGTCGAATCCGAACAGCTACTTTGTTAAAGT
CAAATCATCGAGCTTCAAAGATGTGTATCTGCCGGTTGCATCGATCAGCGAAGAA
CGTAAAAATGACAAAATCCTGTACAAAATCACCGCTAAAGTGGAAAAACTGCAG
CAAGAAATCGAAAGCCGCTATAAAGATAACTTTACGTTCTACCTGGCGAAAAAA
GGTACCGAAGAAACCACGAATTTTACGTCTTTCAGTAACCTGGTTAAAGCGATTA
ATCAGAACCCGTCAGGCACCTACCACCTGGCAGCTTCGCTGAATGCCAACGAAG
TGGAACTGGGTCCGGACGAACGTAGCTATATTAAAGATACCTTCACGGGTCGCCT
GATCGGCGAAAAAGATGGTAAAAACTACGCCATCTACAACCTGAAAAAACCGCT
GTTTGAAAATCTGTCTGGCGCGACCGTTGAAAAACTGTCGCTGAAAAACGTCGCC
ATTAGCGGTAAAGATGACATCGGCTCTCTGGCGAATGAAGCCCAGAACAATACC
AAAATCAAACAAGTGCATGTTGATGGCGTGCTGGCAGGTGAACGTGGCATTGGC
GGTCTGCTGGCGAAAGCCGAACAGTCTAGTATCACCGAATCCTCATTTAAAGGTC
GCATTATCAATACGTATGAAACCACGGCGGCCTACAACATTGGCGGTATGGTTGG
CCACCTGACCGGTGATAAAGCCCTGCTGACCAAAAGCAAAGCAACGGTGGCTAT
CTCGAGCAATACCAACACGTCTGATCAGACCGTTGGCGGTCTGGCTGGTCTGGTC
GATCGTGACGCGCAGATTCAAGACTCTTATGCCGAAGGCGATATCAACAATGTTA
AACATTTTGGTCGCGTGGCGGGTGTTGCCGGCAATCTGTGGGACCGTACCAGTGG
CGATGTTCGCCACGCAGGTTCGCTGACGAATGTCCTGAGCGATGTCAACGTGACC
AATGGTAACGCTATTACGGGCTACCATTACAACGAAATGAAAGTGAAAGATACC
TTCTCTAGTAAAGCGAATCGTGTCTATAACGTGACGCTGGTTAAAGATGAAGTCG
TGTCAAAAGAATCGTTTGAAGAACGCGGCACCATGCTGGACGCATCTCAGATCG
CTAGTAAAAAAGCGGAAATTAACCCGCTGATCCTGCCGACCGTTGAACCGCTGTC
CACGTCAGGTAAAAAAGATAGCGACTTCTCTAAAGTGGCCTATTACCAGGCAAA
ACGTAACCTGACCTACAAAAACATCGAAAAACTGCTGCCGTTCTACAACAAAGC

CACGATCGTCAAATACGGCAACCTGGTGAATGAAAACAGCCTGCTGTATCAGAA
AGAACTGCTGTCTGCGGTGATGATGAAAGACAATCAGGTCATTACCGATATCGTG
AGTAATAAACAAACGGCCAACAAACTGCTGCTGCATTATAAAGATGACCTGTCC
GAAAAACTGGACCTGAAATATCAGAACGATTTCGCAAAACTGGCTGAATACTCA
CTGGGTAATACCGGCCTGCTGTATACGCCGAACCAGTTTCTGTACGATCAAACCT
CGATTATCAAACAGGTGCTGCCGGACCTGCAAAAAGTTGATTACCACTCAGAAG
CAATTCGCAAAACCCTGGGTATCTCGCCGAATGTGAAACAGACGGAACTGTATCT
GGAAGATCAATTTGCTAAAACCAAACAGCAACTGGAAGACTCTCTGAAAAAACT
GCTGAGTGCAGATGCTGGCCTGGCGTCTGCCAATCCGGTTACCGAAGGTTACCTG
GTCGATAAAATTAAACGTAACAAAGAAGCCCTGCTGCTGGGCCTGACCTATCTG
GAACGCTGGTACAATTTCAGCTATGGTCAGGTTAACGTCAAAGACCTGGTGCTGT
ATCATCTGGATTTCTTTGGTAAAGGCAACGCAAGTCCGCTGGATACCCTGATTGA
ACTGGGTAAATCCGGCTTTAACAATCTGCTGGCGAAAAACAATGTTGACACCTAC
GGCATCAGTCTGGCCTCCCAGCATGGTACCACGGACCTGTTTAGCACGCTGGAAC
ACTATCGTAAAGTGTTCCTGCCGAATACCAGTAACAATGATTGGTTTAAATCCGA
AACGAAAGCGTATATTGTGGAAGAAAAAAGCACCATCGAAGAAGTTAAAACGA
AACAGGGTCTGGCGGGCACCAAATACAGTATTGGCGTTTATGATCGCATCACCA
GCGCCACGTGGAAATATCGCAACATGGTGCTGCCGCTGCTGACCCTGCCGGAAC
GCTCTGTGTTCGTTATTAGTACGATGTCCTCACTGGGTTTTGGCGCCTACGACCGT
TATCGCTCGAGCGATCATAAAGCGGGTAAAGCCCTGAATGATTTTGTGGAAGAA
AACGCTCGTGAAACCGCGAAACGTCAGCGCGACCACTATGATTACTGGTATCGC
ATTCTGGATGACAATGCGCGTGAAAAACTGTATCGCAACATCCTGCTGTACGATG
CCTATAAATTCGGCGATGACAATACCGTGGGTAAAGCCACGGAAGTTGCAGATT
TTGACAATCCGAACCCGGCGATGCAACATTTCTTTGGTCCGGTCGGCAATAAAGT
GGGTCATAACCAGCACGGCGCGTATGCGACCGGTGATGCAGTGTATTACATGGG
CTACCGTATGCTGGATAAAGACGGTGCTATTACCTATACGCATGAAATGACCCAC
GATAGTGACCAGGATATCTACCTGGGCGGTTATGGTCGTCGCTCCGGTCTGGGCC
CGGAATTTTTCGCTAAAGGTCTGCTGCAGGCGCCGGATCATCCGGATGACGCAAC
CATTACGATCAATTCAATTCTGAAACACTCGAAAAGCGACTCTACCGAATCGCGT
CGCCTGCAAGTTCTGGATCCGACCACGCGCTTCAACAATGCGGATGACCTGAAAC
AGTATGTGCATAACATGTTTGATGTTGTCTACATGCTGGAATATCTGGAAGGCAA
TAGCATTCTGAAACTGGACACCAACCAGAAACAGCAACTGCTGCGTAAAGTTAC
GAATGAATATCACCCGGACCCGGATGGTAACAAAGTGTACGCGACCAATGTGGT
TCGCAACCTGACGGTCGAAGAAGTGGAACGTCTGCGCTCATTCAACGACCTGATT

GATAACAATATCCTGTCTAGTCGTGAATACGCATCGGGCAAATATGAACGCAAT
GGTTACTTTACCATTAAACTGTTCGCTCCGATCTACGCAGCTCTGAGTAACGACA
TTGGTACGCCGGGTGATCTGATGGGTCGTCGCATCGCCTATGAACTGCTGGCGGC
CAAAGGCTTTAAAGATGGTATGGTTCCGTATATTAGCAATCAGTACGAAGAAGA
AGCGAAACAAAAAGGTAAAACCATCAACCTGTATGGCAAAACCCGTGGTCTGGT
GACGGATGACCTGGTTCTGGAAAAAGTCTTCAACAATCAATACCATACCTGGAG
CGAATTTAAAAAAGCGATGTATCAGGAACGTCAGGACCAATTTGATCGCCTGAA
TAAAGTTACCTTCAACGATACCACGCAGCCGTGGCAAACGTTTGCGAAGAAAAC
CACGTCCTCAGTGGATGAACTGCAGAAACTGATGGACGTCGCCGTGCGTAAAGA
TGCAGAACATAACTACTACCACTGGAACAACTACAACCCGGACATTGATAGCGA
AGTGCACAAACTGAAACGCGCGATCTTCAAAGCGTACCTGGACCAAACCAATGA
CTTCCGTTCTAGTATCTTCGAAAACAAAAAA (SEQ ID NO: 10)

In some embodiments, the nucleic acid provided herein comprises a nucleic acid sequence as set forth in SEQ ID NO: 11. In some embodiments, the nucleic acid as described herein consists of a nucleotide sequence as set forth in SED ID NO: 11, or has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the nucleotide sequence as set forth in SEQ ID NO: 11.

GGCAACACCACGAGCGAAAACGGTCAGACCGAACCGGAACCGTCCAACGGCAA
TTCAACGGAAGATGTTAGTACCGAATCCAATACGTCTAACAGTAACGGTAACGA
AGAAATCAAACAGGAAAACGAACTGGATCCGGACAAAAAAGTCGAAGAACCGG
AAAAAACCCTGGAACTGCGCAATGTGAGCGATCTGGAACTGTATTCCCTGTCAA
ACGGCACGTACAAACAGCATATCTCTCTGGAACAAGTTCCGTCGAATCCGAACA
GCTACTTTGTTAAAGTCAAATCATCGAGCTTCAAAGATGTGTATCTGCCGGTTGC
ATCGATCAGCGAAGAACGTAAAAATGACAAAATCCTGTACAAAATCACCGCTAA
AGTGGAAAAACTGCAGCAAGAAATCGAAAGCCGCTATAAAGATAACTTTACGTT
CTACCTGGCGAAAAAAGGTACCGAAGAAACCACGAATTTTACGTCTTTCAGTAA
CCTGGTTAAAGCGATTAATCAGAACCCGTCAGGCACCTACCACCTGGCAGCTTCG
CTGAATGCCAACGAAGTGGAACTGGGTCCGGACGAACGTAGCTATATTAAAGAT
ACCTTCACGGGTCGCCTGATCGGCGAAAAAGATGGTAAAAACTACGCCATCTAC
AACCTGAAAAAACCGCTGTTTGAAAATCTGTCTGGCGCGACCGTTGAAAAACTGT
CGCTGAAAAACGTCGCCATTAGCGGTAAAGATGACATCGGCTCTCTGGCGAATG

AAGCCCAGAACAATACCAAAATCAAACAAGTGCATGTTGATGGCGTGCTGGCAG
GTGAACGTGGCATTGGCGGTCTGCTGGCGAAAGCCGAACAGTCTAGTATCACCG
AATCCTCATTTAAAGGTCGCATTATCAATACGTATGAAACCACGGCGGCCTACAA
CATTGGCGGTATGGTTGGCCACCTGACCGGTGATAAAGCCCTGCTGACCAAAAG
CAAAGCAACGGTGGCTATCTCGAGCAATACCAACACGTCTGATCAGACCGTTGG
CGGTCTGGCTGGTCTGGTCGATCGTGACGCGCAGATTCAAGACTCTTATGCCGAA
GGCGATATCAACAATGTTAAACATTTTGGTCGCGTGGCGGGTGTTGCCGGCAATC
TGTGGGACCGTACCAGTGGCGATGTTCGCCACGCAGGTTCGCTGACGAATGTCCT
GAGCGATGTCAACGTGACCAATGGTAACGCTATTACGGGCTACCATTACAACGA
AATGAAAGTGAAAGATACCTTCTCTAGTAAAGCGAATCGTGTCTATAACGTGAC
GCTGGTTAAAGATGAAGTCGTGTCAAAAGAATCGTTTGAAGAACGCGGCACCAT
GCTGGACGCATCTCAGATCGCTAGTAAAAAAGCGGAAATTAACCCGCTGATCCT
GCCGACCGTTGAACCGCTGTCCACGTCAGGTAAAAAAGATAGCGACTTCTCTAA
AGTGGCCTATTACCAGGCAAAACGTAACCTGACCTACAAAAACATCGAAAAACT
GCTGCCGTTCTACAACAAAGCCACGATCGTCAAATACGGCAACCTGGTGAATGA
AAACAGCCTGCTGTATCAGAAAGAACTGCTGTCTGCGGTGATGATGAAAGACAA
TCAGGTCATTACCGATATCGTGAGTAATAAACAAACGGCCAACAAACTGCTGCT
GCATTATAAAGATGACCTGTCCGAAAAACTGGACCTGAAATATCAGAACGATTT
CGCAAAACTGGCTGAATACTCACTGGGTAATACCGGCCTGCTGTATACGCCGAAC
CAGTTTCTGTACGATCAAACCTCGATTATCAAACAGGTGCTGCCGGACCTGCAAA
AAGTTGATTACCACTCAGAAGCAATTCGCAAAACCCTGGGTATCTCGCCGAATGT
GAAACAGACGGAACTGTATCTGGAAGATCAATTTGCTAAAACCAAACAGCAACT
GGAAGACTCTCTGAAAAAACTGCTGAGTGCAGATGCTGGCCTGGCGTCTGCCAA
TCCGGTTACCGAAGGTTACCTGGTCGATAAAATTAAACGTAACAAAGAAGCCCT
GCTGCTGGGCCTGACCTATCTGGAACGCTGGTACAATTTCAGCTATGGTCAGGTT
AACGTCAAAGACCTGGTGCTGTATCATCTGGATTTCTTTGGTAAAGGCAACGCAA
GTCCGCTGGATACCCTGATTGAACTGGGTAAATCCGGCTTTAACAATCTGCTGGC
GAAAAACAATGTTGACACCTACGGCATCAGTCTGGCCTCCCAGCATGGTACCAC
GGACCTGTTTAGCACGCTGGAACACTATCGTAAAGTGTTCCTGCCGAATACCAGT
AACAATGATTGGTTTAAATCCGAAACGAAAGCGTATATTGTGGAAGAAAAAAGC
ACCATCGAAGAAGTTAAAACGAAACAGGGTCTGGCGGGCACCAAATACAGTATT
GGCGTTTATGATCGCATCACCAGCGCCACGTGGAAATATCGCAACATGGTGCTGC
CGCTGCTGACCCTGCCGGAACGCTCTGTGTTCGTTATTAGTACGATGTCCTCACTG
GGTTTTGGCGCCTACGACCGTTATCGCTCGAGCGATCATAAAGCGGGTAAAGCCC

TGAATGATTTTGTGGAAGAAAACGCTCGTGAAACCGCGAAACGTCAGCGCGACC
ACTATGATTACTGGTATCGCATTCTGGATGACAATGCGCGTGAAAAACTGTATCG
CAACATCCTGCTGTACGATGCCTATAAATTCGGCGATGACAATACCGTGGGTAAA
GCCACGGAAGTTGCAGATTTTGACAATCCGAACCCGGCGATGCAACATTTCTTTG
GTCCGGTCGGCAATAAAGTGGGTCATAACCAGCACGGCGCGTATGCGACCGGTG
ATGCAGTGTATTACATGGGCTACCGTATGCTGGATAAAGACGGTGCTATTACCTA
TACGCATGAAATGACCCACGATAGTGACCAGGATATCTACCTGGGCGGTTATGGT
CGTCGCTCCGGTCTGGGCCCGGAATTTTTCGCTAAAGGTCTGCTGCAGGCGCCGG
ATCATCCGGATGACGCAACCATTACGATCAATTCAATTCTGAAACACTCGAAAAG
CGACTCTACCGAATCGCGTCGCCTGCAAGTTCTGGATCCGACCACGCGCTTCAAC
AATGCGGATGACCTGAAACAGTATGTGCATAACATGTTTGATGTTGTCTACATGC
TGGAATATCTGGAAGGCAATAGCATTCTGAAACTGGACACCAACCAGAAACAGC
AACTGCTGCGTAAAGTTACGAATGAATATCACCCGGACCCGGATGGTAACAAAG
TGTACGCGACCAATGTGGTTCGCAACCTGACGGTCGAAGAAGTGGAACGTCTGC
GCTCATTCAACGACCTGATTGATAACAATATCCTGTCTAGTCGTGAATACGCATC
GGGCAAATATGAACGCAATGGTTACTTTACCATTAAACTGTTCGCTCCGATCTAC
GCAGCTCTGAGTAACGACATTGGTACGCCGGGTGATCTGATGGGTCGTCGCATCG
CCTATGAACTGCTGGCGGCCAAAGGCTTTAAAGATGGTATGGTTCCGTATATTAG
CAATCAGTACGAAGAAGAAGCGAAACAAAAAGGTAAAACCATCAACCTGTATG
GCAAAACCCGTGGTCTGGTGACGGATGACCTGGTTCTGGAAAAAGTCTTCAACA
ATCAATACCATACCTGGAGCGAATTTAAAAAAGCGATGTATCAGGAACGTCAGG
ACCAATTTGATCGCCTGAATAAAGTTACCTTCAACGATACCACGCAGCCGTGGCA
AACGTTTGCGAAGAAAACCACGTCCTCAGTGGATGAACTGCAGAAACTGATGGA
CGTCGCCGTGCGTAAAGATGCAGAACATAACTACTACCACTGGAACAACTACAA
CCCGGACATTGATAGCGAAGTGCACAAACTGAAACGCGCGATCTTCAAAGCGTA
CCTGGACCAAACCAATGACTTCCGTTCTAGTATCTTCGAAAACAAAAAAAGATCT
GGTGGCGGTGGCTCTcaccatcatcaccaccatcatcatcaccacACATGCCCACCGTGCCCAGCA
CCTGAACTCCTGGGGGGACCGTCAGTCTTCCTCTTCCCCCCAAAACCCAAGGACA
CCCTCATGATCTCCCGGACCCCTGAGGTCACATGCGTGGTGGTGGACGTGAGCCA
CGAAGACCCTGAGGTCAAGTTCAACTGGTACGTGGACGGCGTGGAGGTGCATAA
TGCCAAGACAAAGCCGCGGGAGGAGCAGTACAACAGCACGTACCGGGTGGTCA
GCGTCCTCACCGTCCTGCACCAGGACTGGCTGAATGGCAAGGAGTACAAGTGCA
AGGTCTCCAACAAAGCCCTCCCAGCCCCCATCGAGAAAACCATCTCCAAAGCCA
AAGGGCAGCCCCGAGAACCACAGGTGTACACCCTGCCCCCATCCCGGGATGAGC

TGACCAAGAACCAGGTCAGCCTGACCTGCCTGGTCAAAGGCTTCTATCCCAGCGA
CATCGCCGTGGAGTGGGAGAGCAATGGGCAGCCGGAGAACAACTACAAGACCA
CGCCTCCCGTGCTGGACTCCGACGGCTCCTTCTTCCTCTACAGCAAGCTCACCGT
GGACAAGAGCAGGTGGCAGCAGGGGAACGTCTTCTCATGCTCCGTGATGCATGA
GGCTCTGCACAACCACTACACGCAGAAGAGCCTCTCCCTGTCTCCGGGTAAA

### (SEQ ID NO: 11)

In some embodiments, the nucleic acids provided herein comprises a nucleic acid sequence as set forth in SEQ ID NO: 12. In some embodiments, the nucleic acid as described herein consists of a nucleotide sequence as set forth in SED ID NO: 12, or a nucleotide sequence that has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the nucleotide sequence as set forth in SEQ ID NO: 12.

GGCAACACCACGAGCGAAAACGGTCAGACCGAACCGGAACCGTCCAACGGCAA
TTCAACGGAAGATGTTAGTACCGAATCCAATACGTCTAACAGTAACGGTAACGA
AGAAATCAAACAGGAAAACGAACTGGATCCGGACAAAAAAGTCGAAGAACCGG
AAAAAACCCTGGAACTGCGCAATGTGAGCGATCTGGAACTGTATTCCCTGTCAA
ACGGCACGTACAAACAGCATATCTCTCTGGAACAAGTTCCGTCGAATCCGAACA
GCTACTTTGTTAAAGTCAAATCATCGAGCTTCAAAGATGTGTATCTGCCGGTTGC
ATCGATCAGCGAAGAACGTAAAAATGACAAAATCCTGTACAAAATCACCGCTAA
AGTGGAAAAACTGCAGCAAGAAATCGAAAGCCGCTATAAAGATAACTTTACGTT
CTACCTGGCGAAAAAAGGTACCGAAGAAACCACGAATTTTACGTCTTTCAGTAA
CCTGGTTAAAGCGATTAATCAGAACCCGTCAGGCACCTACCACCTGGCAGCTTCG
CTGAATGCCAACGAAGTGGAACTGGGTCCGGACGAACGTAGCTATATTAAAGAT
ACCTTCACGGGTCGCCTGATCGGCGAAAAAGATGGTAAAAACTACGCCATCTAC
AACCTGAAAAAACCGCTGTTTGAAAATCTGTCTGGCGCGACCGTTGAAAAACTGT
CGCTGAAAAACGTCGCCATTAGCGGTAAAGATGACATCGGCTCTCTGGCGAATG
AAGCCCAGAACAATACCAAAATCAAACAAGTGCATGTTGATGGCGTGCTGGCAG
GTGAACGTGGCATTGGCGGTCTGCTGGCGAAAGCCGAACAGTCTAGTATCACCG
AATCCTCATTTAAAGGTCGCATTATCAATACGTATGAAACCACGGCGGCCTACAA
CATTGGCGGTATGGTTGGCCACCTGACCGGTGATAAAGCCCTGCTGACCAAAAG
CAAAGCAACGGTGGCTATCTCGAGCAATACCAACACGTCTGATCAGACCGTTGG

CGGTCTGGCTGGTCTGGTCGATCGTGACGCGCAGATTCAAGACTCTTATGCCGAA
GGCGATATCAACAATGTTAAACATTTTGGTCGCGTGGCGGGTGTTGCCGGCAATC
TGTGGGACCGTACCAGTGGCGATGTTCGCCACGCAGGTTCGCTGACGAATGTCCT
GAGCGATGTCAACGTGACCAATGGTAACGCTATTACGGGCTACCATTACAACGA
AATGAAAGTGAAAGATACCTTCTCTAGTAAAGCGAATCGTGTCTATAACGTGAC
GCTGGTTAAAGATGAAGTCGTGTCAAAAGAATCGTTTGAAGAACGCGGCACCAT
GCTGGACGCATCTCAGATCGCTAGTAAAAAAGCGGAAATTAACCCGCTGATCCT
GCCGACCGTTGAACCGCTGTCCACGTCAGGTAAAAAAGATAGCGACTTCTCTAA
AGTGGCCTATTACCAGGCAAAACGTAACCTGACCTACAAAAACATCGAAAAACT
GCTGCCGTTCTACAACAAAGCCACGATCGTCAAATACGGCAACCTGGTGAATGA
AAACAGCCTGCTGTATCAGAAAGAACTGCTGTCTGCGGTGATGATGAAAGACAA
TCAGGTCATTACCGATATCGTGAGTAATAAACAAACGGCCAACAAACTGCTGCT
GCATTATAAAGATGACCTGTCCGAAAAACTGGACCTGAAATATCAGAACGATTT
CGCAAAACTGGCTGAATACTCACTGGGTAATACCGGCCTGCTGTATACGCCGAAC
CAGTTTCTGTACGATCAAACCTCGATTATCAAACAGGTGCTGCCGGACCTGCAAA
AAGTTGATTACCACTCAGAAGCAATTCGCAAAACCCTGGGTATCTCGCCGAATGT
GAAACAGACGGAACTGTATCTGGAAGATCAATTTGCTAAAACCAAACAGCAACT
GGAAGACTCTCTGAAAAAACTGCTGAGTGCAGATGCTGGCCTGGCGTCTGCCAA
TCCGGTTACCGAAGGTTACCTGGTCGATAAAATTAAACGTAACAAAGAAGCCCT
GCTGCTGGGCCTGACCTATCTGGAACGCTGGTACAATTTCAGCTATGGTCAGGTT
AACGTCAAAGACCTGGTGCTGTATCATCTGGATTTCTTTGGTAAAGGCAACGCAA
GTCCGCTGGATACCCTGATTGAACTGGGTAAATCCGGCTTTAACAATCTGCTGGC
GAAAAACAATGTTGACACCTACGGCATCAGTCTGGCCTCCCAGCATGGTACCAC
GGACCTGTTTAGCACGCTGGAACACTATCGTAAAGTGTTCCTGCCGAATACCAGT
AACAATGATTGGTTTAAATCCGAAACGAAAGCGTATATTGTGGAAGAAAAAAGC
ACCATCGAAGAAGTTAAAACGAAACAGGGTCTGGCGGGCACCAAATACAGTATT
GGCGTTTATGATCGCATCACCAGCGCCACGTGGAAATATCGCAACATGGTGCTGC
CGCTGCTGACCCTGCCGGAACGCTCTGTGTTCGTTATTAGTACGATGTCCTCACTG
GGTTTTGGCGCCTACGACCGTTATCGCTCGAGCGATCATAAAGCGGGTAAAGCCC
TGAATGATTTTGTGGAAGAAAACGCTCGTGAAACCGCGAAACGTCAGCGCGACC
ACTATGATTACTGGTATCGCATTCTGGATGACAATGCGCGTGAAAAACTGTATCG
CAACATCCTGCTGTACGATGCCTATAAATTCGGCGATGACAATACCGTGGGTAAA
GCCACGGAAGTTGCAGATTTTGACAATCCGAACCCGGCGATGCAACATTTCTTTG
GTCCGGTCGGCAATAAAGTGGGTCATAACCAGCACGGCGCGTATGCGACCGGTG

ATGCAGTGTATTACATGGGCTACCGTATGCTGGATAAAGACGGTGCTATTACCTA
TACGCATGAAATGACCCACGATAGTGACCAGGATATCTACCTGGGCGGTTATGGT
CGTCGCTCCGGTCTGGGCCCGGAATTTTTCGCTAAAGGTCTGCTGCAGGCGCCGG
ATCATCCGGATGACGCAACCATTACGATCAATTCAATTCTGAAACACTCGAAAAG
CGACTCTACCGAATCGCGTCGCCTGCAAGTTCTGGATCCGACCACGCGCTTCAAC
AATGCGGATGACCTGAAACAGTATGTGCATAACATGTTTGATGTTGTCTACATGC
TGGAATATCTGGAAGGCAATAGCATTCTGAAACTGGACACCAACCAGAAACAGC
AACTGCTGCGTAAAGTTACGAATGAATATCACCCGGACCCGGATGGTAACAAAG
TGTACGCGACCAATGTGGTTCGCAACCTGACGGTCGAAGAAGTGGAACGTCTGC
GCTCATTCAACGACCTGATTGATAACAATATCCTGTCTAGTCGTGAATACGCATC
GGGCAAATATGAACGCAATGGTTACTTTACCATTAAACTGTTCGCTCCGATCTAC
GCAGCTCTGAGTAACGACATTGGTACGCCGGGTGATCTGATGGGTCGTCGCATCG
CCTATGAACTGCTGGCGGCCAAAGGCTTTAAAGATGGTATGGTTCCGTATATTAG
CAATCAGTACGAAGAAGAAGCGAAACAAAAAGGTAAAACCATCAACCTGTATG
GCAAAACCCGTGGTCTGGTGACGGATGACCTGGTTCTGGAAAAAGTCTTCAACA
ATCAATACCATACCTGGAGCGAATTTAAAAAAGCGATGTATCAGGAACGTCAGG
ACCAATTTGATCGCCTGAATAAAGTTACCTTCAACGATACCACGCAGCCGTGGCA
AACGTTTGCGAAGAAAACCACGTCCTCAGTGGATGAACTGCAGAAACTGATGGA
CGTCGCCGTGCGTAAAGATGCAGAACATAACTACTACCACTGGAACAACTACAA
CCCGGACATTGATAGCGAAGTGCACAAACTGAAACGCGCGATCTTCAAAGCGTA
CCTGGACCAAACCAATGACTTCCGTTCTAGTATCTTCGAAAACAAAAAAAGATCT
GGCGGTGGTGGTAGCGAATCGAAGTACGGTCCGCCGTGTCCGAGTTGCCCGGCT
CCGGAGTTTCTGGGTGGCCCTAGCGTGTTCTTATTTCCGCCAAAGCCGAAGGACA
CCCTGATGATCAGCCGCACCCCGGAGGTAACTTGCGTCGTCGTGGATGTTTCCCA
AGAAGATCCGGAGGTGCAGTTTAACTGGTATGTGGACGGCGTTGAAGTTCATAA
TGCGAAAACCAAGCCGCGTGAAGAGCAGTTCAACAGCACCTATCGTGTGGTCTC
CGTGTTGACGGTTCTTCACCAGGATTGGTTGAATGGTAAAGAATATAAGTGCAAA
GTTTCCAATAAAGGCCTGCCGTCGAGCATTGAAAAAACGATTAGCAAGGCAAAG
GGCCAACCTCGCGAGCCGCAAGTGTACACTCTGCCGCCGAGCCAAGAGGAAATG
ACCAAAAACCAGGTTTCATTGACCTGCCTGGTTAAGGGTTTTTACCCGAGCGACA
TCGCCGTTGAGTGGGAATCTAACGGCCAGCCGGAGAACAACTACAAGACCACCC
CACCGGTTCTGGACAGCGATGGTTCCTTCTTCCTGTACTCCCGTCTCACCGTGGAC
AAAAGCCGTTGGCAAGAGGGAAACGTGTTCTCTTGTAGCGTGATGCATGAAGCG

CTGCACAATCATTATACGCAGAAATCTCTGAGCCTTTCTCTGGGCAAA (SEQ ID
NO: 12)

In some embodiments, the nucleic acids provided herein comprises a nucleic acid sequence as set forth in SEQ ID NO: 25. In some embodiments, the nucleic acid as described herein consists of the nucleotide sequence as set forth in SED ID NO: 25, or having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the nucleotide sequence as set forth in SEQ ID NO: 25.

GGCAACACCACGAGCGAAAACGGTCAGACCGAACCGGAACCGTCCAACGGCAA
TTCAACGGAAGATGTTAGTACCGAATCCAATACGTCTAACAGTAACGGTAACGA
AGAAATCAAACAGGAAAACGAACTGGATCCGGACAAAAAAGTCGAAGAACCGG
AAAAAACCCTGGAACTGCGCAATGTGAGCGATCTGGAACTGTATTCCCTGTCAA
ACGGCACGTACAAACAGCATATCTCTCTGGAACAAGTTCCGTCGAATCCGAACA
GCTACTTTGTTAAAGTCAAATCATCGAGCTTCAAAGATGTGTATCTGCCGGTTGC
ATCGATCAGCGAAGAACGTAAAAATGACAAAATCCTGTACAAAATCACCGCTAA
AGTGGAAAAACTGCAGCAAGAAATCGAAAGCCGCTATAAAGATAACTTTACGTT
CTACCTGGCGAAAAAAGGTACCGAAGAAACCACGAATTTTACGTCTTTCAGTAA
CCTGGTTAAAGCGATTAATCAGAACCCGTCAGGCACCTACCACCTGGCAGCTTCG
CTGAATGCCAACGAAGTGGAACTGGGTCCGGACGAACGTAGCTATATTAAAGAT
ACCTTCACGGGTCGCCTGATCGGCGAAAAAGATGGTAAAAACTACGCCATCTAC
AACCTGAAAAAACCGCTGTTTGAAAATCTGTCTGGCGCGACCGTTGAAAAACTGT
CGCTGAAAAACGTCGCCATTAGCGGTAAAGATGACATCGGCTCTCTGGCGAATG
AAGCCCAGAACAATACCAAAATCAAACAAGTGCATGTTGATGGCGTGCTGGCAG
GTGAACGTGGCATTGGCGGTCTGCTGGCGAAAGCCGAACAGTCTAGTATCACCG
AATCCTCATTTAAAGGTCGCATTATCAATACGTATGAAACCACGGCGGCCTACAA
CATTGGCGGTATGGTTGGCCACCTGACCGGTGATAAAGCCCTGCTGACCAAAAG
CAAAGCAACGGTGGCTATCTCGAGCAATACCAACACGTCTGATCAGACCGTTGG
CGGTCTGGCTGGTCTGGTCGATCGTGACGCGCAGATTCAAGACTCTTATGCCGAA
GGCGATATCAACAATGTTAAACATTTTGGTCGCGTGGCGGGTGTTGCCGGCAATC
TGTGGGACCGTACCAGTGGCGATGTTCGCCACGCAGGTTCGCTGACGAATGTCCT
GAGCGATGTCAACGTGACCAATGGTAACGCTATTACGGGCTACCATTACAACGA
AATGAAAGTGAAAGATACCTTCTCTAGTAAAGCGAATCGTGTCTATAACGTGAC

GCTGGTTAAAGATGAAGTCGTGTCAAAAGAATCGTTTGAAGAACGCGGCACCAT
GCTGGACGCATCTCAGATCGCTAGTAAAAAAGCGGAAATTAACCCGCTGATCCT
GCCGACCGTTGAACCGCTGTCCACGTCAGGTAAAAAAGATAGCGACTTCTCTAA
AGTGGCCTATTACCAGGCAAAACGTAACCTGACCTACAAAAACATCGAAAAACT
GCTGCCGTTCTACAACAAAGCCACGATCGTCAAATACGGCAACCTGGTGAATGA
AAACAGCCTGCTGTATCAGAAAGAACTGCTGTCTGCGGTGATGATGAAAGACAA
TCAGGTCATTACCGATATCGTGAGTAATAAACAAACGGCCAACAAACTGCTGCT
GCATTATAAAGATGACCTGTCCGAAAAACTGGACCTGAAATATCAGAACGATTT
CGCAAAACTGGCTGAATACTCACTGGGTAATACCGGCCTGCTGTATACGCCGAAC
CAGTTTCTGTACGATCAAACCTCGATTATCAAACAGGTGCTGCCGGACCTGCAAA
AAGTTGATTACCACTCAGAAGCAATTCGCAAAACCCTGGGTATCTCGCCGAATGT
GAAACAGACGGAACTGTATCTGGAAGATCAATTTGCTAAAACCAAACAGCAACT
GGAAGACTCTCTGAAAAAACTGCTGAGTGCAGATGCTGGCCTGGCGTCTGCCAA
TCCGGTTACCGAAGGTTACCTGGTCGATAAAATTAAACGTAACAAAGAAGCCCT
GCTGCTGGGCCTGACCTATCTGGAACGCTGGTACAATTTCAGCTATGGTCAGGTT
AACGTCAAAGACCTGGTGCTGTATCATCTGGATTTCTTTGGTAAAGGCAACGCAA
GTCCGCTGGATACCCTGATTGAACTGGGTAAATCCGGCTTTAACAATCTGCTGGC
GAAAAACAATGTTGACACCTACGGCATCAGTCTGGCCTCCCAGCATGGTACCAC
GGACCTGTTTAGCACGCTGGAACACTATCGTAAAGTGTTCCTGCCGAATACCAGT
AACAATGATTGGTTTAAATCCGAAACGAAAGCGTATATTGTGGAAGAAAAAAGC
ACCATCGAAGAAGTTAAAACGAAACAGGGTCTGGCGGGCACCAAATACAGTATT
GGCGTTTATGATCGCATCACCAGCGCCACGTGGAAATATCGCAACATGGTGCTGC
CGCTGCTGACCCTGCCGGAACGCTCTGTGTTCGTTATTAGTACGATGTCCTCACTG
GGTTTTGGCGCCTACGACCGTTATCGCTCGAGCGATCATAAAGCGGGTAAAGCCC
TGAATGATTTTGTGGAAGAAAACGCTCGTGAAACCGCGAAACGTCAGCGCGACC
ACTATGATTACTGGTATCGCATTCTGGATGACAATGCGCGTGAAAAACTGTATCG
CAACATCCTGCTGTACGATGCCTATAAATTCGGCGATGACAATACCGTGGGTAAA
GCCACGGAAGTTGCAGATTTTGACAATCCGAACCCGGCGATGCAACATTTCTTTG
GTCCGGTCGGCAATAAAGTGGGTCATAACCAGCACGGCGCGTATGCGACCGGTG
ATGCAGTGTATTACATGGGCTACCGTATGCTGGATAAAGACGGTGCTATTACCTA
TACGCATGAAATGACCCACGATAGTGACCAGGATATCTACCTGGGCGGTTATGGT
CGTCGCTCCGGTCTGGGCCCGGAATTTTTCGCTAAAGGTCTGCTGCAGGCGCCGG
ATCATCCGGATGACGCAACCATTACGATCAATTCAATTCTGAAACACTCGAAAAG
CGACTCTACCGAATCGCGTCGCCTGCAAGTTCTGGATCCGACCACGCGCTTCAAC

AATGCGGATGACCTGAAACAGTATGTGCATAACATGTTTGATGTTGTCTACATGC
TGGAATATCTGGAAGGCAATAGCATTCTGAAACTGGACACCAACCAGAAACAGC
AACTGCTGCGTAAAGTTACGAATGAATATCACCCGGACCCGGATGGTAACAAAG
TGTACGCGACCAATGTGGTTCGCAACCTGACGGTCGAAGAAGTGGAACGTCTGC
GCTCATTCAACGACCTGATTGATAACAATATCCTGTCTAGTCGTGAATACGCATC
GGGCAAATATGAACGCAATGGTTACTTTACCATTAAACTGTTCGCTCCGATCTAC
GCAGCTCTGAGTAACGACATTGGTACGCCGGGTGATCTGATGGGTCGTCGCATCG
CCTATGAACTGCTGGCGGCCAAAGGCTTTAAAGATGGTATGGTTCCGTATATTAG
CAATCAGTACGAAGAAGAAGCGAAACAAAAAGGTAAAACCATCAACCTGTATG
GCAAAACCCGTGGTCTGGTGACGGATGACCTGGTTCTGGAAAAAGTCTTCAACA
ATCAATACCATACCTGGAGCGAATTTAAAAAAGCGATGTATCAGGAACGTCAGG
ACCAATTTGATCGCCTGAATAAAGTTACCTTCAACGATACCACGCAGCCGTGGCA
AACGTTTGCGAAGAAAACCACGTCCTCAGTGGATGAACTGCAGAAACTGATGGA
CGTCGCCGTGCGTAAAGATGCAGAACATAACTACTACCACTGGAACAACTACAA
CCCGGACATTGATAGCGAAGTGCACAAACTGAAACGCGCGATCTTCAAAGCGTA
CCTGGACCAAACCAATGACTTCCGTTCTAGTATCTTCGAAAACAAAAAAAGATCT
AGATCTGGATCAAGCGGGTCATCTGGAGAATCGAAGTACGGTCCGCCGTGTCCG
AGTTGCCCGGCTCCGGAGTTTCTGGGTGGCCCTAGCGTGTTCTTATTTCCGCCAA
AGCCGAAGGACACCCTGATGATCAGCCGCACCCCGGAGGTAACTTGCGTCGTCG
TGGATGTTTCCCAAGAAGATCCGGAGGTGCAGTTTAACTGGTATGTGGACGGCGT
TGAAGTTCATAATGCGAAAACCAAGCCGCGTGAAGAGCAGTTCAACAGCACCTA
TCGTGTGGTCTCCGTGTTGACGGTTCTTCACCAGGATTGGTTGAATGGTAAAGAA
TATAAGTGCAAAGTTTCCAATAAAGGCCTGCCGTCGAGCATTGAAAAAACGATT
AGCAAGGCAAAGGGCCAACCTCGCGAGCCGCAAGTGTACACTCTGCCGCCGAGC
CAAGAGGAAATGACCAAAAACCAGGTTTCATTGACCTGCCTGGTTAAGGGTTTTT
ACCCGAGCGACATCGCCGTTGAGTGGGAATCTAACGGCCAGCCGGAGAACAACT
ACAAGACCACCCCACCGGTTCTGGACAGCGATGGTTCCTTCTTCCTGTACTCCCG
TCTCACCGTGGACAAAAGCCGTTGGCAAGAGGGAAACGTGTTCTCTTGTAGCGTG
ATGCATGAAGCGCTGCACAATCATTATACGCAGAAATCTCTGAGCCTTTCTCTGG
GCAAA (SEQ ID NO: 25)

### Vectors and Cells

In another aspect, the present disclosure provides a vector comprising the nucleic acid encoding the truncated form of IgA protease or the fusion protein described herein.

The isolated polynucleotide that encodes the truncated form of IgA protease or the fusion protein described herein can be inserted into vector for further cloning (amplification of the DNA) or for expression, using recombinant techniques known in the art. Many vectors are available. The vector components generally include, but are not limited to, one or more of the followings: a signal sequence, an origin of replication, one or more marker genes, an enhancer element, a promoter (e.g., SV40, CMV, EF-1α), a transcription stop sequence.

In some embodiments, the nucleic acid described herein encodes the truncated form of IgA protease or the fusion protein, with at least one promoter (e.g., SV40, CMV, EF-1α) operably linked to the nucleic acid sequence, and at least one selection marker. Examples of vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40), lambda phage, and M13 phage, plasmid pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, pEF-Bos, etc.

Vectors comprising the nucleic acid sequence encoding the truncated form of IgA protease or the fusion protein described herein can be introduced to a host cell for cloning or gene expression. Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, *Enterobacteriaceae* such as *Escherichia* (e.g., *E. coli), Enterobacter, Erwinia, Klebsiella, Proteus, Salmonella* (e.g., *Salmonella typhimurium), Serratia* (e.g., *Serratia marcescans),* and *Shigella,* as well as *Bacilli* such as *B. subtilis* and *B. licheniformis, Pseudomonas* such as *P. aeruginosa,* and *Streptomyces.* In some embodiments, the cell is a *E.coli* cell.

In addition to prokaryotes, eukaryotic microbes such as filamentous fungi or yeast are also suitable cloning or expression hosts for the vectors encoding the fusion protein described herein. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e.g. K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; yarrowia* (EP 402,226); *Pichia pastoris* (EP 183,070*); Candida; Trichoderma reesia* (EP 244,234); *Neurospora crassa; Schwanniomyces* such as *Schwanniomyces occidentalis;* and filamentous fungi such as, *e.g. Neurospora, Penicillium, Tolypocladium,* and *Aspergillus* hosts such as *A. nidulans* and *A. niger.* In some embodiments, the eukaryotic cell is a mammalian cell. In some embodiments, the mammalian cell is a human cell or a Chinese hamster ovary (CHO) cell. In some embodiments, the mammalian cell is a human embryonic kidney cell 293 (HEK293 cell).

### Pharmaceutical Compositions

In another aspect, the present disclosure provides a pharmaceutical composition comprising the truncated form of IgA protease described herein, comprising the fusion protein described herein, comprising the nucleic acid described herein, comprising the vector described herein or comprising the cell described herein, and a pharmaceutically acceptable carrier.

Pharmaceutical acceptable carriers for use in the pharmaceutical compositions disclosed herein may include, for example, pharmaceutically acceptable liquid, gel, or solid carriers, aqueous vehicles, nonaqueous vehicles, antimicrobial agents, isotonic agents, buffers, antioxidants, anesthetics, suspending/dispending agents, sequestering or chelating agents, diluents, adjuvants, excipients, or non-toxic auxiliary substances, other components known in the art, or various combinations thereof.

Suitable components may include, for example, antioxidants, fillers, binders, disintegrants, buffers, preservatives, lubricants, flavorings, thickeners, coloring agents, emulsifiers or stabilizers such as sugars and cyclodextrins. Suitable antioxidants may include, for example, methionine, ascorbic acid, EDTA, sodium thiosulfate, platinum, catalase, citric acid, cysteine, thioglycerol, thioglycolic acid, thiosorbitol, butylated hydroxanisol, butylated hydroxytoluene, and/or propyl gallate. As disclosed herein, inclusion of one or more antioxidants such as methionine in a composition comprising the truncated form of IgA protease or the fusion protein as described herein decreases oxidation of the truncated form of IgA protease or the fusion protein. Further provided are methods for preventing oxidation of, extending the shelf-life of, and/or improving the efficacy of a truncated form of IgA protease or a fusion protein as described herein by mixing the fusion protein with one or more antioxidants (e.g., methionine).

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

The pharmaceutical compositions can be a liquid solution, suspension, emulsion, pill, capsule, tablet, sustained release formulation, or powder. Oral formulations can include standard carriers such as pharmaceutical grades of mannitol, lactose, starch, magnesium stearate, polyvinyl pyrollidone, sodium saccharine, cellulose, magnesium carbonate, etc.

In certain embodiments, the pharmaceutical compositions are formulated into an injectable composition. The injectable pharmaceutical compositions may be prepared in any conventional form, such as for example liquid solution, suspension, emulsion, or solid forms suitable for generating liquid solution, suspension, or emulsion. Preparations for injection may include sterile and/or non-pyretic solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use, and sterile and/or non-pyretic emulsions. The solutions may be either aqueous or nonaqueous.

In certain embodiments, unit-dose parenteral preparations are packaged in an ampoule, a vial or a syringe with a needle. All preparations for parenteral administration should be sterile and not pyretic, as is known and practiced in the art.

In some embodiments, a sterile, lyophilized powder is prepared by dissolving the truncated form of IgA protease or the fusion protein as disclosed herein in a suitable solvent. The solvent may contain an excipient which improves the stability or other pharmacological components of the powder or reconstituted solution, prepared from the powder. Excipients that may be used include, but are not limited to, water, dextrose, sorbital, fructose, corn syrup, xylitol, glycerin, glucose, sucrose or other suitable agents. The solvent may contain a buffer, such as citrate, sodium or potassium phosphate or other such buffer known to those of skill in the art at, in one embodiment, about neutral pH. Subsequent sterile filtration of the solution followed by lyophilization under standard conditions known to those of skill in the art provides a desirable formulation. In one embodiment, the resulting solution will be apportioned into vials for lyophilization. Each vial can contain a single dosage or multiple dosages of the truncated form of IgA protease or the fusion protein or composition thereof. Overfilling vials with a small amount above that needed for a dose or set of doses (e.g., about 10%) is acceptable so as to facilitate accurate sample withdrawal and accurate dosing. The lyophilized powder can be stored under appropriate conditions, such as at about 4 °C to room temperature.

Reconstitution of a lyophilized powder with water for injection provides a formulation for use in injection administration. In one embodiment, for reconstitution the sterile and/or non-pyretic water or other liquid suitable carrier is added to lyophilized powder. The precise amount depends upon the selected therapy being given, and can be empirically determined.

### Methods of Treating or Preventing Diseases

In another aspect, the present disclosure provides a method of treating or preventing a disease associated with IgA deposition, comprising administering to a subject in need thereof the truncated form of IgA protease described herein, the fusion protein described herein, or the pharmaceutical composition described herein.

In another aspect, the present disclosure provides a method of treating or preventing a disease associated with IgA deposition, comprising administering to a subject in need thereof an IgA protease or the truncated form thereof, a fusion protein comprising the IgA protease or the truncated form thereof, or the pharmaceutical composition comprising the IgA protease or the truncated form thereof or the fusion protein, wherein the amino acid sequence of the truncated form of IgA protease is selected form the group consisting of: SEQ ID NO: 26, SEQ ID NO: 27, SEQ ID NO: 28, SEQ ID NO: 29, SEQ ID NO: 30 or the combination thereof. In some embodiments, the truncated form of IgA protease have at least 70% *(e.g.,* at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, 95%, 96%, 97%, 98%, or 99%) sequence identity to the polypeptide having an amino acid sequence as set forth in SEQ ID NO: 26, SEQ ID NO: 27, SEQ ID NO: 28, SEQ ID NO: 29 or SEQ ID NO: 30. In some embodiments, the truncated form of IgA protease has at least 70% sequence identity (e.g., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the polypeptide having an amino acid sequence as set forth in SEQ ID NO: 26, SEQ ID NO: 27, SEQ ID NO: 28, SEQ ID NO: 29, SEQ ID NO: 30, and still retains the function or activity (such as, proteolytic activity or enzymatic activity of specifically cleaving IgA) of IgA protease.

The amino acid sequences of SEQ ID NOs: 26~30 are shown in the following table.

| **SEQ ID NO** | **Amino Acid Sequence** |
|---|---|
| 26 | QETAGVHYKYVADSELSSEEKKQLVYDIPTYVENDDETYYLVYK LNSQNQLAELPNTGSKNERQALVAGASLAALGILIFAVSKKKVK NKTVLHLVLVAGIGNGVLVSVHALENHLLLNYNTDYELTSGEKL PLPKEISGYTYIGYIKEGKTTSDFEVSNQEKSAATPTKQQKVDYN VTPNFVDHPSTVQAIQEQTPVSSTKPTEVQVVEKPFSTELINPRKE EKQSSDSQEQLAEHKNLETKKEEKISPKEKTGVNTLNPQDEVLS GQLNKPELLYREETIETKIDFQEEIQENPDLAEGTVRVKQEGKLG KKVEIIRIFSVNKEEVSREIISTSTTAPIPRIVEKGTKKTQIIKEQPET GVEHKDVQSGAIVEPAIQPELSEAVVSDKGVPEVQPALPEAVVSD KGEPEQVATLPEYKGNIEQVKPETPVEKTKEEVPVKPTEETPVNP |

| | |
|---|---|
| | NEGTTEGTSIQEAENPVQPAEESTTNSEKVSPDTSSENTGEVSSNP SDSTTSVGESNKPEHNDSKNENSEKTVEEVPVNPNEGTVEGTSN QETEKPVQPAEETQTNSGKIANENTGEVSNKPSDSKPPVEESNQP EKNGTATKPENSGNTTSENGQPEPEPSNGNSTEDVSTESNTSNSN GNEEIKQENELDPDKKVEDPEKKLELRNVSDLELYSLSNGTYKQ HISLEQVPSNPNSYFVKVKSSSFKDVYLPVASISEERKNDKILYKIT AKVEKLQQEIESRYKDNFTFYLAKKGTEETTNFTSFSNLVKAINQ NLSGTYRLGASLNANEVELGPDERSYIKDTFTGRLIGEKDGKNY AIYNLKKPLFENLSGATVEKLSLKNVAISGKDDIGSLANEAQNNT KIKQVHVDGVLAGERGIGGLLAKADQSSITESSFKGRIVNTYETK SAYNIGGLVGHLTGDRASINSSKSTVVISSNTNSSDQTVGGISGLV DKDAHIQNSYSEGDINNSQRFGKVAGIAGNLWDRESNSENHAGR LTNVLSDVNVTNGNAISGYHYNGMKITDAFSNKANKVFNVTLE KDEVVSKESFEERGTMLDASQIASKKAEINLLTPPIVEPLSTSGKK DSDFSKIAHYQANRALVYKNIEKLLPFYNKTTIVKYGNLVNENSL LYQKELLSAVMMKDNQVITDIVSNKQTANKLLLHYKDDLSEKL DLKYQNDFAKLAEYSLGNTGLLYTPNQFLYDQTSIIKQVLPDLQ KVDYHSEDIRKTLGISPNVKQTELYLEDQFAKTKQQLEDSLKKLL SADAGLASANPVTEGYLVDKIKRNKEALLLGLTYLERWYNFSYG QVNVKDLVLYHLDFFGKGNASPLDTLIELGKSGFNNLLAKNNVD TYGISLASQHGTTDLFSTLEHYRKVFLPNTSNNDWFKSETKAYIV EEKSTIEEVKTKQGLASTKYSIGVYDRITSATWKYRNMVLPLLTL PERSVFVISTMSSLGFGAYDRYRSSDHKAGKALNDFVEENARET AKRQRDHYDYWYRILDDNAREKLYRNILLYDAYKFGDDNTVGK ATEVADFDNPNPAMQHFFGPVGNKVGHNQHGAYATGDAVYYM GYRMLDKDGAITYTHEMTHDSDQDIYLGGYGRRSGLGPEFFAK GLLQAPDHPDDATITINSILKHSKSDSTESRRLQVLDPTTRFNNAD DLKQYVHNMFDVVYMLEYLEGNSILKLDTNQKQQLLRKVTNE YHPDPDGNKVYATNVVRNLTVEEVERLRSFNDLIDNNILSSREYA SGKYERNGYFTIKLFAPIYAALSNDIGTPGDLMGRRIAYELLAAK GFKDGMVPYISNQYEEEAKQKGKTINLYGKTRGLVTDDLVLEKV FNNQYHTWSEFKKAMYQERQDQFDRLNKVTFNDTTQPWQTFA KKTTSSVDELQKLMDVAVRKDAEHNYYHWNNYNPDIDSEVHK |

| | |
|---|---|
| | LKRAIFKAYLDQTNDFRSSIFENKK |
| 27 | QETAGVHYKYVADSELSSEEKKQLVYDIPTYVENDDETYYLVYK LNSQNQLAELPNTGSKNEMQALVAGASLATLGILIFAVSKKKVK NKTVLHLVLVAGIGNGVLVSVHALENHLLLNYNTDYELTSGEKL PLPKEISGYTYIGYIKEGKTTSDFEVSNQEKSAATPTKQQKVDYN VTPNFVDHPSTVQAIQEQTPVSSTKPTEVQVVEKPFSTELINPRKE EKQSSDSQEQLAEHKNLETKKEEKISPKEKTGVNTLNPQDEVLS GQLNKPELLYREETIETKIDFQEEIQENPDLAEGTVRVKQEGKLG KKVEIVRIFSVNKEEVSREIVSTSTTAPSPRIVEKGTKKTQVIKEQP ETGVEHKDVQSGAIVEPAIQPELPEAVVSDKGEPEVQPTLPEAVV TDKGETEVQPESPDTVVSDKGEPEQVATLPEYKGNIEQVKPETPV EKTKEEVPVKPTEEVPVKPTEETPVNPNEGTTEGTSIQEAENPVQ PAEESTTNSEKVSPDTSSENTGEVSSNPSDSTTSVGESNKPEHNDS KNENSEKTVEEVPVNPNEGTVEGTSNQETEKPVQPAEETQTNSG KIANENTGEVSNKPSDSKPPVEESNQPEKNGTATKPENSGNTTSE NGHPEPEPSNGNSTEDVSTESNTSNSNGNEEIKQENELDPDKKVE DPEKKLELRNVSDLELYSLSNGTYKQHISLEQVPSNPNSYFVKVK SSSFKDVYLPVASISEERKNDKILYKITAKVEKLQQEIESRYKDNF TFYLAKKGTEETTNFTSFSNLVKAINQNLAGTYHLAASLNANEV ELANTDNAYIKGTFTGKLIGKNNGKHYAIYNLKKSLFDSLSNATV ENLSLKSVNISGKNDIGSLANEAKNGTTINEVHVDGLLGGERGIG GLVAKVDQSNITNSSFTGRIVNTYETKSAYNIGGLVGHLTGDRASI NSSKSTVVISSNTNSSDQTVGGIAGLVDKDAHIQNSYSEGDINNS QRFGKVAGIAGNLWDRESNSENHAGRLTNVLSDVNVTNGNAIS GYHYNGMKITDAFSNKANKVFNVTLEKDEVVSKESFEERGTML DASQIASKKAEINLLTPPIVEPLSTSGKKDSDFSKIAHYQANRALV YKNIEKLLPFYNKATIVKYGNLVKENSILYQKELLSAVMMKDDQ VITDIISNKQTANKLLLHYKDHSSEKLELKYKDDFANLAEYSIGD SGLLYTPNQFLYDQTSIINQVLPELNRVNYQSDAVRNTLGISPEVK LTELYLEEQFTKTKQDLANSLKKLLSADAGLAGDNPVTRGYLVD KIKNNKEALLLGLTYLERWYNFSYGQVNVKDLVMYHPDFFGKG NTSPLDTLIELGKSGFNNLLAKNNVDTYAISLASHHGTTDLFSTL ENYRKVFLPDKTNNDWFKSQTKAYIVEEKSNIEEVKTKQELVGT |

| | |
|---|---|
| | KYSIGVYDRITSATWKYRNMVLPLLTLPEKSVFVISTISSLGFGAY DRYRNSEHKAGKDLNDFVEENARETAKRQRDHYDYWYRILDN NAREKLYRNILLYDAYKLGDDNTVGKATKVASFDDPNPAMKYFF GPVGNKVGHNGHGAYATGDAVYYMGYRMLDKDGAITYTHEM THDSDQDIYLGGYGRRSGLGPEFFAKGLLQAPDQPSDATITINSIL KHKTSDSTEGQRLQVLDPTTRFNDAADLQNYVHNMFDLIYMLE YLEGQSIVNKLSVYQKMAALRKIENKYVKDPADGNEVYATNVV KELTEAEARNLNSFESLIDHNILSAREYQSGDYERNGYYTIKLFA PIYSALSSEKGTPGDLMGRRMAYELLAAKGFKDGMVPYISNQFE ADARANNKTITSYGKTKGLVTDTLVLQKLFNGQYNTWSDFKKA MYKERQDKFNKLNKISFKDPSQPWTRNIIKTIHSVNELQNLMNE AVRKDTETPHWYNYNPEIDSAVHKLKRAIFKAYLDQTNDFRSSIF ENKK |
| 28 | QENVAVHYKYVTDTELSGQEKDLIVKDIPKIAEDSESTYYLVYR MDEKAQLGQLPNTGGQNSLTSVLTGGALASIGLLIFVVSKKKGK KKALLNVILVTGMGSGLASSVQAIENQLLLQYNQEYQLSQGDSL PLPRALSGYTYLGYIKQDKEINQQETAARDQKLDYTVQPHFQAN EGGQKAGDEQKAPSSTSPADKTIPSQDLSNKKPSGIASVDPQDEV LAGRVNKPELLYKDQEIVTKLDVSEVVQENPDLAEGTIHVKQEG RAGKKIELVRIFTVENQEISREVLSTKVEEALPRIVEKGTKKAVAA SEAPQSARKGEPETQAPLPEYTGEQAGAVVAPETAEKPEYTGTQ AGAVVEPEQVAPLPEYQGTQAGAIVEPEQVEPEVGGVQSGALVE PETTDKPAYTGEQSGAIVEPEQVPPTPEYTGTQAGAIVDPEIAEKP EYTDTQSGAIVEPETQSSLPEYKGEQAGAIVEPEQVAPLPEYTGN TEQVKPETPTEKPKEKDPEKTLELRNVSDLELYSQTNGTYKQHV SLDGVPSNPDTYFVKVKSSSFKDVYLPVASITAEIKNGQPVYKITA KAEKLQQELENKYVDNFTFYLAKKAREETTTFTSFSNLVKAINQ NLSGTYHLAASLNANEVELEPEAKSYIKGTFTGQLIGEKDGKQY AIYNLKKPLFETLSSATVEKLSLKNVSISGKDDIGALSYEAQNGT KIQQVHVDGVLAGERGIGGLLAKAEQSSITESSFKGRIINTYETTA AYNIGGLVGHLTGDKALLTKSKATVAISSNTNSSDQTVAGLAGLV DQDAQIQDSYAEGDINNAKHFGRVAGVAGYLWDRKTNEEQHAG RLTNVLSDVNVTNGNAITGYHYNGMKVKDTFSSKANRVYNVTL |

| | |
|---|---|
| | VKDEVVSKESFEERGTMLDASQIESKKAAINPLTLPTVEPLSTSG KKDSDFSKVAHYQAKRALAYKNIEKLLPFYNKATIVKYGNLVNE NSLLYQKELLSAVMMKDNQVITDIVSNKETANKLLLHYKDHSSE KLNLKYQDDFAKLAEYSLGDTGLLYTPNQFLYDQSSIIKQVLPDL QKIDYHSEAIRKTLGISPKVEQTELYLEDQFAKTKQHLEDSLKKL LSADAGLAGANPVTKGYLVDKIKRNKEALLLGLTYLERWYNFS YGQVNVKDLVLYHLDFFGKGNASPLDTLIELGKSGFNNLLAKNN VDTYGISLANQHGTTDLFSTLENYRKVFLPTTSNNDWFKSETKA YIVEEKSTIEEVKAKQGLAGTKYSVGVYDRITSATWKYRNMVLP LLTLPEKSVFVISTMSSLGFGAYDRYRNSDHKAGKALNDFVEEN ARETAKRQRDHYDYWYRILDNEGREKLYRTILLYDAYKFGDDTT SGKATLEAKFDSSNPAMKNFFGPVGNKVVHNQHGAYATGDGVY YMSYRMLDKDGAITYTHEMTHDSDQDIYLGGYGRRSGLGPEFF AKGLLQAPDQPSDATITINSILRHSKSDSAEGSRLQVLDPTERFQN ASDLQSYVHNMFDLIYMLEYLEGQSIVNKLNVYQKMAALRKIE NKYVKDPADGNEVYATNVVKELTEAEAQNLNSFESLIDHNILSA REYQSGEYERNGYYTIKLFAPIFSALSSEKGTPGDLMGRRIAYEL LAAKGFKDGMVPYISNQYEEVAKQKGKTINLYGKERGLVTDKL VLDKVFEGKYSSWANFKKAMYKERVDQFENLKQVTFKDPTKP WPSYATKTINSVTELQELMDQAVLQDAVAPRWSNYNPEYDSAVH KLKRAIFKAYLDQTKDFRTSIFKK |
| 29 | QENVAVHYKYVTDTELSRQEKDLIVKDIPKIAEDSESTYYLVYR MDEKAQLGQLPNTGGQHSLTSVLSGGVMASIGLLIFVVSKKKGK KKALLNVILITGMGSGLVSSVQAIENQLLLQYNQEYQLSQGDSLP LPRTLSGYTYLGYIKQDKDASLQETAARGQKPDYTVQPRFQTNE GGQKAGDEQKATSPTTPAEKPIPSQDSSNQNPSGLASVDPQDEVL AGRVNKPELLYKDQEIVTKLDFSEVVQENSELAEGTIRVKQEGH AGKKVEVVRIFTVENQEISREVLSTNVEEALPRIVEKGTKKAVVP SEASQSARKGEPETQASLPEYKGEQAGAIVAPETAEKPEYTGTQA GAVVEPEQVAPQPEYQGTQAGAIVEPEQVEPEVGGVQSGALVEP EMAEKPSYTGEQSGAIVEPEQVPPTQEYTETQAGAVVAPETTETP EYTDNQSGAIVEPEIQSSLPEYKGEQAGAIVEPEQVAPLPEYTGN TEQVKPEAPTEKPKEKDPEKTLELRNVSDLELYSQTNGTYKQHV |

| | |
|---|---|
| | SLDSVPKNTETYFVKVKSSAVKDVYLPVSSITAETKNGQSVYKIT AKAEKLQQELENKYVDNFTFYLAKKATEETTTFTSFSNLVKAIK QNLSGTYYLAASLNANEVELDPDEKSYIKGTFTGQLIGEKDGKQ YAIYNLKKPLFETLSGARVEKLSLKNVSISGKDDIGSLAYEAQNG TKIKQVHIDGVLAGERGIGGLLAKADQSSITESSFKGRIINTYETT AAYNIGGLVGHLTGDKALLSKSKATVAISSNTNTSDQTVGGLAGL VDQDAQIQNSYAEGDINNVKHFGRVAGVAGNLWDRTSGDVKYA GSLTNVLSDVNVTNGNAITGYHYNGMKVKDTFSSKANRVYNVT LFKDEVVSRESFEERGTMLDASQIESKKAAINPLTLPTVEPLSTSG KKDSDFSKVTHYQAKRALAYKNIEKLLPFYNKATIVKYGNLVNE NSLLYQKELLSAVMMKDNQVITDIVSNKETANKLLLHYKDHSSE KLNLKYQDDFAKLAEYSLGDTGLLYTPNQFLYDQSSIIKQVLPDL QKVDYHSEAIKETLGISPNVKQTELYLEDQFAKTKEQLEDSLKKL LSADAGLAGDNPVTKGYLVDKIKHNKEALLLGLTYLERWYNFS YGQVNVKDLVLYHLDFFGKGNASPLDALIELGKSGFNNLLAKN NVDTYGISLASQHGTTDLFSTLEHYRKVFLPEKSNNDWFKSQTK AYIVEEKSTIEEVKTKQGQTGSKYSIGVYDRITSATWKYRNMVLP LLTLPERSVFVISTMSSLGFGAYDRYRNSEHKAGKALNDFVEEN ARETAKRQRDHYDYWYRILDNEGREKLYRTILLYDAYKFGDDTT SGKATVEAKFDSSNPAMKNFFGPVGNKVVHNQHGAYATGDGVY YMSYRMLDKDGAITYTHEMTHDSDQDIYLGGYGRRSGLGPEFF AKGLLQAPDQPSDATITINSILKHSTSDSTEGSRLQVLDPTERFQK AADLQNYVHNMFDLIYMLEYLEGQSIVNKLSVYQKMAALRKIE NKYVKDPSDGNEVYATNVVKELTAEEAQKLTSFESLIDHNILSAR EYQSGEYERNGYYTIKLFAPIFSALSSEKGTPGDLMGRRIAYELL AAKGFKDGMVPYISNQYEEVAKQKGKTINLYGKERGLVTDKLV LDKVFEGKYSSWANFKKAMYKERVDQFENLKQVTFKDPTKPW PSYATKTINSVTELQELMDQAVLQDAVAPRWSDYKPEIDSAVHKL KRAIFKAYLDQTKDFRTSIFKK |
| 30 | QENVSVHYKYVTDTELSSHEKDLIVKDFPNIAEDSESTYYLVYR MDEKAQLGQLPHTGEHNNLVTALTGGALASIGLLIFAVSRKKGN KKALLKVVLITGVGSGLVSSVQAIENQLLLQYNQEYQLSQGDSL PLPRVLSGYTYLGYIKQDKKINQLETVARDQKLDDTVHPHFQSN |

| | |
|---|---|
| | EGGQNVRDERKNPSPTSPADKSIPSQDSPNQKPSGIASVDPHDEV LAGRLNKPELLYKDQEIVTKLDVLEVVQENPELAEGTIRVKQEG HVGKKVEVVRIFTVENQEITREILSTKVEKSLPRIVEKGTKKAVAP SEAPQSARKGEPETHASLPEYKGEQSGAIVAPEKTDKPSYTGTQS GAIVEPEQVAPQPEYKGIQSGAIVEPEKQEPEVGGAQSGALVEPE TADKPSYTGPQSGAVVEPEQVAPQPEYKGTQAGAIVEPETQPSLP EYKGEQSGAIVAPEKTAKPSYTGTQSGAIVEPEKVAPQPEYKGTQ AGAIVEPETHASLPEYKGEQSGAIVAPETTEKTEYTGIQSGVIVEP ETADKPSYTGTQSGAVVEPEQVAPQPEYTGTQAGTIVEPETLASL PEYKGEQSGAVVAPETADKPEYTGIQSGAIVEPEQAPSKPSYTGT QSGAIVEPEQIAPLPEYTGNIEQAKPENPAEKPKEKDPEKTLELRN VSDLELYSQTNGTYKQHVSLDSVPSNPDTYFVKVKSSSFKDVYL PVASITAEIKNGQSVYKIIAKAEKLQQELENKYVDNFTFYLAKKA TEETTTFTSFSNLVKAINKNLSGTYHLAASLNANEVELGPDDRSY IKGTFTGQLMGEKDGKQYAIYNLKKPLFATLRGATVEKLSLKNV SISGKDDIGSLANEATDNTKIKQVHVDGVLAGERGIGGLLAKAD QSSITESSFKGRIVNTYETTAAYNIGGLVGHLTGGRASLTKSKATV AISSNTNSSDQTVGGLAGLVDQDAHIQDSYAEGDINNVKHFGRV AGVAGYLWDRKTNEEQHAGRLTNVLSDVNVTNGNAITGYHYN EMKVANTFSNKANRVYNVTLVREEVVSKESFVERGTILDASEVT NKKAEITPLTPPIVEPLSTSGSKESDFSKVKHYQVKRALAYKNIEK LLPFYNKATIVKYGNLVNENSLLYQKELLSAVMMKDDQVITDIV SNKQTANKLLLHYQDHSSETFQIKYQTDFAKLAEYSLGDTGLLY TPNQFLYNQETIIQQVLPELKKVNYHSDAIRNTLGISPKVKQTELY LEEQFAKTKEHLEDSLRKLLSADAGLVENNQVMTGYIVDKIKRN KEALLLGMSYLERWYNFSYGQVNIKDLVLYHLDFFGKGNASPL DTLIELGKSGFNNLLAKNNVDTYGISLASHHGTTDLFSTLENYR KVFLPNTSNNDWFKKQTKAFIVEEKSTIAEVKAKQEQAGTKYSI GVYDRITSSTWKYRNMVLPLLTLPERSVFVISTLSSLGFGAYDRY RNSDYRAGDKLNKFVEENARETAKRQRDHYDYWYRILDNQGR EKLYRTILLYDAYKFGDDHTEGKASTIADFENSNPAMQHFFGPVG NKVVHNHHGAYATGDGVYYMSYRMLDKDGAITYTHEMTHDSD QDIYLGGYGRRSGLGPEFFAKGLLQAPDHPNDATITINSILKHSKS |

| | |
|---|---|
| | DSLEGSRLQVLDPTERFKDASDLQKYVHNMFDLIYLLEYLEGQS IVKKLNVSQKMEALRKIENKYVKDPADGNDVYATNVVKDLTEE EAKKLTSFDSLIDNNILSAREYQVGTYERNGYFTIKLFAPIFSALSS EKGTPGDLMGRRIAYELLAAKGFKDGMVPYISNQYEEIAKQNG QTINLYGKKQGLVTDKLVLEKLFGGQYSSWAAFKKAMYQERVA QFDHLNKVTFKDPTQSWMSNATKTIQRVNELQELMDQAVLKDA EGTRWSDYNPETDSAVHKLKRAVFKAYLDQTNDFRTSIFANKK |

In another aspect, the present disclosure provides use of the truncated form of IgA protease described herein, the fusion protein described herein, or the pharmaceutical composition described herein in the manufacture of a medicament for treating or preventing a disease associated with IgA deposition.

In another aspect, the present disclosure provides use of an IgA protease or the truncated form thereof, the fusion protein comprising the IgA protease or the truncated form thereof, or the pharmaceutical composition comprising the IgA protease or the truncated form or the fusion protein, in the manufacture of a medicament for treating or preventing a disease associated with IgA deposition, wherein the amino acid sequence of the IgA protease is selected form the group consisting of: SEQ ID NO: 26, SEQ ID NO: 27, SEQ ID NO: 28, SEQ ID NO: 29, SEQ ID NO: 30 or the combination thereof. In some embodiments, the amino acid sequence of the IgA protease is the amino acid sequence formed by removing the signal peptide sequence from the amino acid sequence as set forth in SEQ ID NO: 26, SEQ ID NO: 27, SEQ ID NO: 28, SEQ ID NO: 29 or SEQ ID NO: 30. In some embodiments, the truncated form of IgA protease has at least 70% (*e.g.,* at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%) sequence identity to the polypeptide having an amino acid sequence as set forth in SEQ ID NO: 26, SEQ ID NO: 27, SEQ ID NO: 28, SEQ ID NO: 29 or SEQ ID NO: 30. In some embodiments, the truncated form of IgA protease has at least 70% sequence identity (*e.g*., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the polypeptide having an amino acid sequence as set forth in SEQ ID NO: 26, SEQ ID NO: 27, SEQ ID NO: 28, SEQ ID NO: 29 or SEQ ID NO: 30, and still retains the function or activity (such as, proteolytic activity or enzymatic activity of specifically cleaving IgA) of IgA protease.

In another aspect, the present disclosure provides the truncated form of IgA protease described herein, the fusion protein described herein, or the pharmaceutical composition described herein for treating or preventing a disease associated with IgA deposition.

In another aspect, the present disclosure provides an IgA protease or the truncated form thereof, the fusion protein comprising the IgA protease or the truncated form thereof, or the pharmaceutical composition comprising the IgA protease or the truncated form thereof or the fusion protein for treating or preventing a disease associated with IgA deposition, wherein the amino acid sequence of the IgA protease is selected form the group consisting of: SEQ ID NO: 26, SEQ ID NO: 27, SEQ ID NO: 28, SEQ ID NO: 29, SEQ ID NO: 30 or the combination thereof. In some embodiments, the amino acid sequence of the IgA protease is the amino acid sequence formed by removing the signal peptide sequence from the amino acid sequence as set forth in SEQ ID NO: 26, SEQ ID NO: 27, SEQ ID NO: 28, SEQ ID NO: 29 or SEQ ID NO: 30. In some embodiments, the truncated form of IgA protease has at least 70% (*e.g.,* at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%) sequence identity to the polypeptide having an amino acid sequence as set forth in SEQ ID NO: 26, SEQ ID NO: 27, SEQ ID NO: 28, SEQ ID NO: 29 or SEQ ID NO: 30. In some embodiments, the truncated form of IgA protease has at least 70% sequence identity (*e.g*., having at least 75%, at least 80%, at least 81%, at least 82%, at least 83%, at least 84%, at least 85%, at least 86%, at least 87%, at least 88%, at least 89%, at least 90%,at least 91%, at least 92%, at least 93%, at least 94%, at least 95%, at least 96%, at least 97%, at least 98%, at least 99% sequence identity) to the polypeptide having an amino acid sequence as set forth in SEQ ID NO: 26, SEQ ID NO: 27, SEQ ID NO: 28, SEQ ID NO: 29 or SEQ ID NO: 30, and still retains the function or activity (such as, proteolytic activity or enzymatic activity of specifically cleaving IgA) of IgA protease.

In some embodiments, the disease associated with IgA deposition described herein comprises IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis. In some embodiments, the disease associated with IgA deposition described herein is IgA nephropathy. In some embodiments, the disease associated with IgA deposition described herein is IgA1 nephropathy. In some embodiments, the disease associated with IgA deposition described herein is IgA vasculitis. In some embodiments, the disease associated with IgA deposition described herein is Kawasaki disease.

### EXAMPLES

The biological materials involved in all examples, such as *E. coli* strains, various cloning and expression plasmids, culture media, tool enzymes, buffers, and various culture methods, protein extraction and purification methods, and other molecular biology manipulations, are familiar to those skilled in the art and can be found in "Molecular Cloning, Sambrook et al. (Laboratory Manual, Cold Spring Harbor, 1989)" and "A Concise Guide to Molecular Biology (F. Osborne et al., translated by Yan Ziying et al., Beijing, Science Press, 1998)".

### Example 1: Study on the active sites of TIGR4 IgA protease

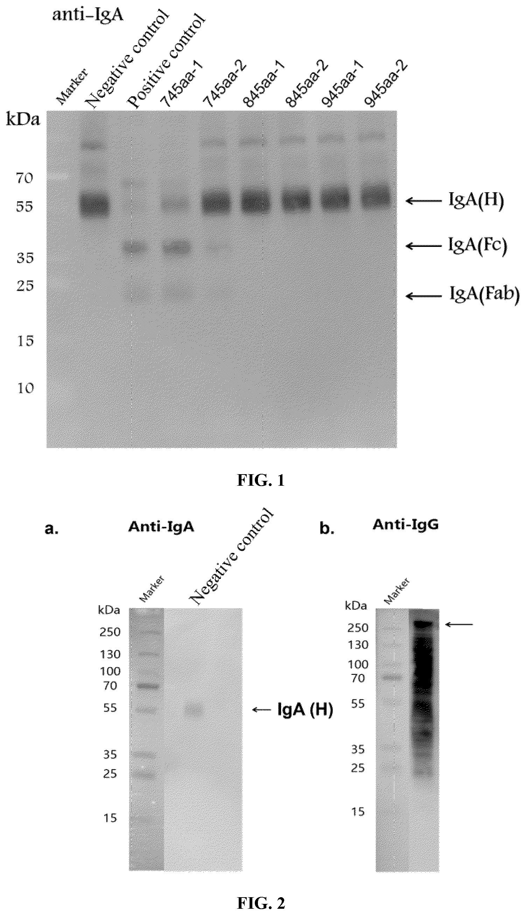

To construct a PET30a-TIGR4 plasmid, the inventors removed the signal peptide (namely, the amino acids from position 1 to position 42 of the amino acid sequence as set forth in SEQ ID NO: 1) from the N-terminus of a wild-type IgA protease (the amino acid sequence of which was as set forth in SEQ ID NO: 1) from a *Streptococcus pneumoniae* TIGR4 strain. The inventors then used the PET30a-TIGR4 plasmid as a template and constructed a series of truncated forms of TIGR4 IgA protease, which were expressed in prokaryotic cells (*Ecoli*) for activity-site study of TIGR4 IgA protease. As shown in FIG. 1, the IgA cleaving activity of the C-terminal truncated form TIGR4 (745-2004) (the amino acid sequence of which was as set forth in SEQ ID NO: 3), formed by removing amino acid residues 1 to 744 of SEQ ID NO: 1, begun to weaken; both the C-terminal truncated form TIGR4 (845-2004) (the amino acid sequence of which was as set forth in SEQ ID NO: 4) formed by removing amino acid residues 1 to 844 of SEQ ID NO: 1, and the C-terminal truncated form TIGR4 (945-2004) formed by removing amino acid residues 1 to 944 of SEQ ID NO: 1, did not have *in vitro* self-cleaving activity. Therefore, in conclusion, the active sites of the TIGR4 IgA protease were between amino acids from position 43 to position 745.

### Example 2: Preparation of fusion protein comprising the truncated form of TIGR4 IgA protease

### 2.1 Plasmid Construction

To construct a PET30a-IgG1 Fc-TIGR4 (43-2004) plasmid, the inventors removed the signal peptide (namely, amino acids from position 1 to position 42 of the amino acid sequence as set forth in SEQ ID NO: 1) from the N-terminus of a wild-type IgA protease (the amino acid sequence of which was as set forth in SEQ ID NO: 1) from a *Streptococcus pneumoniae* TIGR4 strain, and then added the Fc sequence (HR-CH2-CH3, the amino acid sequence of which was as set forth in SEQ ID NO: 13) of human IgG1 to the N-terminus of the amino acid sequence of the IgA protease with signal peptide removed (namely, the truncated form of IgA protease consisting of the amino acids from position 43 to position 2004 of the amino acid sequence as set forth in SEQ ID NO: 1). According to similar strategies, the inventors also constructed a PET30a-IgG1 Fc-TIGR4 (665-2004) plasmid.

To construct a pET30a-TIGR4 (665-2004)-IgG1 Fc plasmid, the inventors removed amino acid residues 1 to 664 of the amino acid sequence as set forth in SEQ ID NO: 1 from the N-terminus of a wild-type IgA protease (the amino acid sequence of which was as set forth in SEQ ID NO: 1) from a Streptococcus *pneumoniae* TIGR4 strain, and added the Fc sequence (the amino acid sequence of which was as set forth in SEQ ID NO: 13) of human IgG1 to the C-terminus of the truncated form of TIGR4 (665-2004) IgA protease. According to similar strategies, the inventors also constructed two pET30a-TIGR4 (665-2004)-IgG4 Fc plasmids (with different linkers).

### 2.2 Preparation Method of Fusion Proteins

The five plasmid expression vectors constructed in Example 2.1 were transformed into *E.coli* (BL21-DE3) competent cells, respectively, which were selected for resistance by LB agar dishes containing 50 µg/ml kanamycin; and then monoclonal colonies were picked into an LB culture medium containing the corresponding antibiotics and shaken till an exponential growth phase (OD600:0.6-0.8) was reached. After the exponential growth phase was achieved, 0.1-0.5 mM isopropyl-β-D-thiogalactoside (IPTG) was added for induction. The expression was induced at a low temperature of 16°C for 24 h (or at a temperature of 37°C for 3 h). The pET30a-TIGR4 (665-2004)-IgG1 Fc plasmid was also transfected into HEK293 cells of a eukaryotic expression system. After completion of expression, the *E. coli* cells were sonicated and centrifuged at high speed according to conventional methods. The supernatant was retained and then purified by affinity chromatography and molecular sieve purification to obtain the recombinant fusion protein.

The amino acid sequence of the fusion protein expressed by the PET30a-IgG1 Fc-TIGR4 (43-2004) plasmid (hereinafter referred to as "Fusion Protein 1") was as set forth in SEQ ID NO: 5, and its coding nucleic acid sequence was as set forth in SEQ ID NO: 9. The Fusion Protein 1 comprises an IgG1 Fc domain as set forth in SEQ ID NO: 13 and a truncated form TIGR4 (43-2004).

The amino acid sequence of the fusion protein expressed by the PET30a-IgG1 Fc-TIGR4 (665-2004) plasmid (hereinafter referred to as "Fusion Protein 2") was as set forth in SEQ ID NO: 6, and its coding nucleic acid sequence was as set forth in SEQ ID NO: 10. The Fusion Protein 2 comprises an IgG1 Fc domain as set forth in SEQ ID NO: 13 and a truncated form TIGR4 (665-2004) as set forth in SEQ ID NO: 2.

The amino acid sequence of the fusion protein expressed by the pET30a-TIGR4 (665-2004)-IgG1 Fc plasmid (hereinafter referred to as "Fusion Protein 3") was as set forth in SEQ ID NO: 7, and its coding nucleic acid sequence was as set forth in SEQ ID NO: 11. The Fusion Protein 3 comprises a truncated form TIGR4 (665-2004) as set forth in SEQ ID NO: 2 and an IgG1 Fc domain as set forth in SEQ ID NO: 13.

The amino acid sequence of the fusion protein expressed by one pET30a-TIGR4 (665-2004)-IgG4 Fc plasmid (hereinafter referred to as "Fusion Protein 4") was as set forth in SEQ ID NO: 8, and its coding nucleic acid sequence was as set forth in SEQ ID NO: 12. The Fusion Protein 4 comprises a truncated form TIGR4 (665-2004) as set forth in SEQ ID NO: 2 and an IgG4 Fc domain as set forth in SEQ ID NO: 14;

The amino acid sequence of the fusion protein expressed by the other pET30a-TIGR4 (665-2004)-IgG4 Fc plasmid (hereinafter referred to as "Fusion Protein 5") was as set forth in SEQ ID NO: 24, and its coding nucleic acid sequence was as set forth in SEQ ID NO: 25. The Fusion Protein 5 comprises a truncated form TIGR4 (665-2004) as set forth in SEQ ID NO: 2 and an IgG4 Fc domain as set forth in SEQ ID NO: 14.

### 2.3 In Vitro Activity Assay Method

The obtained Fusion Protein 1, Fusion Protein 2, Fusion Protein 3, Fusion Protein 4 and Fusion Protein 5 comprising the truncated forms of TIGR4 IgA protease were mixed *in vitro* with a substrate IgA purified from the plasma of a healthy person and subject to reaction at 37°C overnight, followed by Western blot to verify their cleaving activities against the substrate IgA.

### 2.4 In Vivo Activity Assay Method

The obtained Fusion Protein 4 and Fusion Protein 5 comprising the truncated form of TIGR4 IgA protease was injected into humanized IgA1 alpha chain knock-in (α1KI-Tg) C57BL/6 mice via tail vein. Blood samples were collected before injection, and 2 h, 1 day (d), 2 d, 3 d, 5 d and 7 d after injection, respectively, followed by Western blot validation.

### 2.5 Results

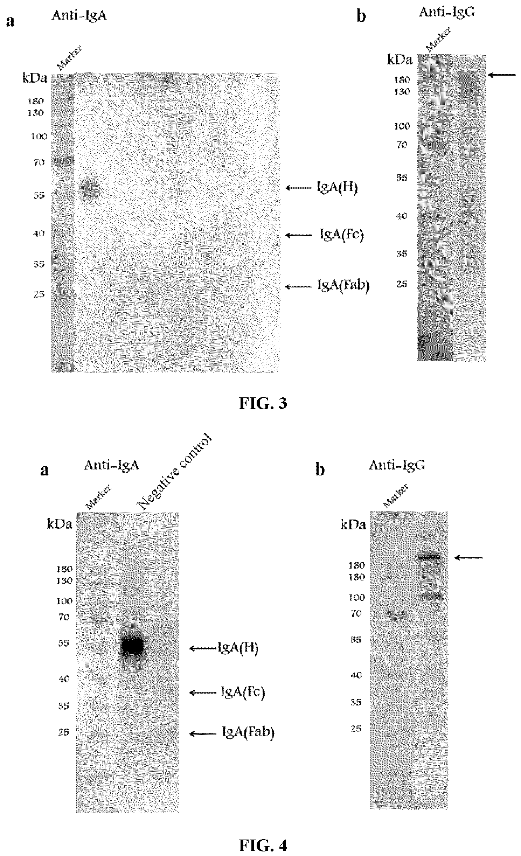

The *in vitro* activity assay showed that Fusion Protein 1 and Fusion Protein 2 had cleaving activities against IgA *in vitro* (as shown in FIG. 2a and FIG. 3a, respectively). but the Fusion Protein 1 and the Fusion Protein 2 had many non-full-length protease expressions (as shown in FIG. 2b and FIG. 3b, respectively).

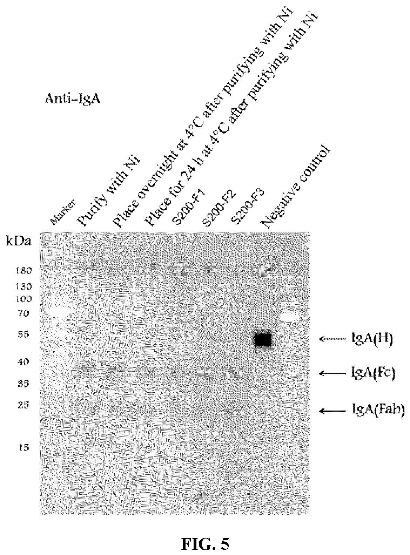

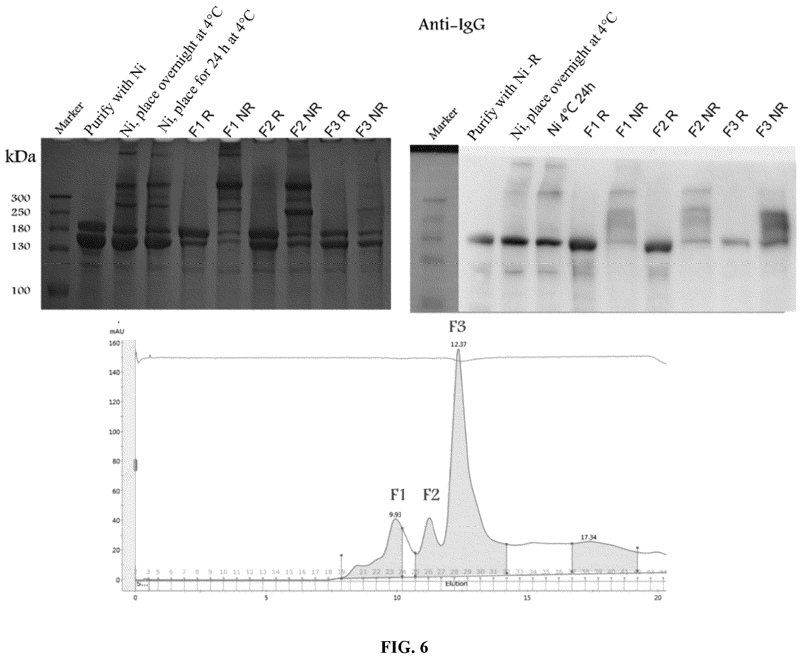

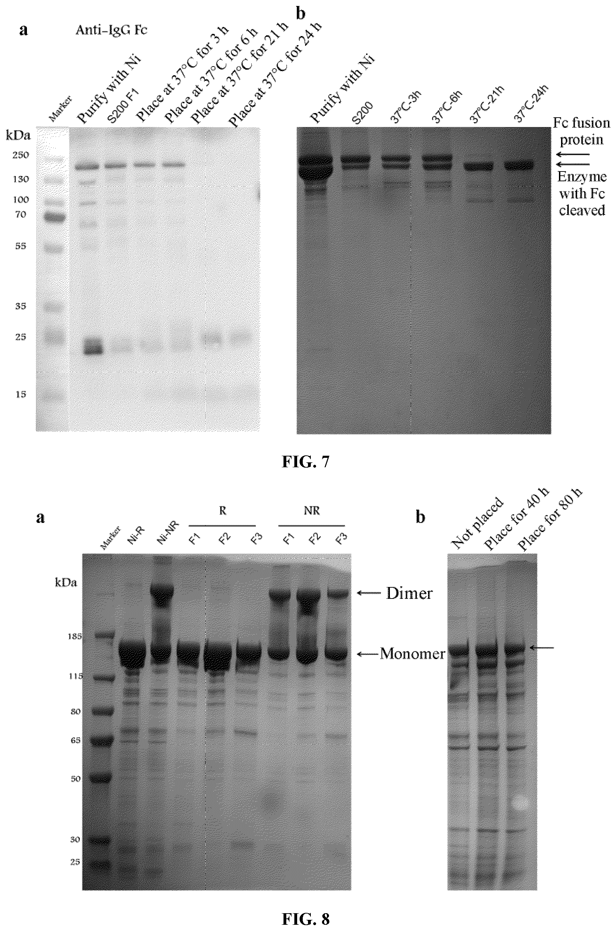

The *in vitro* activity assay showed that the Fusion Protein 3 was successfully expressed from the pET30a-TIGR4 (665-2004)-IgG1 Fc plasmid in the prokaryotic cells (as shown in FIG. 4b), and also had *in vitro* cleaving activity against IgA (as shown in FIG. 4a). The pET30a-TIGR4 (665-2004)-IgG1 Fc plasmid was expressed in the HEK293 eukaryotic cells, but the *in vitro* cleaving activity against IgA was not detected (data was not shown). In addition, activity verification after nickel column purification or S200 purification showed that the Fusion Protein 3 obtained by purifying with nickel retained the *in vitro* cleaving activity against IgA after being placed overnight at 4°C or 24 h; and F1, F2 and F3 flow-through peaks obtained by purifying with S200 also had the *in vitro* cleaving activity against IgA (as shown in FIG. 5). The ratio of dimers formed in the Fusion Protein 3 in F1 and F2 was the highest (as shown in FIG. 6). The obtained flow-through peaks were placed at 37°C, and the result showed that obvious bands of the Fusion Protein 3 retained after 6 h for F1, but when the flow-through peaks were placed for 21h or longer, the Fusion Protein 3 was cleaved, and only the truncated form of IgA protease TIGR4 (665-2004) with Fc cleaved and some residual amino acids from the IgG1 Fc were left (as shown in FIG. 7a and FIG. 7b).

The *in vitro* activity assay showed that the Fusion Protein 4 was also successfully expressed from the pET30a-TIGR4 (665-2004)-IgG4 Fc plasmid, and a part of F1, a part of F2 and a part of F3 formed a dimer form (as shown in FIG. 8a). In addition, stability assay showed that a sample purified with nickel retained a clear target band after being placed at 37°C for 40 h and even 80 h, indicating that the Fusion Protein 4 had good stability (as shown in FIG. 8b).

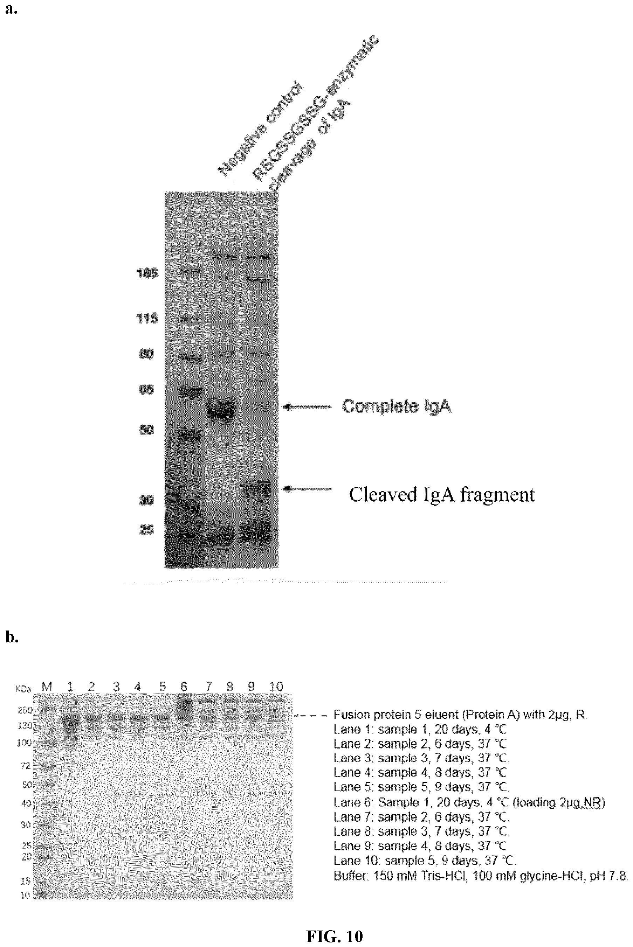

The *in vitro* activity assay showed that the Fusion Protein 5 was also successfully expressed from the other pET30a-TIGR4 (665-2004)-IgG4 Fc plasmid and also had the *in vitro* cleaving activity against IgA (as shown in FIG. 10a). In addition, stability assay showed that a sample purified with nickel column still had a clear target band after being placed at 37°C for 9 days, indicating that the Fusion Protein 5 had good stability (as shown in FIG. 10b).

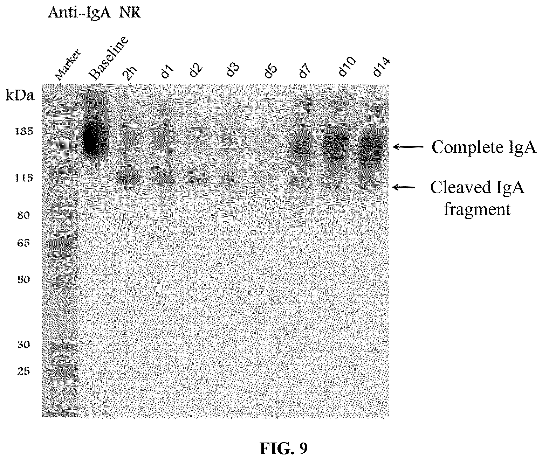

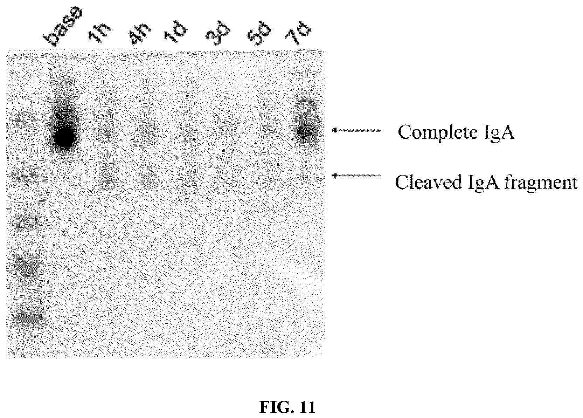

The *in vivo* activity experiments also showed that after the humanized IgA1 (alpha 1KI-Tg) C57BL/6 mice received a single-needle tail intravenous injection of the Fusion Protein 4, the IgA in blood was cleaved into an Fc segment and a Fab segment by the Fusion Protein 4, which lasted for at least 5 d (as shown in FIG. 9). The *in vivo* activity experiments also showed that after the humanized IgA1 (alpha 1KI-Tg) C57BL/6 mice received a single-needle tail intravenous injection of the Fusion Protein 5, the IgA in blood was cleaved into an Fc segment and a Fab segment by the Fusion Protein 5, which lasted for at least 5 d (as shown in FIG. 11).

### Example 3: Exploring other IgA Proteases

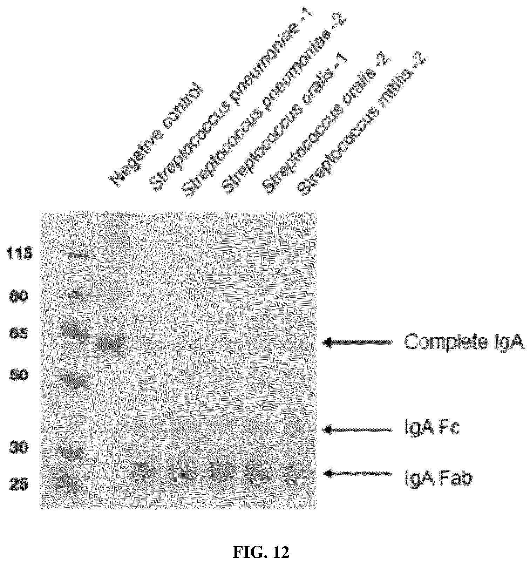

The inventors screened several amino acid sequences with certain homology to the wild-type IgA protease of *Streptococcus pneumoniae* TIGR4 strain from the metagenomic database, and synthesized five TIGR4 homologoases. Their amino acid sequences are as set forth in SEQ ID NO: 26, SEQ ID NO: 27, SEQ ID NO: 28, SEQ ID NO: 29 and SEQ ID NO: 30, respectively. According to the *in vitro* activity assay described in Example 2.3, the inventors tested the cleavage activity of these TIGR4 homologoases against IgA1. The results are shown in FIG. 12. *"Streptococcus pneumoniae-*1*"* in FIG. 12 indicates that the polypeptide set forth in SEQ ID NO: 26 was mixed with the substrate IgA1 *in vitro*; *"Streptococcus pneumoniae-2"* indicates that the polypeptide set forth in SEQ ID NO: 27 was mixed with the substrate IgA1 *in vitro; "Streptococcus oralis-1"* indicates that the polypeptide set forth in SEQ ID NO: 28 was mixed with the substrate IgA1 *in vitro*; *"Streptococcus oralis-2"* means that the polypeptide set forth in SEQ ID NO: 29 was mixed with the substrate IgA1 *in vitro*; *"Streptococcus mitilis-2"* indicates that the polypeptide set forth in SEQ ID NO: 30 was mixed with the substrate IgA1 *in vitro.*

As shown in FIG. 12, all of the polypeptides set forth in SEQ ID NO: 26, SEQ ID NO: 27, SEQ ID NO: 28, SEQ ID NO: 29, and SEQ ID NO: 30 had enzymatic cleavage activities against IgA1.

Although the present disclosure presents and describes the invention in a particular manner by reference to particular examples, it should be understood by those skilled in the art that the disclosure above may be subject to various variations in form and detail without departing from the main concept and scope of protection disclosed in the present disclosure.

## Claims

1. A fusion protein, comprising a first polypeptide and a second polypeptide, wherein the first polypeptide is a truncated form of IgA protease, and the second polypeptide cannot be cleaved by an IgA protease or a truncated form of IgA protease.

2. The fusion protein of claim 1, wherein the truncated form of IgA protease comprises a non-natural truncated fragment of a wild-type IgA protease obtained from or derived from a *Streptococcus pneumoniae* TIGR4 strain, or having at least 70% sequence identity to the non-natural truncated fragment.

3. The fusion protein of claim 2, wherein the non-natural truncated fragment has an amino acid mutation, deletion, insertion or modification compared to the wild-type IgA protease of *Streptococcus pneumoniae* TIGR4 strain, such that the truncated form of IgA protease loses or reduces its self-cleaving function.

4. The fusion protein of claim 3, wherein the amino acid mutation, deletion, insertion or modification occurs at a natural self-cleaving site of the wild-type IgA protease of *Streptococcus pneumoniae* TIGR4 strain, within 5 amino acid residues upstream and/or within 5 amino acid residues downstream of the natural self-cleaving site.

5. The fusion protein of any one of claims 2 to 4, wherein the non-natural truncated fragment is a N-terminal or C-terminal truncated fragment of the wild-type IgA protease obtained from or derived from a *Streptococcus pneumoniae* TIGR4 strain.

6. The fusion protein of any one of claims 2 to 5, wherein deposit number of the *Streptococcus pneumoniae* TIGR4 strain is ATCC BAA-334.

7. The fusion protein of claim 5 or 6, wherein the C-terminal truncated fragment comprises a polypeptide fragment of at least 703 continuous amino acids starting from position 43 of the wild-type IgA protease obtained from or derived from a *Streptococcus pneumoniae* TIGR4 strain, or having at least 70% sequence identity to the polypeptide fragment.

8. The fusion protein of claim 7, wherein an amino acid sequence of the wild-type IgA protease of *Streptococcus pneumoniae* TIGR4 strain is as set forth in SEQ ID NO: 1.

9. The fusion protein of claim 8, wherein the natural self-cleaving site is between position 43 and position 745 of the amino acid sequence as set forth in SEQ ID NO: 1.

10. The fusion protein of claim 8, wherein the natural self-cleaving site is between position 71 and position 72, between position 210 and position 211, between position 228 and position 229, between position 239 and position 240, between position 245 and position 246, between position 382 and position 383, between position 418 and position 419, between position 439 and position 440, between position 490 and position 491, between position 506 and position 507, between position 509 and position 510, between position 514 and position 515, between position 533 and position 534, between position 536 and position 537, between position 563 and position 564, between position 594 and position 595, between position 613 and position 614, between position 616 and position 617, between position 639 and position 640, between position 645 and position 646, or between position 678 and position 679 of the amino acid sequence as set forth in SEQ ID NO: 1.

11. The fusion protein of any one of the preceding claims, wherein the first polypeptide comprises a polypeptide fragment of at least 1300 (*e.g.,* at least 1310, at least 1320, at least 1330, or at least 1340) continuous amino acids starting from position 665 of the amino acid sequence as set forth in SEQ ID NO: 1.

12. The fusion protein of any one of the preceding claims, wherein the first polypeptide comprises a polypeptide fragment of amino acids starting from position 665 to position 2004 of the amino acid sequence as set forth in SEQ ID NO: 1, or having at least 70% sequence identity to the polypeptide fragment.

13. The fusion protein of claim 12, having an amino acid conservative substitution at one or more sites compared to the amino acid sequence of the polypeptide fragment.

14. The fusion protein of any one of the preceding claims, having an enzymatic activity of specifically cleaving human IgA.

15. The fusion protein of claim 14, having an enzymatic activity of specifically cleaving human IgA heavy chain.

16. The fusion protein of claim 15, having an enzymatic activity of specifically cleaving human IgA heavy chain hinge region.

17. The fusion protein of any one of claims 14 to 16, having an enzymatic activity of specifically cleaving human IgA1.

18. The fusion protein of any one of the preceding claims, wherein the first polypeptide comprises an amino acid as set forth in SEQ ID NO: 2.

19. The fusion protein of any one of the preceding claims, wherein the second polypeptide is located at N-terminus or C-terminus of the first polypeptide.

20. The fusion protein of any one of the preceding claims, wherein the first polypeptide and the second polypeptide are linked via a linker.

21. The fusion protein of any one of claims 1-19, wherein the first polypeptide and the second polypeptide are directly linked to each other.

22. The fusion protein of claim 20, wherein the linker is selected from the group consisting of a cleavable linker, a non-cleavable linker, a peptide linker, a flexible linker, a rigid linker, a helical linker and a non-helical linker.

23. The fusion protein of claim 22, wherein the linker comprises a peptide linker.

24. The fusion protein of claim 23, wherein the peptide linker comprises a linker comprising a glycine and a serine.

25. The fusion protein of claim 24, wherein the linker comprising a glycine and a serine comprises one or more repeats of a sequence as set forth in SEQ ID NO: 15 (GGGS), SEQ ID NO: 16 (GGGGS), SEQ ID NO: 17 (GGGGGGS) or SEQ ID NO: 19 (GSS).

26. The fusion protein of claim 25, wherein the linker comprises an amino acid sequence as set forth in SEQ ID NO: 18 (GGGGSGGGGSGGGGS), SEQ ID NO: 20 (GSSGSSG) or SEQ ID NO: 21 (RSGSSGSSG).

27. The fusion protein of any one of the preceding claims, wherein the second polypeptide comprises an amino acid sequence for extending half-life of the first polypeptide in a subject.

28. The fusion protein of claim 27, wherein the second polypeptide is selected from: an Fc domain and albumin.

29. The fusion protein of claim 28, wherein the Fc domain comprises a hinge region.

30. The fusion protein of claim 29, wherein the Fc domain is derived from human IgG Fc domain.

31. The fusion protein of claim 30, wherein the Fc domain is derived from human IgG4 Fc domain.

32. The fusion protein of any one of claims 28 to 31, wherein the Fc domain comprises an amino acid sequence having at least 70%, at least 75%, at least 80%, at least 85%, at least 90% or at least 95% sequence identity to SEQ ID NO: 14.

33. The fusion protein of claim 32, wherein the Fc domain comprises an amino acid sequence as set forth in SEQ ID NO: 14.

34. The fusion protein of any one of claims 28 to 33, wherein the Fc domain comprises one or more mutations that extend half-life of the fusion protein.

35. The fusion protein of any one of claims 28 to 34, wherein the Fc domain is linked to C-terminus or N-terminus of the first polypeptide.

36. The fusion protein of any one of the preceding claims, wherein the amino acid sequence of the first polypeptide is as set forth in SEQ ID NO: 2, and the amino acid sequence of the second polypeptide is as set forth in SEQ ID NO: 14.

37. The fusion protein of claim 36, wherein the first polypeptide and the second polypeptide are directly linked via a linker as set forth in SEQ ID NO: 20 or SEQ ID NO: 21.

38. The fusion protein of claim 36, wherein the amino acid sequence of the fusion protein is as set forth in SEQ ID NO: 8 or SEQ ID NO: 24.

39. The fusion protein of claim 28, wherein the albumin comprises one or more domains of human serum albumin.

40. The fusion protein of claim 39, wherein the albumin comprises a D3 domain of human serum albumin.

41. The fusion protein of any one of the preceding claims, further comprising a label.

42. The fusion protein of claim 41, wherein the label is selected from the group consisting of a fluorescent label, a luminescent label, a purification label and a chromogenic label.

43. The fusion protein of claim 41 or 42, wherein the label is selected from the group consisting of a c-Myc tag, an HA tag, a VSV-G tag, a FLAG tag, a V5 tag and a HIS tag.

44. The fusion protein of claim 43, wherein the label is a HIS tag comprising 6, 7, 8, 9, 10 or more histidine.

45. The fusion protein of any one of claims 41 to 44, wherein the second polypeptide is located at C-terminus of the first polypeptide and the label is located at C-terminus of the second polypeptide.

46. The fusion protein of any one of claims 41 to 45, wherein the fusion protein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days in blood circulation of a subject.

47. An isolated nucleic acid, comprising a nucleotide sequence encoding the fusion protein of any one of claims 1 to 46.

48. The nucleic acid of claim 47, comprising a nucleotide sequence as set forth in SEQ ID NO: 12 or SEQ ID NO: 25 or a nucleotide sequence having at least 70% sequence identity to SEQ ID NO: 12 or SEQ ID NO: 25.

49. A vector comprising the nucleic acid of claim 47 or 48.

50. A cell comprising the nucleic acid of claim 47 or 48 or the vector of claim 49.

51. The cell of claim 50, wherein the cell is a prokaryotic cell or a eukaryotic cell.

52. The cell of claim 51, wherein the prokaryotic cell is an *E. coli* cell.

53. The cell of claim 51, wherein the eukaryotic cell is a mammalian cell.

54. The cell of claim 53, wherein the mammalian cell is a human cell or a Chinese hamster ovary (CHO) cell.

55. The cell of claim 54, wherein the mammalian cell is a human embryonic kidney cell 293 (HEK293 cell).

56. A pharmaceutical composition, comprising the fusion protein of any one of claims 1 to 46, comprising the nucleic acid of claim 47 or 48, comprising the vector of claim 49 or comprising the cell of any one of claims 50 to 55, and a pharmaceutically acceptable carrier.

57. A method of producing a fusion protein comprising a step of culturing the cell of any one of claims 50 to 55.

58. A method of treating or preventing a disease associated with IgA deposition, comprising administering to a subject in need thereof the fusion protein of any one of claims 1 to 46 or the pharmaceutical composition of claim 56.

59. A method of treating or preventing a disease associated with IgA deposition, comprising administering to a subject in need thereof an IgA protease or the truncated form thereof, a fusion protein comprising the IgA protease or the truncated form thereof, or a pharmaceutical composition comprising the IgA protease or the truncated form thereof or the fusion protein, wherein, the amino acid sequence of the IgA protease is selected from the group consisting of: SEQ ID NO: 26, SEQ ID NO: 27, SEQ ID NO: 28, SEQ ID NO: 29, SEQ ID NO: 30 or combination thereof.

60. Use of the fusion protein of any one of claims 1 to 46 or the pharmaceutical composition of claim 56 in the manufacture of a medicament for treating or preventing a disease associated with IgA deposition.

61. Use of an IgA protease or the truncated form thereof, a fusion protein comprising the IgA protease or the truncated form thereof, or a pharmaceutical composition comprising the IgA protease or the truncated form thereof or the fusion protein in the manufacture of a medicament for treating or preventing a disease associated with IgA deposition, wherein, the amino acid sequence of the IgA protease is selected from the group consisting of: SEQ ID NO: 26, SEQ ID NO: 27, SEQ ID NO: 28, SEQ ID NO: 29, SEQ ID NO: 30 or combination thereof

62. The method of claim 58 or 59 or the use of claim 60 or 61, wherein the disease associated with IgA deposition is selected from the group consisting of IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis.

63. The method of claim 58 or 59 or the use of claim 60 or 61, wherein the disease associated with IgA deposition is IgA nephropathy, IgA vasculitis or Kawasaki disease.
